# EUROPEAN PATENT APPLICATION

(11) **EP 3 361 251 A1**
(43) Date of publication of application: **15.08.2018**
(21) Application number: 16853603.5
(22) Date of filing: 05.10.2016
(51) Int. Cl.: G01N 33/53, A61K 35/74, A61K 47/42, C12Q 1/02, G01N 33/574

(54) **METHOD FOR DETECTING CANCER CELLS, REAGENT FOR INTRODUCING SUBSTANCE INTO CANCER CELLS, AND COMPOSITION FOR TREATING CANCER**

(30) Priority: 05.10.2015 JP 2015197950; 05.10.2015 JP 2015198045; 30.03.2016 JP 2016069683
(71) Applicant: National Institute of Advanced Industrial Science and Technology, Tokyo 100-8921 (JP); University of Tsukuba, Tsukuba-shi, Ibaraki 305-8577 (JP)
(72) Inventor: TATENO, Hiroaki, Tsukuba-shi Ibaraki 305-8568 (JP); HIRABAYASHI, Jun, Tsukuba-shi Ibaraki 305-8568 (JP); ASASHIMA, Makoto, Tsukuba-shi Ibaraki 305-8565 (JP); ITOU, Yuzuru, Tsukuba-shi Ibaraki 305-8565 (JP); ONUMA, Yasuko, Tsukuba-shi Ibaraki 305-8565 (JP); ODA, Tatsuya, Tsukuba-shi Ibaraki 305-8577 (JP); OHKOHCHI, Nobuhiro, Tsukuba-shi Ibaraki 305-8577 (JP); SHIMOMURA, Osamu, Tsukuba-shi Ibaraki 305-8577 (JP)
(74) Representative: Hoffmann Eitle
(86) International application number: PCT/JP2016/079577
(87) International publication number: WO 2017/061449

(57) **Abstract**

As a technique for specifically detecting cancer cells, provided is a method for detecting cancer cells, including the steps of: bringing BC2LCN lectin into contact with a test sample; and determining the presence or absence or the amount of a sugar chain having a BC2LCN lectin binding activity in the test sample, in which the test sample is a body fluid sample of a test individual.

SEQUENCE LISTING

<110>  NATIONAL INSTITUTE OF ADVANCED INDUSTRIAL SCIENCE AND
       TECHNOLOGY
       UNIVERSITY OF TSUKUBA

<120>  METHOD FOR DETECTING CANCER CELLS, REAGENT FOR INTRODUCING
       SUBSTANCES INTO CANCER CELLS, AND COMPOSTION FOR TREATING CANCER

<130>  PSK-9030WO

<150>  JP2015-197950
<151>  2015-10-05

<150>  JP2015-198045
<151>  2015-10-05

<150>  JP2016-069683
<151>  2016-03-30

<160>  6

<170>  PatentIn version 3.5

<210>  1
<211>  156
<212>  PRT
<213>  Artificial Sequence

<220>
<223>  BC2LCN

<400>  1

Met Pro Leu Leu Ser Ala Ser Ile Val Ser Ala Pro Val Val Thr Ser
1               5                   10                  15

Glu Thr Tyr Val Asp Ile Pro Gly Leu Tyr Leu Asp Val Ala Lys Ala
            20                  25                  30

Gly Ile Arg Asp Gly Lys Leu Gln Val Ile Leu Asn Val Pro Thr Pro
        35                  40                  45

Tyr Ala Thr Gly Asn Asn Phe Pro Gly Ile Tyr Phe Ala Ile Ala Thr
    50                  55                  60

Asn Gln Gly Val Val Ala Asp Gly Cys Phe Thr Tyr Ser Ser Lys Val
65                  70                  75                  80

Pro Glu Ser Thr Gly Arg Met Pro Phe Thr Leu Val Ala Thr Ile Asp
                85                  90                  95

Val Gly Ser Gly Val Thr Phe Val Lys Gly Gln Trp Lys Ser Val Arg
            100                 105                 110

Gly Ser Ala Met His Ile Asp Ser Tyr Ala Ser Leu Ser Ala Ile Trp

115                 120                 125

Gly Thr Ala Ala Pro Ser Ser Gln Gly Ser Gly Asn Gln Gly Ala Glu
    130                 135                 140

Thr Gly Gly Thr Gly Ala Gly Asn Ile Gly Gly Gly
145                 150                 155

<210>  2
<211>  396
<212>  PRT
<213>  Artificial Sequence

<220>
<223>  rBC2LCN-ETA

<400>  2

Met Pro Leu Leu Ser Ala Ser Ile Val Ser Ala Pro Val Val Thr Ser
1               5                   10                  15

Glu Thr Tyr Val Asp Ile Pro Gly Leu Tyr Leu Asp Val Ala Lys Ala
            20                  25                  30

Gly Ile Arg Asp Gly Lys Leu Gln Val Ile Leu Asn Val Pro Thr Pro
        35                  40                  45

Tyr Ala Thr Gly Asn Asn Phe Pro Gly Ile Tyr Phe Ala Ile Ala Thr
    50                  55                  60

Asn Gln Gly Val Val Ala Asp Gly Cys Phe Thr Tyr Ser Ser Lys Val
65                  70                  75                  80

Pro Glu Ser Thr Gly Arg Met Pro Phe Thr Leu Val Ala Thr Ile Asp
                85                  90                  95

Val Gly Ser Gly Val Thr Phe Val Lys Gly Gln Trp Lys Ser Val Arg
            100                 105                 110

Gly Ser Ala Met His Ile Asp Ser Tyr Ala Ser Leu Ser Ala Ile Trp
        115                 120                 125

Gly Thr Ala Ala Pro Ser Ser Gln Gly Ser Gly Asn Gln Gly Ala Glu
    130                 135                 140

Thr Gly Gly Thr Gly Ala Gly Asn Ile Gly Gly Gly Ala Leu Glu Gly
145                 150                 155                 160

Ser Gly Gly Gly Gly Ser Gly Gly Gly Glu Phe Leu Gly Asp Gly Gly
                165                 170                 175

Asp Val Ser Phe Ser Thr Arg Gly Thr Gln Asn Trp Thr Val Glu Arg
            180                 185                 190

Leu Leu Gln Ala His Arg Gln Leu Glu Glu Arg Gly Tyr Val Phe Val
        195                 200                 205

Gly Tyr His Gly Thr Phe Leu Glu Ala Ala Gln Ser Ile Val Phe Gly
    210                 215                 220

Gly Val Arg Ala Arg Ser Gln Asp Leu Asp Ala Ile Trp Arg Gly Phe
225                 230                 235                 240

Tyr Ile Ala Gly Asp Pro Ala Leu Ala Tyr Gly Tyr Ala Gln Asp Gln
                245                 250                 255

Glu Pro Asp Ala Arg Gly Arg Ile Arg Asn Gly Ala Leu Leu Arg Val
            260                 265                 270

Tyr Val Pro Arg Ser Ser Leu Pro Gly Phe Tyr Arg Thr Ser Leu Thr
        275                 280                 285

Leu Ala Ala Pro Glu Ala Ala Gly Glu Val Glu Arg Leu Ile Gly His
    290                 295                 300

Pro Leu Pro Leu Arg Leu Asp Ala Ile Thr Gly Pro Glu Glu Glu Gly
305                 310                 315                 320

Gly Arg Leu Glu Thr Ile Leu Gly Trp Pro Leu Ala Glu Arg Thr Val
                325                 330                 335

Val Ile Pro Ser Ala Ile Pro Thr Asp Pro Arg Asn Val Gly Gly Asp
            340                 345                 350

Leu Asp Pro Ser Ser Ile Pro Asp Lys Glu Gln Ala Ile Ser Ala Leu
        355                 360                 365

Pro Asp Tyr Ala Ser Gln Pro Gly Lys Pro Pro Arg Glu Asp Leu Lys
    370                 375                 380

Pro Pro His His His His His His Lys Asp Glu Leu
385                 390                 395

<210>  3
<211>  367
<212>  PRT
<213>  Artificial Sequence

<220>
<223>  DomainI-III(PE38)

<400>  3

Glu Gly Gly Ser Leu Ala Ala Leu Thr Ala His Gln Ala Cys His Leu
1               5                   10                  15

Pro Leu Glu Thr Phe Thr Arg His Arg Gln Pro Arg Gly Trp Glu Gln
            20                  25                  30

Leu Glu Gln Cys Gly Tyr Pro Val Gln Arg Leu Val Ala Leu Tyr Leu
        35                  40                  45

Ala Ala Arg Leu Ser Trp Asn Gln Val Asp Gln Val Ile Arg Asn Ala
    50                  55                  60

Leu Ala Ser Pro Gly Ser Gly Gly Asp Leu Gly Glu Ala Ile Arg Glu
65                  70                  75                  80

Gln Pro Glu Gln Ala Arg Leu Ala Leu Thr Leu Ala Ala Ala Glu Ser
                85                  90                  95

Glu Arg Phe Val Arg Gln Gly Thr Gly Asn Asp Glu Ala Gly Ala Ala
            100                 105                 110

Asn Ala Asp Val Val Ser Leu Thr Cys Pro Val Ala Ala Gly Glu Cys
        115                 120                 125

Ala Gly Pro Ala Asp Ser Gly Asp Ala Leu Leu Glu Arg Asn Tyr Pro
    130                 135                 140

Thr Gly Ala Glu Phe Leu Gly Asp Gly Gly Asp Val Ser Phe Ser Thr
145                 150                 155                 160

Arg Gly Thr Gln Asn Trp Thr Val Glu Arg Leu Leu Gln Ala His Arg
                165                 170                 175

Gln Leu Glu Glu Arg Gly Tyr Val Phe Val Gly Tyr His Gly Thr Phe
            180                 185                 190

Leu Glu Ala Ala Gln Ser Ile Val Phe Gly Gly Val Arg Ala Arg Ser
        195                 200                 205

Gln Asp Leu Asp Ala Ile Trp Arg Gly Phe Tyr Ile Ala Gly Asp Pro
    210                 215                 220

Ala Leu Ala Tyr Gly Tyr Ala Gln Asp Gln Glu Pro Asp Ala Arg Gly
225                 230                 235                 240

Arg Ile Arg Asn Gly Ala Leu Leu Arg Val Tyr Val Pro Arg Ser Ser
                245                 250                 255

Leu Pro Gly Phe Tyr Arg Thr Ser Leu Thr Leu Ala Ala Pro Glu Ala
            260                 265                 270

Ala Gly Glu Val Glu Arg Leu Ile Gly His Pro Leu Pro Leu Arg Leu
        275                 280                 285

Asp Ala Ile Thr Gly Pro Glu Glu Glu Gly Gly Arg Leu Glu Thr Ile
    290                 295                 300

Leu Gly Trp Pro Leu Ala Glu Arg Thr Val Val Ile Pro Ser Ala Ile
305                 310                 315                 320

Pro Thr Asp Pro Arg Asn Val Gly Gly Asp Leu Asp Pro Ser Ser Ile
                325                 330                 335

Pro Asp Lys Glu Gln Ala Ile Ser Ala Leu Pro Asp Tyr Ala Ser Gln
            340                 345                 350

Pro Gly Lys Pro Pro His His His His His His Lys Asp Glu Leu
        355                 360                 365

<210>  4
<211>  5
<212>  PRT
<213>  Artificial Sequence

<220>
<223>  Spacer1

<400>  4

Gly Ser Gly Gly Gly
1               5

<210>  5
<211>  4
<212>  PRT
<213>  Artificial Sequence

<220>
<223>  Spacer2

<400>  5

Gly Gly Gly Ser
1

<210>  6

<211>  4
<212>  PRT
<213>  Artificial Sequence

<220>
<223>  ER retention signal

<400>  6

Lys Asp Glu Leu
1

## Description

### Technical Field

The present invention relates to a reagent and method for detecting cancer cells, a reagent and method for separating cancer cells, a reagent for introducing a substance into cancer cells, and a composition for treating, e.g., a cancer. The present invention more specifically relates to, e.g., a reagent for use in detecting cancer cells using lectin binding to a sugar chain specifically expressed on a cancer cell surface.

The present invention also relates to a lectin probe that can specifically stain and concentrate high-grade cancer cells such as cancer stem cells of a predetermined cancer type and a method for detecting the presence of high-grade cancer cells and concentrating the cancer cells by using the probe.

The present invention also relates to a method for selectively removing cancer cells by a fusion protein of lectin probe, which takes advantages of the property of the lectin probe being incorporated into cancer cells.

The present invention also relates to a method for diagnosing and treating a high-grade cancer particularly including an anticancer-drug resistant cancer; more specifically, relates to a method for detecting a high-grade cancer (for example, cancer cells such as pancreatic cancer, particularly anticancer-drug resistant cancer cells; cancer stem cells); a method for diagnosing a degree of malignancy of a cancer and a cancer cell detecting agent for diagnosis; a method for killing/removing cancer cells; and an anti-cancer agent, particularly a therapeutic agent for a drug-resistant cancer. Owing to the method of the invention, a novel therapeutic method effective for treating a cancer can be provided. The present invention can be used in the cancer research field and drug development/medical field including cancer diagnosis and cancer therapy.

### Background Art

Cancer is the leading cause of death in Japan. There are many different types of cancers (e.g., breast cancer, prostate cancer, lung cancer, stomach cancer, large intestine cancer) and causes of cancer vary. Because of this, morphology of pathological tissue/cell, gene expression and protein/sugar chain expression on a cell surface vary depending on individual types of cancers. In addition, even in a single cancer tissue, it is frequently observed that the cancer tissue is constituted of various types of cancer cells (cancer cells different in malignancy, cancer stem cells). Because of this, the diagnosis method regularly used, and the type of the cancer marker effectively used vary depending on the types of cancer cells.

For example, breast cancer is the number one cancer of those that females are affected, in Japan. The probability of Japanese women diagnosed as breast cancer in the lifetime is one out of 16 and the incidence tends to increase. About 20% of female patients with breast cancer die of the breast cancer. For early detection and effective treatment for breast cancer, basic and application research have been aggressively carried out. Breast cancer is non-invasively screened and examined by, e.g., mammography. If a malignant lesion is found or cancer is suspected, a small amount of cells or tissue pieces is collected by aspiration biopsy cytology and needle biopsy (tissue diagnosis). The aspiration biopsy cytology and needle biopsy (tissue diagnosis) are diagnostic methods relatively low patient charge, compared to surgical biopsy for collecting a tissue but inferior in diagnostic accuracy to surgical biopsy, and sometimes undiagnosed.

Prostate cancer is the third-ranked cancer of those that males are affected in Japan and incidence tends to increase. Particularly in Japan, patients died of prostate cancer occupy about 3.5% of patients died of cancer. Recently, coping with the problem became an urgent task. Prostate cancer is screened by a blood test (PSA test) and checked by, e.g., rectal examination and transrectal ultrasonograph. As a result, if a cancer is suspected, pathological tissue diagnosis by needle biopsy is carried out for evaluation.

Small-cell lung cancer (SCLC) occupies about 15% of bronchogenic cancers. It is said that growth of the cancer cells and metastasis thereof extremely quickly occur, and malignancy thereof is high compared to other lung cancers (adenocarcinoma, squamous cell carcinoma, large-cell cancer). Thus, diagnosis in the early stage and effective treatment are inevitably required. Lung cancer is screened by chest roentgenogram. If a pathological change is suspected, the presence or absence of cancer cells in the sputum is examined by sputum examination. Further, the pathological tissue is evaluated by endoscopic examination and needle biopsy for diagnosis.

In any case, biopsy is required for definite diagnosis of cancer. However, if diagnosis cannot be made since the amount of cells is extremely low, as described above, tissue collection should be additionally performed, and a large physical burden is to be given to the patient. In the circumstances, in order to make definite diagnosis highly effectively by using even a small amount of cells obtained by, e.g., puncture suction, development of a method for staining the cells is required. In particular, in determining therapeutic strategy for the patient, determining a degree of malignancy of cancer of the patient is the most important item for diagnosis.

Since individual types of cancer cells differ in pathological tissue/cell morphology, gene expression and expression of cell-surface protein/sugar chain, it is difficult to prepare an anti-cancer agent and antibiotic universally used. If an anti-cancer agent is selected, the agent is not effective in many cancers. An anti-cancer agent is required to efficiently kill cancer cells alone without fail or suppress growth of the cells; however, actually, many anti-cancer agents inevitably produce a side effect on the normal tissue such as a peripheral tissue. This is a serious problem.

Up to present, researches for detecting cancer cells and development of an anti-cancer agent by recognizing a protein present on the surfaces of various cancer cells, have been aggressively made; however, since the same protein is also expressed in the same level on normal cells, unfavorable effects in view of specificity were mostly obtained. In order to enhance specificity to cancer cells, recently, attention has been focused on the fact that a sugar chain of a glycoprotein and a glycolipid present in the serum of a cancer patient changes. Even if the amount of a protein from which a sugar chain is extended is the same, the amount of the sugar chain increases in a cancer-specific manner. Thus, a technique using a substance which can recognize and detect a sugar chain as an (sugar-chain) epitope, i.e., an anti-glycoprotein antibody or an anti-glycolipid antibody, for detecting cancer cells, has been aggressively developed. Furthermore, production of vaccines using these sugar chain antigens has been activated (Non Patent Literature 1).

In the meantime, as one of the malignant cancers the most feared of various cancer types, pancreatic cancer is known. Pancreatic cancer generally refers to a cancer developed from the pancreas. The pancreas has exocrine glands secreting digestive enzymes (e.g., amylase, trypsin, lipase) and endocrine glands secreting hormones (e.g., insulin). The pancreatic cancers are roughly classified into exocrine system (digestive enzyme secretory system) cancer and endocrine system (hormone secretory system) cancer, and the exocrine system cancer occupies 95%. Of them, invasive pancreatic duct cancer developed in the epithelium of the pancreatic duct most commonly occurs and occupies 85% of the entirety. Because of this, pancreatic cancer generally refers to invasive pancreatic duct cancer. In "epithelial cancer" or "digestive system epithelial cancer" in the specification, invasive pancreatic duct cancer is included.

The 5-year survival rate of pancreatic cancer is 5.5%. The survival rate of pancreatic cancer of all cancers is extremely low. Early diagnosis/surgical resection is only a treatment for pancreatic cancer leading to permanent cure. The percentage of patients whose cancer is determined as an excisable cancer at the time of diagnosis is at most about 20%. It is said that even if the whole cancer can be removed by surgical resection, about 90% of the patients will experience recurrence. This is considered because pancreatic cancer spreads to, e.g., the liver in the early stage and develops distant metastasis and peritoneal metastasis.

If surgical resection can be fortunately made, there is a high possibility that pancreatic cancer develops micro-metastasis or remains, with the result that a postoperative therapy such as a chemotherapy is required after the surgery. A chemotherapy is also applied to recurrent pancreatic cancer after the resection. As the chemotherapy to pancreatic cancer having distant metastasis, which fails to be a target for resection and pancreatic cancer once resected but renewed, a chemotherapy using gemcitabine hydrochloride (GEM, Gemzar) has been applied as a standard therapy since 2001. Also, since 2006, TS-1 (also called as S-1) (Tegafur-gimeracil-oteracil potassium combination drug) has come to be used as a newly approved anti-cancer agent covered by insurance in this country.

However, since it is difficult for these chemotherapeutic agents to provide a permanent cure, development of a new drug is strongly desired. A trial study to directly attack cancer stem cells that will be developed into drug-resistant cancer cells has been widely conducted by using, e.g., breast cancer, as a target. A treatment with e.g., an anti-CD176 antibody targeting a cancer stem cell marker, CD176 (Patent Literature 1) and a therapy with a vaccine containing a glycolipid GloboH antigen and targeting, e.g., pancreatic cancer (Patent Literature 2) are mentioned; however, there are no reports that they are actually applied to pancreatic cancer.

Thereafter, erlotinib (molecular target therapeutic drug) and a new therapy such as FOLFIRINOX therapy, which is a reinforced chemotherapy for large intestine cancer, have been developed. However, these treatments are expensive, just helpful for extending life expectancy by several months and far from permanent cure for pancreatic cancer.

In the circumstances as mentioned above, development of a novel therapy and novel anti-cancer agent against pancreatic cancer have been strongly desired.

As described above, since pancreatic cancer is a highly malignant cancer which develops metastasis in the early stage and application of early treatment has a large effect on the prognosis, it is extremely important to find the cancer in the early stage. In addition, since an accurate determination on whether the cancer is resectable or not is required at the time of diagnosis, it is important to accurately determine a degree of malignancy of the cancer. However, observing expression of various pancreatic cancer marker genes in a pancreatic cancer tissue which must be taken by biopsy, puts a large burden on a subject. For the reason, noninvasive diagnosis is essential. Also, in pancreatic cancer, search for a pancreatic cancer marker in serum samples has been heretofore actively carried out.

In particular, a blood protein marker, which is a product of a gene specifically expressed to pancreatic cancer and found in the blood, has heretofore attracted attention as a tumor marker. Many serum protein markers have been suggested, which include a plasma apolipoprotein, ApoAII (Patent Literature 3), a modified α-fibrinogen protein (Patent Literature 4), a complement C3 precursor (Patent Literature 5), CXCL4L1 (Patent Literature 6) belonging to the CXC-chemokine family, REG4 (Patent Literature 7) belonging to the REG family, a soluble antigen, 3C4-Ag (Patent Literature 8) derived from a pancreatic cancer specific antigen, PaCa-Ag1, human pancreatic ribonuclease 1 (Patent Literature 9) and protein myoferrin (Patent Literature 10).

Recently, attention has been focused on the fact that a sugar chain of a glycoprotein and a glycolipid in the serum changes and a technique for detecting a sugar chain increasing specifically to pancreatic cancer, as a pancreatic cancer marker, has been actively developed. For example, a diagnostic method for pancreatic cancer based on measuring the amounts of a plurality of specific sugar chains in the serum (Patent Literatures 11, 12); a sugar chain marker constituted of a fucosylated sugar chain structure (by focusing attention on the fact that the sugar chain structure at a specific position in a sugar chain of human haptoglobin is fucosylated specifically to pancreatic cancer)(Patent Literature 13); and a diagnostic method for pancreatic cancer by detecting a pathologically changed human haptoglobin by using fucose α 1→6 specific lectin derived from Basidiomycete (Patent Literature 14), are proposed.

Pancreatic cancer is a malignant cancer since postoperative recurrence rate thereof is high and the cancer quite quickly acquires resistance to a chemotherapeutic agent. To know prognosis, many trails have been made for detecting pancreatic cancer stem cells or anticancer agent-resistant pancreatic cancer cells. For example, a method of detecting resistance to an anti-cancer agent for pancreatic cancer by immunohistochemical staining of AnneXin-II protein (Patent Literature 15) is mentioned. Other than this, a method for detecting tumor-associated carbohydrate antigens (CD176, CD174, CD173), which were identified as antigens specifically expressing in many cancer stem cells, as cancer stem cell markers (Patent Literature 16), is also known. As mentioned above, as a pancreatic cancer diagnosis method, not only a noninvasive diagnosis method for finding pancreatic cancer in the early stage but also a diagnosis method for finding malignant pancreatic cancer such as an anticancer drug-resistant cancer, has been desired.

The present inventors previously made global analysis on sugar chain profile of human iPS cells (114 specimens) prepared from five different types of cells (skin, fetal lung, endometrium, placental artery, amniotic membrane) and human ES cells (9 specimens) by using a lectin microarray. As a result, it was found that although original somatic cells had different sugar chain profiles per tissue, the iPS cells produced showed almost the same sugar chain profile. From this, it was found that if an initializing gene is introduced, the cells will equally have analogous sugar chain structure to ES cells. As a result that lectin array data of human ES/iPS cells and human somatic cells were precisely analyzed, it was estimated that the expression levels of α2-6Sia, α1-2Fuc and type 1 LacNAc in undifferentiated human ES/iPS cells remarkably increase compared to the somatic cells. The estimation was supported by expression analysis of a glycosyltransferase gene using DNA array and a method using a mass spectrometer (Non Patent Literature 2).

BC2LCN lectin is a recombinant, which is obtained by expressing BC2LCN lectin (YP_002232818), which corresponds to the N-terminal domain of BC2L-C protein derived from a gram negative bacterium (Burkholderia cenocepacia), in transformed E. coli, and which recognizes sugars at non-reducing ends of a complex sugar chain: "Fucα1-2Galβ1-3GlcNAc" and "Fucα1-2Galβ1-3GlcNAc" (Non Patent Literatures 2, 3).

The present inventors found, in the experiment using a lectin array as mentioned above, that BC2LCN lectin reacts with all human ES/iPS cells but does not react with somatic cells differentiated at all. It is interpreted that the lectin specifically reacts with both of sugar chain structures: "Fucα1-2Galβ1-3GlcNAc (= H type 1 structure)" and "Fucα1-2Galβ1-3GalNAc (= H type 3 structure)", which have two (α1-2Fuc and type 1 LacNAc) out of the sugar chains: "α1-2Fuc", "type 1 LacNAc" and "α2-6Sia", which are highly expressed in human ES/iPS cells but rarely expressed in differentiated somatic cells. From this, it was suggested that two types of sugar chains (ligands) recognized by BC2LCN lectin are novel undifferentiation sugar chain markers characterizing undifferentiated cells, and that BC2LCN lectin can be used as probes specific to the undifferentiation sugar chain markers: "Fucα1-2Galβ1-3GlcNAc" and/or "Fucα1-2Galβ1-3GalNAc" (hereinafter both are sometimes collectively referred to as "Fucα1-2Galβ1-3GlcNAc/GalNAc").

The present inventors further found that the sugar chain structures, especially, a sugar chain structure containing H type 3 sugar chain in a large amount, is expressed in a large amount over the entire cell surface like a cluster, in the ES/iPS cells, and that fluorescently labelled BC2LCN lectin can be used in specifically staining the ES/iPS cells alone in accordance with a general immunohistochemical method, in other words, can be used as an ES/iPS cell staining probe (Patent Literatures 17, 18).

### Citation List

### Patent Literatures

Patent Literature 1: National Publication of International Patent Application No. 2013-517487
Patent Literature 2: Japanese Patent No. 5628158
Patent Literature 3: Japanese Patent Laid-Open No. 2010-175452
Patent Literature 4: Japanese Patent Laid-Open No. 2009-29731
Patent Literature 5: Japanese Patent Laid-Open No. 2007-51880
Patent Literature 6: National Publication of International Patent Application No. 2012-509683
Patent Literature 7: National Publication of International Patent Application No. 2009-528507
Patent Literature 8: National Publication of International Patent Application No. 2007-525410
Patent Literature 9: Japanese Patent Laid-Open No. 2010-180174
Patent Literature 10: National Publication of International Patent Application No. 2013-545992
Patent Literature 11: Japanese Patent Laid-Open No. 2012-63139
Patent Literature 12: Japanese Patent Laid-Open No. 2013-83490
Patent Literature 13: Japanese Patent Laid-Open No. 2009-168470
Patent Literature 14: WO2011/089988
Patent Literature 15: WO2006/129729
Patent Literature 16: National Publication of International Patent Application No. 2013-517487
Patent Literature 17: WO2013/065302
Patent Literature 18: WO2013/128914
Patent Literature 19: WO2014/126146

### Non Patent Literatures

Non Patent Literature 1: Heimburg-Molinaro J, et al., Vaccine, 2011, 29(48): 8802-26.
Non Patent Literature 2: Tateno H, et al., J. Biol. Chem., 2011, 286(23): 20345-53.
Non Patent Literature 3: Sulak O, et al., Structure, 2010, 18(1): 59-72.
Non Patent Literature 4: Chang WW. et al., Proc. Natl. Acad. Sci. USA, 2008, 105(33): 11667-11672.
Non Patent Literature 5: C. Li, D. et al., Cancer Res., 67, 2007, 1030-1037.
Non Patent Literature 6: P. C. Hermann, et al., Cell Stem Cell, 2007, 1, 313-323.
Non Patent Literature 7: Hoang B, et al., J Pharm. 2014 Aug 25; 471(1-2): 224-33.
Non Patent Literature 8: Kondo,T., et al, J. Biol. Chem., 1998. 263, 9470-9475.
Non Patent Literature 9: Tateno H, et al., Stem Cell Reports, 2015, 4(5): 811-20.

### Summary of Invention

### Technical Problem

A primary object of the present invention is to provide a technique for specifically detecting, separating or killing cancer cells.

### Solution to Problem

This time, the present inventors obtained the following findings:
(1) BC2LCN lectin recognizes and binds to a glycoprotein present on the cell surfaces of breast cancer cells and prostate cancer cells (see, Examples 1-1, 1-2, 1-3).
(2) BC2LCN lectin has responsiveness to breast cancer, lung cancer, pancreatic cancer, large intestine cancer, stomach cancer, bile duct cancer, uterine cancer and ovarian cancer; specifically has responsiveness to an epithelial cancer, particularly to digestive-system epithelial cancer and breast cancer; and exhibits particularly high responsiveness to large intestine cancer and bile duct cancer (see, Examples 1-4, 1-5, 1-6, 1-7, 1-8).
(3) BC2LCN lectin does not react with a normal tissue (see, Examples 1-9).
(4) BC2LCN-positive cancer cells have an anchorage-independent high proliferation potential and highly express a known cancer stem cell marker (Example 2).
(5) A BC2LCN/cell killing toxin fusion protein (BC2LCN-ETA) extremely efficiently kills BC2LCN-positive cancer cells (Example 3).
(6) In mouse model subcutaneously transplanted with Capan-1 cells (BC2LCN-positive) exhibiting an analogous incidence to clinical pancreatic cancer, cancer cells remaining after a treatment with an anti-cancer agent are strongly positive to BC2LCN (Example 4), and a fusion protein (BC2LCN-ETA and BC2LCN-PE38) formed of BC2LCN and a cell killing toxin, exhibits a remarkable antitumor effect (Example 5).
(7) Cancer cells contained in cells can be detected by using the culture supernatant of the cells, and a cancer in a living body can be detected by using a body fluid sample taken from an individual affected with the cancer (Example 6).

Based on the above findings, the present invention, in an aspect, provides the following [1] to [82].
[1] A reagent for use in detecting cancer cells containing BC2LCN lectin.
[2] The reagent according to [1], in which the cancer cells are large intestine cancer cells, bile duct cancer cells, pancreatic cancer cells, stomach cancer cells, breast cancer cells, lung cancer cells, prostate cancer cells, uterus cancer cells, ovary cancer cells or brain tumor cells.
[3] The reagent according to [2], in which the cancer cells are epithelial cancer cells.
[4] The reagent according to [3], in which the cancer cells are digestive-system epithelial cancer cells or breast cancer cells.
[5] The reagent according to [4], in which the cancer cells are large intestine cancer cells or bile duct cancer cells.
[6] The reagent according to [1], in which the cancer cells are high-grade cancer cells.
[7] The reagent according to [6], in which the high-grade cancer cells are drug resistant cancer cells or cancer stem cells.
[8] The reagent according to [6], in which the high-grade cancer cells are pancreatic cancer cells.
[9] BC2LCN lectin for use in detecting cancer cells.
[10] Use of BC2LCN lectin for detecting cancer cells.
[11] A method for detecting cancer cells, including a step of determining the presence or absence or the amount of a sugar chain having a BC2LCN lectin binding activity in a test sample.
[12] The detection method according to [11], further including a step for bringing BC2LCN lectin into contact with a test sample.
[13] The detection method according to [11] or [12], in which the cancer cells are large intestine cancer cells, bile duct cancer cells, pancreatic cancer cells, stomach cancer cells, breast cancer cells, lung cancer cells, prostate cancer cells, uterus cancer cells, ovary cancer cells or brain tumor cells.
[14] The detection method according to [13], in which the cancer cells are epithelial cancer cells.
[15] The detection method according to [14], in which the cancer cells are digestive-system epithelial cancer cells or breast cancer cells.
[16] The detection method according to [15], in which the cancer cells are large intestine cancer cells or bile duct cancer cells.
[17] The detection method according to [11] or [12], in which the cancer cells are high-grade cancer cells.
[18] The detection method according to [17], in which the high-grade cancer cells are drug resistant cancer cells or cancer stem cells.
[19] The detection method according to [18], in which the high-grade cancer cells are pancreatic cancer cells.
[20] The detection method according to any one of [11] to [19], in which the test sample is a tissue sample or cell sample derived from a tumor tissue or a peripheral tissue thereof excised out from an organ or a tissue of a test individual or derived from a biopsy material.
[21] The detection method according to any one of [11] to [19], in which the test sample is a body fluid sample of a test individual.
[22] The detection method according to [21], in which the body fluid sample is a blood-derived sample selected from whole blood, serum and plasma.
[23] A kit or apparatus for detecting the presence or absence of cancer cells in a test sample, containing at least the following (1) to (3):
   (1) BC2LCN lectin,
   (2) a labelling agent, and
   (3) a means or device for detecting a label.
[24] A reagent for separating cancer cells, containing BC2LCN lectin.
[25] The reagent according to [24], in which the cancer cells are large intestine cancer cells, bile duct cancer cells, pancreatic cancer cells, stomach cancer cells, breast cancer cells, lung cancer cells, prostate cancer cells, uterus cancer cells, ovary cancer cells or brain tumor cells.
[26] The reagent according to [25], in which the cancer cells are epithelial cancer cells.
[27] The reagent according to [26], in which the cancer cells are digestive-system epithelial cancer cells or breast cancer cells.
[28] The reagent according to [27], in which the cancer cells are large intestine cancer cells or bile duct cancer cells.
[29] The reagent according to [24], in which the cancer cells are high-grade cancer cells.
[30] The reagent according to [29], in which the high-grade cancer cells are drug resistant cancer cells or cancer stem cells.
[31] The reagent according to [30], in which the high-grade cancer cells are pancreatic cancer cells.
[32] BC2LCN lectin for use in separating cancer cells.
[33] Use of BC2LCN lectin for separating cancer cells.
[34] A method for separating cancer cells, including the steps of: bringing BC2LCN lectin into contact with a test sample, and separating cells to which BC2LCN lectin is bound and cells to which BC2LCN lectin is not bound.
[35] The separation method according to [34], in which the cancer cells are large intestine cancer cells, bile duct cancer cells, pancreatic cancer cells, stomach cancer cells, breast cancer cells, lung cancer cells, prostate cancer cells, uterus cancer cells, ovary cancer cells or brain tumor cells.
[36] The separation method according to [35], in which the cancer cells are epithelial cancer cells.
[37] The separation method according to [36], in which the cancer cells are digestive-system epithelial cancer cells or breast cancer cells.
[38] The separation method according to [37], in which the cancer cells are large intestine cancer cells or bile duct cancer cells.
[39] The separation method according to [34], in which the cancer cells are high-grade cancer cells.
[40] The separation method according to [39], in which the high-grade cancer cells are drug resistant cancer cells or cancer stem cells.
[41] The separation method according to [40], in which the high-grade cancer cells are pancreatic cancer cells.
[42] The separation method according to any one of [34] to [41], in which the test sample is a tissue sample or cell sample derived from a tumor tissue or a peripheral tissue thereof excised out from an organ or a tissue of a test individual or derived from a biopsy material.
[43] A kit or apparatus for separating cancer cells in the test sample, containing at least the following (1) and (2):
   (1) labelled BC2LCN lectin, and
   (2) a means or a device of detecting the label and separating labelled cells.
[44] A method for collecting data for determining that a test individual is affected with a cancer, including a step of measuring the amount of a sugar chain having a BC2LCN lectin binding activity in a test sample of a test individual suspected to be affected with a cancer, in which if the amount is significantly high compared to the amount of a healthy person, it is determined that the test individual is affected with a cancer.
[45] A method for diagnosing a cancer, including a step of measuring the amount of a sugar chain having a BC2LCN lectin binding activity in a test sample of a test individual suspected to be affected with a cancer, in which if the amount is significantly high compared to the amount of a healthy person, it is determined that the test individual is affected with a cancer.
[46] A method for collecting data for determining that a test individual has poor prognosis, including a step of measuring the amount of a sugar chain having a BC2LCN lectin binding activity in a test sample of a test individual affected with a cancer, in which if the amount is significantly high compared to the amount of a healthy person or a patient with a low-grade cancer, it is determined that the test individual has poor prognosis.
[47] A method for determining prognosis of a test individual, including a step of measuring the amount of a sugar chain having a BC2LCN lectin binding activity in a test sample of a test individual affected with a cancer, in which if the amount is high compared to the amount of a healthy person or a patient with a low-grade cancer, it is determined that the test individual has poor prognosis.
[48] A method for collecting data for determining that a treatment is effective, including the steps of:
   measuring the amount of a sugar chain having a BC2LCN lectin binding activity in a test sample of a test individual to which a cancer therapy is applied and
   comparing the above amount to the amount of a sugar chain having a BC2LCN lectin binding activity previously measured before the therapy in a test sample of a test individual, in which if the amount after the therapy is significantly low compared to the amount previously measured before the therapy, it is determined that the therapy is effective.
[49] A method for determining a therapeutic effect, including the steps of:
   measuring the amount of a sugar chain having a BC2LCN lectin binding activity in a test sample of a test individual to which a cancer therapy is applied, and
   comparing the above amount to the amount of a sugar chain having a BC2LCN lectin binding activity in a test sample of a test individual previously measured before the therapy, in which if the amount after the treatment is significantly low compared to the amount before the therapy, it is determined that the treatment is effective.
[50] The method according to any one of [44] to [49], in which the cancer is large intestine cancer, bile duct cancer, pancreatic cancer, stomach cancer, breast cancer, lung cancer, prostate cancer, uterine cancer, ovarian cancer or brain tumor.
[51] The method according to [50], in which the cancer is an epithelial cancer.
[52] The method according to [51], in which the cancer is digestive-system epithelial cancer or breast cancer.
[53] The method according to [52], in which the cancer is large intestine cancer or bile duct cancer.
[54] The method according to any one of [44] to [49], in which the cancer is a high-grade cancer.
[55] The method according to [54], in which the high-grade cancer is a drug-resistant cancer.
[56] The method according to [55], in which the high-grade cancer is pancreatic cancer.
[57] The method according to any one of [44] to [56], in which the test sample is a tissue sample or cell sample derived from a tumor tissue or a peripheral tissue thereof excised out from an organ or a tissue of a test individual or derived from a biopsy material.
[58] The method according to any one of [44] to [56], in which the test sample is a body fluid sample of a test individual.
[59] The method according to [58], in which the body fluid sample is a blood-derived sample selected from whole blood, serum and plasma.
[60] A reagent containing BC2LCN lectin and a substance to be fused with BC2LCN lectin, for introducing the substance into cancer cells.
[61] The reagent according to [60], in which the substance is a substance which can exhibit cytotoxicity in cancer cells.
[62] The reagent according to [61], in which the substance which can exhibit cytotoxicity in cancer cells is a toxic protein or a domain thereof having an ability to kill cells.
[63] The reagent according to [62], in which the substance which can exhibit cytotoxicity in cancer cells is a cell killing domain derived from pseudomonas exotoxin A.
[64] The reagent according to [63], in which the domain derived from pseudomonas exotoxin A and having an ability to kill cells is Domain I-III (PE38) represented by SEQ ID NO: 3.
[65] The reagent according to any one of [60] to [64], in which the cancer cells are large intestine cancer cells, bile duct cancer cells, pancreatic cancer cells, stomach cancer cells, breast cancer cells, lung cancer cells, prostate cancer cells, uterus cancer cells, ovary cancer cells or brain tumor cells.
[66] The reagent according to [65], in which the cancer cells are epithelial cancer cells.
[67] The reagent according to [66], in which the cancer cells are digestive-system epithelial cancer cells or breast cancer cells.
[68] The reagent according to [67], in which the cancer cells are large intestine cancer cells or bile duct cancer cells.
[69] The reagent according to any one of [60] to [64], in which the cancer cells are high-grade cancer cells.
[70] The reagent according to [69], in which the high-grade cancer cells are drug resistant cancer cells or cancer stem cells.
[71] The reagent according to [69], in which the high-grade cancer cells are pancreatic cancer cells.
[72] A composition for treating cancer, containing a BC2LCN-toxin fusion, which is prepared by fusing BC2LCN lectin and a substance which can exhibit cytotoxicity in cancer cells, as an active ingredient and a pharmacologically acceptable carrier.
[73] The composition according to [72], in which the substance which can exhibit cytotoxicity in cancer cells is a toxic protein or a domain thereof having an ability to kill cells.
[74] The composition according to [73], in which the substance which can exhibit cytotoxicity in cancer cells is a domain derived from pseudomonas exotoxin A and having an ability to kill cells.
[75] The composition according to [74], in which the domain derived from pseudomonas exotoxin A and having an ability to kill cells is Domain I-III (PE38) represented by SEQ ID NO: 3.
[76] The composition according to any one of [72] to [75], for use together with or in combination with a therapeutic composition applicable to a known cancer.
[77] The composition according to any one of [72] to [76], in which the cancer is large intestine cancer, bile duct cancer, pancreatic cancer, stomach cancer, breast cancer, lung cancer, prostate cancer, uterine cancer, ovarian cancer or brain tumor.
[78] The composition according to [77], in which the cancer is an epithelial cancer.
[79] The composition according to [78], in which the cancer is digestive-system epithelial cancer or breast cancer.
[80] The composition according to [79], in which the cancer is large intestine cancer or bile duct cancer.
[81] The composition according to any one of [72] to [76], in which the cancer is a high-grade cancer.
[82] The composition according to [81], in which the high-grade cancer is pancreatic cancer.

The present invention also provides, in an aspect, the following items <1> to <29>.
<1> A agent for use in detecting cancer stem cells, containing BC2LCN lectin as an active ingredient.
   Herein, BC2LCN lectin may be directly or indirectly labelled. Alternatively, when BC2LCN lectin is immobilized onto a base material and a test sample solution or a suspension to be overlaid may be labelled and subjected to detection.
<2> An agent for use in detecting high-grade cancer cells or drug resistant cancer cells, containing BC2LCN lectin as an active ingredient.
   Herein, BC2LCN lectin may be directly or indirectly labelled, or immobilized onto a base material.
<3> An agent for use in diagnosing a cancer, containing BC2LCN lectin as an active ingredient.
   Herein, BC2LCN lectin may be directly or indirectly labelled, or immobilized onto a base material.
<4> The agent for use in diagnosing a cancer, according to <3>, in which the cancer diagnostic agent is a diagnostic agent for determining whether a test individual is affected with a cancer or not or a degree of malignancy thereof, or for predicting prognosis of a test individual.
<5> A separation/purification reagent for concentrating "high-grade cancer cells", containing BC2LCN lectin as an active ingredient.
   The "high-grade cancer cells" can be concentrated by reacting labelled BC2LCN lectin with a "sample containing high-grade cancer cells" and subjecting the resultant mixture to a flow cytometer provided with a cell sorter; or alternatively, by reacting magnetic beads having BC2LCN lectin bound thereto with a "sample containing high-grade cancer cells" and subjecting the resultant sample mixture to a magnetic cell separator.
<6> A method for detecting cancer cells, characterized by measuring the presence or absence or the amount of a sugar chain having a BC2LCN lectin binding activity in a test sample by using BC2LCN lectin.
   Herein, a typical sugar chain having a BC2LCN lectin binding activity is a sugar chain having "Fucα1-2Galβ1-3GlcNAc/GalNAc" at a non-reducing end.
   BC2LCN lectin may be directly or indirectly labelled. Alternatively, detection can be made by immobilizing BC2LCN lectin to a base material and overlaying a test sample solution or a suspension labelled.
<7> The detection method according to <6>, in which the test sample is a tissue sample or cell sample derived from a tumor tissue or a peripheral tissue thereof excised out from an organ or a tissue of a test individual or derived from a biopsy material.
<8> The detection method according to <6>, in which the test sample is a body fluid sample of a test individual.
   Herein, examples of "body fluid sample" include a blood-derived sample including whole blood, serum, plasma and joint fluid of a test individual; and a body fluid (sample) such as lymph fluid, saliva and urine. Other than these, an extract derived from a tissue suspected to be a tumor tissue or a tumor, such as an extract derived from a pancreatic tissue, is included.
<9> A method for determining the presence or absence of "high-grade cancer cells" in a test sample, characterized by including a step of bringing BC2LCN lectin into contact with a test sample suspected to contain "high-grade cancer cells".
   Herein, BC2LCN lectin may be directly or indirectly labelled and may be immobilized onto a base material.
<10> A kit or apparatus for determining the presence or absence of "high-grade cancer cells" in a test sample and containing at least the following (1) to (3);
   (1) BC2LCN lectin,
   (2) a labelling agent, and
   (3) a means or device for detecting a label.

   Herein, the labelling agent (2) may be used for directly or indirectly labelling BC2LCN lectin. In the case where BC2LCN lectin is immobilized onto a base material, the labelling agent (2) can be used for labelling the test sample solution or suspension to be reacted. As the detection device (3), for example, flow cytometry analysis using FACS instrument can be applied to the former case; whereas an evanescent wave excitation light detection system can be applied to the latter case.
<11> A kit or apparatus for separating, concentrating or screening "high-grade cancer cells" in a test sample, containing at least the following items (1) and (2);
   (1) labelled BC2LCN lectin,
   (2) a means or device for detecting and separating a label.

   Herein, as the separator (2), e.g., a flow cytometer provided with a cell sorter or a magnetic cell separator can be used.
<12> A method for collecting data for determining that a test individual is affected with a cancer, including a step of measuring the amount of a sugar chain having a BC2LCN lectin binding activity in a test sample of the test individual suspected to be affected with a cancer by using BC2LCN lectin, in which if the amount is significantly high compared to the amount of a healthy person, it is determined that the test individual is affected with a cancer.
   The invention can be applied to a method for diagnosing onset of cancer, including the steps of: measuring the amount of a sugar chain having a BC2LCN lectin binding activity in a test sample of a test individual suspected to be affected with a cancer by using BC2LCN lectin, and determining whether the amount is significantly high compared to the amount of a healthy person.
<13> A method for collecting data for determining whether or not the cancer that a test individual has is a high-grade cancer containing cancer stem cells or a cancer having drug resistance, including a step of measuring the amount of a sugar chain having a BC2LCN lectin binding activity in a test sample of the test individual affected with the cancer by using BC2LCN lectin.
   The invention can be applied to a method for diagnosing a degree of malignancy of a cancer or a method for determining an optimal therapy, including the steps of: measuring the amount of a sugar chain having a BC2LCN lectin binding activity in a test sample of a test individual affected with a cancer by using BC2LCN lectin, and determining whether or not the cancer that a test individual has is a high-grade cancer containing cancer stem cells or a cancer having drug resistance.
<14> A method for collecting data for predicting prognosis of a test individual, including a step of measuring the amount of a sugar chain having a BC2LCN lectin binding activity in a test sample of a test individual affected with the cancer by using BC2LCN lectin.
   The invention can be applied to a method for predicting prognosis of a test individual (affected with a cancer), including a step of measuring the amount of a sugar chain having a BC2LCN lectin binding activity in a test sample of a test individual affected with the cancer by using BC2LCN lectin.
<15> A method for collecting data for determining efficacy of a therapy, including the steps of: measuring the expression level of a BC2LCN-positive sugar chain by using BC2LCN lectin in a test sample of a test individual to which a cancer therapy is applied; and comparing the above expression level to an expression level of the BC2LCN-positive sugar chain in a test sample of the test individual previously measured before the therapy.
   This method can be applied to a method for determining efficacy of the therapy applied by finding difference in expression level of a BC2LCN-positive sugar chain in test samples before and after the therapy, when, e.g., a surgical treatment such as surgery, chemical immunological treatment such as a treatment with an anti-cancer agent, or a radiotherapy is applied to a test individual affected with a cancer.
<16> An agent for introducing a compound into "high-grade cancer cells", containing BC2LCN lectin as an active ingredient, for transporting a compound into "high-grade cancer cells", characterized in that the compound to be transported is fused with BC2LCN lectin.
<17> An agent for killing "high-grade cancer cells" present in a test cell sample, containing a BC2LCN-toxin fusion, which is prepared by fusing BC2LCN lectin and a substance which is cytotoxic in cells, as an active ingredient.
<18> The killing agent according to <17>, in which the substance which is cytotoxic in cells is a toxic protein or a domain thereof having an ability to kill cells.
<19> An agent for killing cancer cells characterized by containing a "BC2LCN-toxin" fusion, which is prepared by fusing BC2LCN lectin and a substance which can exhibit cytotoxicity in cancer cells, as an active ingredient.
<20> The agent for killing cancer cells according to <19>, in which the substance which can exhibit cytotoxicity in cancer cells is a cell killing domain derived from a pseudomonas exotoxin A.
<21> The agent for killing cancer cells according to <20>, in which the cell killing domain derived from a pseudomonas exotoxin A is Domain I-III (PE38) represented by SEQ ID No: 3.
<22> The agent for killing cancer cells according to any one of <19> to <21>, in which the cancer cells are pancreatic cancer cells.
<23> The agent for killing cancer cells according to any one of <19> to <22>, in which the cancer cells include high-grade cancer cells or cancer cells having drug resistance.
<24> A composition for cancer treatment or therapy, characterized by containing a BC2LCN-toxin fusion, which is prepared by fusing BC2LCN lectin and a substance which can exhibit cytotoxicity in cancer cells, as an active ingredient, and a pharmacologically acceptable carrier.
<25> The composition according to <24>, in which the substance which can exhibit cytotoxicity in cancer cells is a cell killing domain derived from a pseudomonas exotoxin A.
<26> The composition according to <25>, in which the cell killing domain is Domain I-III (PE38) derived from pseudomonas exotoxin A represented by SEQ ID No: 3.
<27> The composition according to any one of <24> to <26>, in which the cancer is pancreatic cancer.
<28> The composition according to any one of <24> to <27>, in which the cancer is high-grade cancer having cancer stem cells or cancer having drug resistance.
<29> The composition according to any one of <24> to <28>, in which the composition for treating a cancer can be used together with or in combination with a therapeutic composition applicable to a known cancer.

The terms used in the present invention are defined as follows:
"Cancer" refers to a malignant tumor and sarcoma.

"Cancer stem cells" refer to cancer cells having a "replication competence" and "differentiation potential (reproducibility of tissue morphology)".

In the field of regenerative medicine, "Stemness" is defined as "replication competence" and "pluripotency". The "pluripotency" herein refers to the potential of a single cell to differentiate into many distinct types of cells.

As far as differentiation potential is concerned, cancer stem cells do not have the "pluripotency" as mentioned above. The "differentiation potential (reproducibility of tissue morphology)" that cancer stem cells have refers to a potency of cancer stem cells to differentiate into cells of the organ from which the cancer stem cells are derived. In other words, the "differentiation potential (reproducibility of tissue morphology)" that cancer stem cells have can be said to a potency of the cancer stem cells to reproduce morphology of cells of the organ from which the cancer stem cells are derived.

The cancer stem cells characteristically have a low differentiation degree described later. The cancer stem cells can be identified by using a known cell marker as an index. Examples of the cell marker include EPCAM (Non Patent Literature 4), CD24, CD44 (Non Patent Literature 5), CD133 (Non Patent Literature 6) and ERBB2 (Non Patent Literature 7). The cancer stem cells in the present invention do not include undifferentiated stem cells, more specifically, cells (for example, ES cells (embryonic stem cells) and iPS cells (induced pluripotent stem cells)) having replication competence as well as "pluripotency", which means the potential to differentiate into many distinct types of cells from a single cell.

As the index of determining a degree of malignancy of a cancer, many types of indexes from clinical indexes to histopathological indexes are used. Generally, indexes for a site of occurrence, tissue type and a differentiation degree of a cancer are frequently used. As to the site of occurrence, cancers developed in the gall bladder and pancreas are highly progressive, invasive and metastatic, and provide poor prognosis (5-year relative survival rate) and are classified into the most malignant cancer. In contrast, cancers developed in the prostate, breast gland and thyroid gland slowly progress, have a low metastatic property and good prognosis and are classified into the least malignant cancer. The 5-year relative survival rates of cancers of the uterine body, large intestine, cervix, stomach, ovary, lung, esophagus and liver decrease in this order, and malignancy thereof increases.

The tissue type is used for classifying cancers developed in the same tissue based on the types of cells. For example, lung cancers can be roughly classified into squamous cell carcinoma, adenocarcinoma, large cell carcinoma and small cell carcinoma.

The differentiation degree of a cancer refers to the degree of deviation (heteromorphy) from the normal tissue or cells. As the deviation of the structure and shape of a cancer tissue and cancer cells from those of a normal tissue and cells increases, the degree of malignancy (of the cancer tissue and cancer cells) is determined to be high. Regarding the structure, as ambiguity of the boundary of tissues increases or irregularity in alignment of the cells increases, the degree of malignancy is determined to be high. Regarding the shape of cancer cells, as the irregularity of the nucleus and cytoplasm increases, as the size of the cytoplasm, nucleus or nucleolus increases, the degree of staining of each item increases, or the number of nucleoli increases, the degree of malignancy is determined to be high.

In the present invention, "high-grade cancer cells" refer to malignant cancer cells determined in accordance with the index conventionally used and, in particular, refer to cancer cells having a high anchorage-independent proliferation potency and cancer cells having drug resistance and cancer stem cells.

The "anchorage-independent proliferation potency" means the ability of cells to survive and proliferate without adhering to an extracellular matrix (anchor). As this ability of the cells increases, the degree of malignancy is determined to be high. The anchorage-independent proliferation potency can be evaluated by measuring the number of proliferated cells, for example, by a colony formation test using a soft agar medium.

The "drug resistant cancer cells" refer to cells resistant to an anti-cancer agent used in a chemotherapy and surviving without being killed. In a cell population of drug resistant cancer cells, cancer stem cells are frequently contained. Examples of the anti-cancer agent include, but are not particularly limited to, an antimetabolic drug (e.g., 5-FU, gemcitabine hydrochloride), an alkylating agent (e.g., cyclophosphamide), a platinum-containing drug (e.g., oxaliplatin, cisplatin), a plant alkaloid (e.g., paclitaxel, docetaxel) and other molecularly targeted therapeutic agents (e.g., trastuzumab, imatinib, bevacizumab).

In the present invention, "cytotoxicity" includes an activity inducing cell death (apoptosis and necrosis). In addition, a wide variety of activities to suppress normal functions of cells such as cell division, proliferation and differentiation are included.

### Advantageous Effects of Invention

A technology for specifically detecting, separating or killing cancer cells is provided by the present invention. A fluorescently labeled BC2LCN lectin provided by the present invention can be used as an excellent cancer cell-specific labeled probe, which can specifically detect a sugar chain expressed on cancer cells. Cancer cells can be specifically and highly sensitively labelled by reacting the cancer cell-specific labelled probe of the present invention directly with, e.g., a pathological specimen. Accordingly, patient's cancer cells can be simply and effectively screened by using the cancer cell-specific labelled probe of the present invention. Speed-up of cancer diagnosis/treatment can be expected by application of the probe. Note that, the above effect can be obtained by using the probe in combination with a conventional technical means such as an antibody in the form of a kit. Due to this, they can be expected to act synergistically to detect and concentrate cancer cells.

The effect of the present invention is to provide a method for detecting cancer cells using BC2LCN lectin, particularly, a method for detecting cancer cells having drug resistance. BC2LCN lectin can be used as a cancer diagnostic agent for determining, e.g., onset of a cancer, a degree of malignancy of the cancer developed and prognosis of a cancer patient, and can be also used for confirming the effect of a cancer therapy. Further, if labelled BC2LCN lectin is previously administered to an area affected (with cancer) in a cancer tissue resection surgery, only a cancerization area can be clearly stained.

A toxin can be integrated to cancer cells by using the ability of BC2LCN lectin itself to migrate into cancer cells. Owing to this, a strong anti-cancer agent that can kill cancer cells can be provided. More specifically, an anti-cancer agent containing a BC2LCN lectin-toxin fusion, for example, BC2LCN-PE38, as an active ingredient, can be provided by the present invention. Particularly, the fusion of the invention is useful if it is used in combination with a known anti-cancer agent or as a therapeutic composition for patients affected with a drug-resistant cancer. Further, it is expected that if various research factors are allowed to incorporate into BC2LCN lectin, BC2LCN lectin can be used as a career for the factors in cancer research.

As another application, if micro RNA inhibiting a gene responsible for malignancy, in other words, a "transforming factor", is introduced in cancer cells, the cancer cells can be transformed into normal cells.

### Brief Description of Drawings

[Figure 1] The figure shows preparation of BC2LCN-PE38
[Figure 2A] The figure shows various cancer cell strains (MCF-7, T-47D, MDA-MB-157 and SK-MEL-28 cells) stained with FITC-labelled BC2LCN lectin.
[Figure 2B] The figure shows various cancer cell strains and a fibroblast strain (DU-145, LNaCap, PC-3 and TIG3 cells) stained with FITC-labelled BC2LCN lectin.
[Figure 3] The figure (A) shows flow cytometric analysis of various cancer cell strains and a fibroblast strain by Hilyte Fluor™ 647 labelled BC2LCN lectin; and (B) shows flow cytometric analysis of a fibroblast strain and a stem cell strain with Hilyte Fluor™ 647 labelled BC2LCN lectin.
[Figure 4] The figure shows Lectin array analysis using cell-membrane protein fractions of various cancer cells.
[Figure 5] The figure (A) shows staining of a breast cancer tissue section with HRP-labelled BC2LCN lectin; (B) shows staining of a lung cancer tissue section with HRP-labelled BC2LCN lectin; and (C) shows staining of brain tumor tissue section with HRP-labelled BC2LCN lectin.
[Figure 6] The figure shows BC2LCN lectin staining of tumor sites of a human clinical pancreatic cancer (specimen number 1).
[Figure 7] The figure shows BC2LCN lectin staining of tumor sites of a human clinical pancreatic cancer (specimen number 2).
[Figure 8] The figure shows BC2LCN lectin staining of tumor sites of a human clinical pancreatic cancer (specimen number 3).
[Figure 9] The figure shows BC2LCN lectin staining of tumor sites of human clinical large intestine cancer.
[Figure 10A] The figure shows lectin staining of various cases (stomach cancer, large intestine cancer, mammary gland cancer, liver cancer, pancreatic cancer, bile duct cancer, lung cancer) using labelled BC2LCN lectin.
[Figure 10B] The figure shows lectin staining of various cases (uterine body cancer, cervical cancer, prostate cancer, renal cancer, bladder cancer, testicular cancer, ovarian cancer, endocrine system cancer, another organ cancer) using labelled BC2LCN lectin.
[Figure 11A] The figure shows histopathological examination of BC2LCN lectin binding sites of breast cancer tissue sections using a human cancer tissue array.
[Figure 11B] The figure shows histopathological examination of BC2LCN lectin binding sites of lung cancer tissue sections using a human cancer tissue array.
[Figure 11C] The figure shows histopathological examination of BC2LCN lectin binding sites of brain tumor tissue sections using a human cancer tissue array.
[Figure 12A] The figure shows staining of human normal tissues (pancreas, spleen, breast, esophagus, skeletal muscle, salivary gland, gall bladder, thyroid gland, kidney, appendix, uterus, stomach) with HRP-labelled BC2LCN lectin.
[Figure 12B] The figure shows staining of human normal tissues (placenta, testes, palatine tonsil, large intestine, liver, brain, skin, small intestine, parathyroid gland, lymph nodes, fat, artery) with HRP-labelled BC2LCN lectin.
[Figure 12C] The figure shows staining of human normal tissues (bladder, thymus, lung, large intestine, heart, prostate, ovary, breast) with HRP-labelled BC2LCN lectin.
[Figure 13] The figure (A) shows the flow cytometric analysis (results) of a prostate cancer cell strain (PC-3) sorted with BC2LCN lectin; (B) shows the observation (results) of adherent culture of cells of a prostate cancer cell strain (PC-3) sorted with BC2LCN lectin; and (C) shows the verification result of proliferation of cells of a prostate cancer cell strain (PC-3) sorted with BC2LCN lectin.
[Figure 14] The figure (A) shows the observation (results) of cells of prostate cancer cell strain (PC-3) sorted with BC2LCN lectin and confirmed to be malignant; and (B) shows verification results of proliferation of cells of a prostate cancer cell strain (PC-3) sorted with BC2LCN lectin in a non-adherent culture.
[Figure 15] The figure shows sort results of cells of a prostate cancer cell strain (PC-3) by BC2LCN lectin and expression analysis of a cancer stem cell marker.
[Figure 16A] The figure shows the cytotoxicity of BC2LCN-ETA on breast cancer cell MCF-7 strain
[Figure 16B] The figure shows the cytotoxicity of BC2LCN-ETA on breast cancer cell T-47D strain
[Figure 16C] The figure shows the cytotoxicity of BC2LCN-ETA on breast cancer cell MDA-MB-157 strain.
[Figure 16D] The figure shows the cytotoxicity of BC2LCN-ETA on prostate cancer cell DU-145 strain.
[Figure 16E] The figure shows the cytotoxicity of BC2LCN-ETA on prostate cancer cell LNCaP strain.
[Figure 16F] The figure shows the cytotoxicity of BC2LCN-ETA on prostate cancer cell PC3 strain.
[Figure 16G] The figure shows the cytotoxicity of BC2LCN-ETA on fibroblast TIG3 strain.
[Figure 16H] The figure shows the cytotoxicity of BC2LCN-ETA on melanoma cell SK-MEL-28 strain.
[Figure 17] The figure shows internalization of FITC-labelled BC2LCN lectin into breast cancer cell MCF-7 strain.
[Figure 18] The figure shows morphology of cancer cells of mice having xenografts of six types of pancreatic cancer cell strains.
[Figure 19] The figure shows the degree (strength) of response to BC2LCN lectin to six types of pancreatic cancer cell strains obtained by a high-density lectin microarray.
[Figure 20] The figure shows binding of BC2LCN lectin to six types of pancreatic cancer cell strains analyzed by flow cytometry.
[Figure 21] The figure shows BC2LCN lectin staining of a tumor site of a Capan-1 transplanted mouse model.
[Figure 22] The figure shows BC2LCN lectin staining of a tumor site of a human pancreatic cancer transplanted mouse model (PC-3 line).
[Figure 23] The figure shows BC2LCN lectin staining of a tumor site of a human pancreatic cancer transplanted mouse model (PC-3 line) after treatment with GEM.
[Figure 24] The figure shows purification of BC2LCN-PE38.
[Figure 25] The figure shows the cytotoxicity of BC2LCN-PE38 to Capan-1.
[Figure 26] The figure shows the cytotoxicity of BC2LCN-PE38 to cancer cells in a Capan-1 transplanted mouse model.
[Figure 27] The figure shows the cytotoxicity of BC2LCN-PE38 to cancer cells in a Capan-1 transplanted mouse model; (A) effect of BC2LCN-PE38 on mouse body weight; (B) tumor suppression effect of BC2LCN-PE38; and (C) tumor size of a Capan-1 transplanted mouse model treated with BC2LCN-PE38.
[Figure 28] The figure shows observation of a pathology site of a Capan-1 transplanted mouse model treated with BC2LCN-PE38.
[Figure 29AB] The figure shows an antitumor effect of BC2LCN-PE38 in a pancreatic cancer patient-derived cancer cell transplanted (PDX) mouse model: (A) a change in tumor volume after treatment with BC2LCN-PE38; and (B) change in tumor size with time.
[Figure 29CD] The figure shows an antitumor effect of BC2LCN-PE38 in a pancreatic cancer patient-derived cancer cell transplanted (PDX) mouse model: (C) a change in tumor weight; (D) a change in body weight of a mouse.
[Figure 30A] The figure shows the dissemination state in the gastrointestinal tract (14 days after transplantation) of a disseminated metastasis model: Capan-1 intraperitoneally transplanted mouse.
[Figure 30B] The figure shows comparison of dissemination suppression effect of BC2LCN-PE38 in disseminated metastasis models: Capan-1 and SUIT-2 transplanted mice [Figure 30CD] The figure shows (C) a method for counting the number of cancer cells disseminated in the gastrointestinal tract of disseminated metastasis model: nude mice and (D) comparison of cancer cells disseminated in Capan-1 and SUIT-2 transplanted mice.
[Figure 31] The figure shows HRP-labelled BC2LCN lectin staining of an intestinal tissue piece of a disseminated metastasis model.
[Figure 32] The figure shows an antitumor effect of BC2LCN-PE38 by administration in blood of a Capan-1 transplanted mouse: (A) effect of reducing cancer cells disseminated in BC2LCN-PE38 administration group compared to a Control group and BC2LCN administration group; (B) change in body weight of mouse; (C) whole body condition and ascites storage in comparison with a Control group.
[Figure 33] The figure shows a toxicity experiment: mortality rates of cases where BC2LCN-PE38 (1 µg to 15 µg) was intraperitoneally administrated to mice (6 week female WT mice).
[Figure 34] The figure shows the detection results of Fucα1-2Galβ1-3GlcNAc/GalNAc in a culture supernatant of Capan-1.
[Figure 35] The figure shows the detection results of Fucα1-2Galβ1-3GlcNAc/GalNAc in the serum of a cancer transplanted mouse.
[Figure 36] The figure shows the detection results of Fucα1-2Galβ1-3GlcNAc/GalNAc in the serum of a patient before and after cancer removal.
[Figure 37] The figure shows the detection results of Fucα1-2Gaβ1-3GlcNAc/GalNAc in the serum of a patient before and after cancer removal.
[Figure 38] The figure shows the detection results of Fucα1-2Galβ1-3GlcNAc/GalNAc in the serum of a patient with large intestine cancer.

### Description of Embodiments

### 1. Cancer cell detection reagent and cancer cell separation reagent

### (1) BC2LCN lectin

The reagent for use in detecting cancer cells and reagent for separating cancer cells according to the present invention contain BC2LCN lectin. BC2LCN lectin binds to "Fucα1-2Galβ1-3GlcNAc (H type 1 sugar chain)" and "Fucα1-2Galβ1-3GalNAc (H type 3 sugar chain)" (hereinafter both are collectively referred to also as "Fucα1-2Galβ1-3GlcNAc/GalNAc) present on the surface of cancer cells, with high affinity and reacts with the cancer cells with high specificity. Further, BC2LCN lectin also binds to free "Fucα1-2Galβ1-3GlcNAc/GalNAc", separated from the cell surface of cancer cells in a cancer tissue or cultured cancer cells and present in a body fluid or in a culture supernatant. In the present invention, the term "cancer cell-specific probe" refers to a probe containing "BC2LCN lectin".

"BC2LCN lectin" is lectin derived from gram-negative bacterium (Burkholderia cenocepacia) and corresponds to an N terminal domain (GenBank/NCBI-GI Registration No: YP_002232818) of the protein called BC2L-C (Non Patent Literature 3). It is known that BC2LCN lectin forms a trimer and recognizes a sugar chain by sandwiching it between two subunits.

From analysis using a sugar chain array, it has been found that BC2LCN lectin recognizes Fucα1-2Galβ1-3GlcNAc/GalNAc, which is known as an undifferentiated sugar chain marker.

Since BC2LCN lectin contains no sugar chain, BC2LCN lectin can be produced in a large amount by a transformed bacterium. More specifically, BC2LCN lectin is produced by optimizing BC2LCN gene encoding the amino acid sequence (SEQ ID No: 1) of GenBank/NCBI-GI Registration No: YP_002232818 (Genome ID: 206562055) for a host and allowing the gene to express in e.g., transformed E. coli; and can be purified by a customary protein purification means. Hereinafter, recombinant BC2LCN used in embodiments of the present invention will be referred to also as "rBC2LCN".

In the sugar-chain structure of "Fucα1-2Galβ1-3GlcNAc", the hydroxyl group at the 4-position of GlcNAc may be substituted with a monosaccharide (preferably, fucose), or branched or non-branched oligosaccharide chain (preferably, a sugar chain consisting of 2 to 5 saccharides). Since the sugar chain, which serves as a membrane component on the surface of cancer cells, has a structure where GlcNAc binds to a non-reducing end such as a glycoprotein, a glycolipid or a sugar at the 1-position, a sugar chain secreted in a body fluid or in a culture supernatant may have a structure where GlcNAc binds to an OH group or a non-reducing end of another sugar, a protein or a lipid, or another molecule at the 1-position. More specifically, the sugar chain structure can be represented by the following formula (Formula 1): 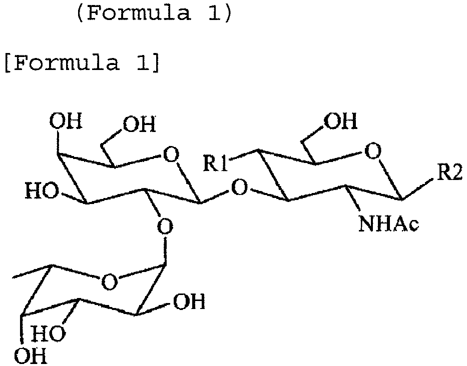 where R1 represents an OH group or a sugar chain, for example, 4αFuc group; R2 represents an OH group or a sugar chain, a protein, a lipid or another molecule.

Similarly, in the sugar chain structure of "Fucα1-2Galβ1-3GalNAc", the hydroxyl group at the 1-position of GalNAc may be substituted with a branched or non-branched oligosaccharide chain (preferably a sugar chain consisting of 2 to 5 saccharides). Since the sugar chain, which serves as a membrane component on the surface of cancer cells, has a structure where GalNAc binds to a non-reducing end such as a glycoprotein, a glycolipid or a sugar at the 1-position, a sugar chain secreted in a body fluid or in a culture supernatant may have a structure where GalNAc binds to an OH group or a non-reducing end of another sugar, a protein or a lipid, or another molecule at the 1-position. More specifically, the sugar chain structure can be represented by the following formula (Formula 2): 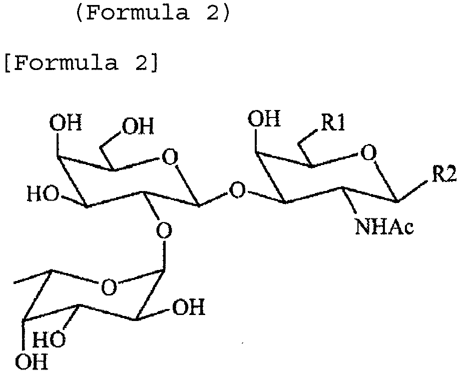 where R1 represents an OH group or a sugar chain, for example, Galβ1-4Glc group; R2 represents an OH group or a sugar chain, a protein, a lipid or another molecule.

In the present invention, "BC2LCN lectin" may be a part of the fragment represented by SEQ ID No: 1 or a fusion protein prepared by adding an amino acid sequence of a tag sequence or a protein label thereto as long as the partial fragment or the fusion protein can specifically recognize the sugar chain structure of Fucα1-2Galβ1-3GlcNAc/GalNAc. Alternatively, "BC2LCN lectin" may have a deletion, substitution, insertion or addition of a less than 10 % of amino acids in the full length sequence represented by SEQ ID No: 1. In short, the term "BC2LCN lectin" used in the present invention includes a BC2LCN lectin variant.

BC2LCN lectin may not necessarily have a full length amino acid sequence represented by SEQ ID No: 1. Even if BC2LCN lectin may partly have a deletion, substitution, insertion and addition of amino acids in the sequence represented by SEQ ID No: 1, as long as it specifically recognizes Fucα1-2Galβ1-3GlcNAc/GalNAc, BC2LCN lectin is sufficiently used.

More specifically, BC2LCN lectin can be defined as follows:
"a protein comprising the amino acid sequence represented by SEQ ID No: 1 or an amino acid sequence having a deletion, substitution, insertion or addition of one or several amino acids in the amino acid sequence and specifically recognizing a sugar chain structure of Fucα1-2Galβ1-3GlcNAc or Fucα1-2Galβ1-3GalNAc".

Note that, several amino acids used herein refer to 20 or less, preferably 10 or less, more preferably 5 or less, and particularly preferably, 2 amino acids.

### (2) Method for labelling BC2LCN lectin

In the present invention, BC2LCN lectin is labelled with e.g., a fluorescent tag, an enzyme, a nucleic acid chain, biotin or magnetic beads in accordance with a routine method. A preferable label substance varies depending upon the use. For example, a fluorescent label is preferable for staining cells and flow cytometric analyses. Examples of a preferable fluorescent dye used herein include "Cy3", "Cy5", "FITC", "Hilyte Fluor™ 647", "phycoerythrin" and "allophycocyanin". In labelling BC2LCN lectin, a method of Hohsaka et al. (Iijima et al. (2009) ChemBioChem, 10, 999-1006) known as a method for introducing a fluorescent labelled amino acid into an arbitrary site of an amino acid sequence, is used. In this manner, a mutant having a fluorescent labelled amino acid introduced in a predetermined site of BC2LCN lectin can be produced.

For use in separating cells, other than a fluorescent dye, magnetic beads are useful as a label. For example, if the ThermoFisher method (https://www.thermofisher.com/jp/ja/home/clinical/diagnos tic-development/molecular-diagnostic-test-development/bead-based-ivd-assays/customized-dynabeadsoem-supply.html) is used, magnetic bead-labelled BC2LCN lectin can be prepared.

In confirming distribution in a large tissue through which light is not transmitted, an enzyme such as "horseradish peroxidase", "alkaline phosphatase" and "detection system using a biotin-avidin reaction" can be used. At this time, if an enzyme or biotin activated with an NHS group or a maleimide group by using, e.g., a method of Dojindo-sha (http://dominoweb.dojindo.co.jp/goodsr7.nsf/ByItemLInfo/0 8?OpenDocument), a primary amino group (NH₂ group) or a thiol group (SH group, sulfhydryl group) of BC2LCN lectin can be labeled.

### 2. Cancer cell detection method and cancer cell separation method

### (1) Test sample

In the method for detecting and separating cancer cells according to the present invention, a test sample can be a tumor tissue or a peripheral tissue thereof, which is excised out from an organ or a tissue and which possibly contains cancer cells (BC2LCN-positive cancer cells), on the surface of which Fucα1-2Galβ1-3GlcNAc/GalNAc is expressed. Furthermore, the test sample may be a tissue sample or a cell sample, which is derived from a biopsy material and which possibly contains a BC2LCN-positive cancer cells. Moreover, the test sample may be a body fluid sample (e.g., a sample derived from blood such as whole blood, serum and plasma; interstitial fluid, lymph fluid, saliva, gastric juice, urine, cerebrospinal fluid and tissue extract) taken from a test individual suspected to have BC2LCN-positive cancer cells. Of them, a body fluid sample, particularly a blood-derived sample, is preferable, in consideration of burden on, e.g., a test individual.

Cancer cells contained in a tumor tissue are not limited to cancer cells originated from the tumor tissue and may be cancer cells spread from another organ or tissue.

BC2LCN-positive cancer cells include cancer cells turned cancerous in a living body, cancer cells separated from a living body and cancer cells separated from a living body and cultured.

Examples of the cancer cells include cells of a cancer such as tongue cancer, laryngeal cancer, pharyngeal cancer, esophagus cancer, lung cancer, stomach cancer, liver cancer, bile duct cancer, pancreatic cancer, large intestine cancer, kidney cancer, bladder cancer, urothelial cancer, prostatic cancer, uterine cancer, ovarian cancer, testicular cancer, breast cancer, thyroid cancer, leukemia, malignant lymphoma, plasmacytoma, myeloma, melanoma; and cells of a malignant tumor such as brain tumor and malignant sarcoma.

BC2LCN lectin has responsiveness to an epithelial cancer, particularly digestive-system epithelial cancer and breast cancer. Of them, it has been found that BC2LCN lectin exhibits highly responsiveness to large intestine cancer and bile duct cancer (see Examples).

Accordingly, as the cancer cells, particularly, epithelial cancers of tongue cancer, laryngeal cancer, pharyngeal cancer, esophagus cancer, lung cancer, stomach cancer, duodenal cancer, liver cancer, bile duct cancer, gall bladder cancer, pancreatic cancer, large intestine cancer, kidney cancer, bladder cancer, urothelial cancer, prostatic cancer, uterine cancer, ovarian cancer, breast cancer and thyroid cancer are preferable; digestive-system epithelial cancers of tongue cancer, pharyngeal cancer, esophagus cancer, stomach cancer, duodenal cancer, liver cancer, bile duct cancer, gall bladder cancer, pancreatic cancer, and large intestine cancer, and breast cancer are more preferable; and large intestine cancer and bile duct cancer are particularly preferable.

It has been found that BC2LCN lectin has highly responsiveness to high-grade cancer cells, particularly, drug resistant cancer cells and cancer stem cells (see, Examples).

Accordingly, as the cancer cells, cells of a high-grade cancer such as pancreatic cancer, bile duct cancer, gall bladder cancer and lung cancer, can be mentioned. Of these cancer cells described above, cancer cells having drug resistance or cancer stem cells can be mentioned.

As a method for culturing test cells, adherent (cell) culture performed in a culture vessel is generally employed. The adherent culture is performed by placing test cells on a plastic dish uncoated or coated with feeder cells or a coating agent such as an extracellular matrix extract or non-coating plastic dish or by attaching test cells onto, e.g., surface of beads and suspending the beads in the culture vessel. Alternatively, a floating cell culture method in which test cells are directly suspended in a culture solution and a culture method performed in the soft agar without using an anchor may be employed.

### (2) Procedure for cancer cell detection method and cancer cell separation method

The method for detecting cancer cells according to the present invention has a step (A) of bringing BC2LCN lectin into contact with a test sample and step (B) of determining the presence or absence or the amount of a sugar chain (Fucα1-2Galβ1-3GlcNAc/GalNAc) having a BC2LCN lectin binding activity in the test sample.

The method for separating cancer cells according to the present invention has a step (A) of bringing BC2LCN lectin into contact with a test sample and a step (C) of separating cells to which BC2LCN lectin binds from cells to which BC2LCN lectin does not bind.

### (2-1) Step (A)

When a cell sample is used as a test sample, step (A) can be carried out as follows. In the case of test cells cultured by attaching the test cells to a base material in the culture vessel, if a labeled-probe solution containing BC2LCN lectin is supplied to a (culture) solution covering the test cells, the labeled-probe binds to Fucα1-2Galβ1-3GlcNAc/GalNAc without being influenced by the presence or absence of, e.g., feeder cells. In this manner, cancer cells are labelled.

Even in the case of test cells cultured while being suspended, if the labeled-probe solution is supplied to the culture solution, the labeled-probe binds to Fucα1-2Galβ1-3GlcNAc/GalNAc. In this manner, cancer cells can be labelled.

When a tissue sample is used as a test sample, step (A) can be carried out as follows. A labeled-probe solution containing BC2LCN lectin is brought directly into contact with a tissue piece or a tissue piece chemically fixed. In this manner, the labeled-probe binds to Fucα1-2Galβ1-3GlcNAc/GalNAc to label cancer cells in the tissue piece.

Alternatively, a (pathological) section is prepared by slicing a tissue sample directly or after chemical fixation, into thin sections in accordance with a routine method and attaching each of the sections onto a slide glass, and then, the labeled-probe solution may be brought into contact with the section. In this manner, the labeled-probe binds to Fucα1-2Galβ1-3GlcNAc/GalNAc to label cancer cells in the tissue section.

Alternatively, a tissue sample is treated with an enzyme to dissociate cells to prepare a cell sample. The cell sample may be treated in accordance with the aforementioned step of the case of using a cell sample as a test sample.

When a body fluid sample is used as a test sample, step (A) can be carried out as follows.

A labeled-probe solution containing BC2LCN lectin is brought directly into contact with the body fluid sample.

Alternatively, a body fluid sample is brought into contact with a support on which a cancer cell-specific probe is immobilized. In this manner, Fucα1-2Galβ1-3GlcNAc/GalNAc in the body fluid sample is trapped by the probe immobilized on the support. As the support, e.g., a support generally used such as a plate, slide glass and membrane may be used.

### (2-2) Step (B)

When a cell sample is used as a test sample, step (B) can be carried out as follows. In either one of cases where test cells are subjected to adherent culture and suspension culture, the cancer cell-specific labeled-probe solution of the present invention is added to a culture solution of test cells, and thereafter, the amount of the label on the surface of the test cells is measured. In this manner, the presence or absence or the amount of Fucα1-2Galβ1-3GlcNAc/GalNAc can be determined. Consequently, the presence of cancer cells can be accurately detected and evaluated.

When a tissue sample is used as a test sample, step (B) can be carried out as follows.

A tissue sample (tissue piece) is used as it is or a tissue section is prepared from the tissue sample. The tissue piece or section is brought into contact with the cancer cell-specific labeled-probe solution of the present invention. Thereafter, the amount of the label on the surface of the tissue piece or tissue section is measured. In this manner, the presence or absence or the amount of Fucα1-2Galβ1-3GlcNAc/GalNAc can be determined. Consequently, the presence of cancer cells can be accurately detected and evaluated.

If necessary, the liquid may be exchanged with, e.g., a buffer solution or physiological saline. If so, the influence of, e.g., medium components, can be easily removed. Since the amount of a label of cancer cells attached to dead cells can be measured in the same manner as in living cells, the cells, which are chemically fixed in advance with, e.g., formalin, are more easily handled.

When a body fluid sample is used as a test sample, step (B) can be carried out as follows.

After Fucα1-2Galβ1-3GlcNAc/GalNAc bounded by labeled-probe is separated by, e.g., an electrophoretic method or HPLC known in the art, the amount of a label is measured. In this manner, the presence or absence or the amount of Fucα1-2Galβ1-3GlcNAc/GalNAc can be determined.

When a body fluid sample used as a test sample is directly brought into contact with a labeled-probe solution containing BC2LCN lectin, in step (B), measurement must be performed after free labeled-probe is separated from the labeled-probe bound Fucα1-2Galβ1-3GlcNAc/GalNAc. Examples of the separation method include chromatography, high performance liquid chromatography, electrophoresis, capillary electrophoresis, capillary chip electrophoresis and a method using an automated immunoassay apparatus such as LiBASys (manufactured by Shimadzu Corporation). As the specific condition for the separation method, any condition may be employed as long as labeled-probe bound Fucα1-2Galβ1-3GlcNAc/GalNAc can be separated. Conditions other that this may be set in accordance with those of a method known in the art. For example, if HPLC is used, separation can be made in accordance with the method described in Anal. Chem. 65, 5, 613-616 (1993) or Japanese Patent Laid-Open No. 9-301995. If capillary electrophoresis is used, separation can be made in accordance with the method described in, e.g., J. Chromatogr. 593 253-258 (1992), Anal. Chem. 64 1926-1932 (1992) or WO2007/027495. If, e.g., LiBASys is used as the automated immunoassay apparatus, separation can be made in accordance with the method described in Biological Sample Analysis vol. 22, No. 4, 303-308 (1999). After separation, the amount of a label in labeled-probe bound Fucα1-2Galβ1-3GlcNAc/GalNAc is measured. In this manner, the presence or absence or the amount of Fucα1-2Galβ1-3GlcNAc/GalNAc can be determined.

When a body fluid sample used as a test sample is brought into contact with a support to which a cancer cell-specific probe is immobilized, step (B) can be carried out as follows.

To the support by which Fucα1-2Galβ1-3GlcNAc/GalNAc is trapped, a solution of a labelled antibody capable of binding to a complex of BC2LCN lectin-Fucα1-2Galβ1 3GlcNAc/GalNAc or labelled lectin (for example, R-10G labelled antibody) is further brought into contact. Thereafter the amount of label on the surface of the support is measured. In this manner, the presence or absence or the amount of Fucα1-2Galβ1-3GlcNAc/GalNAc can be determined.

Consequently, the presence of cancer cells in a test individual from which the body fluid sample was taken can be accurately detected and evaluated.

### (2-3) Step (C)

When a cell sample is used as a test sample, step (C) can be carried out as follows.

Suspended cells in a solution after step (A) can be directly subjected to isolation of cancer cells by a cell sorter or a magnetic cell separator. The cancer cell-specific labeled-probe of the present invention has high specificity and affinity sufficient to label cells, even if the cells are suspended in a solution. Such properties (specificity and affinity) are extremely advantageous in minimizing adverse effect on cancer cells particularly when they are isolated and in easily isolating cancer cells.

As mentioned above, the cancer cell-specific labeled-probe of the present invention can stain not only living cancer cells but also cancer cells killed by chemical fixation; as well as not only adherent cancer cells but also suspended cancer cells. The "suspended cancer cells" used herein include "cancer cells obtained by suspension culture". Other than these cells, "cancer cells obtained by treating cancer cells prepared in adherent culture with a proteolytic enzyme and suspending them" are both included. In the case of cells in a culture solution, cells in a buffer solution from which medium components are removed and cells in a solution such as physiological saline, are both included. In contrast, "adherent cancer cells" used herein include cancer cells obtained by adherent culture after they are attached on a base material such as a dish and cancer cells obtained by suspension culture after they are attached on a base material such as beads.

In the case of cancer cells present in a sample taken from a living body or a dead body, the sample may or may not be chemically fixed. Furthermore, cancer cells dissociated by enzymatic treatment and suspended in, e.g., a buffer may be included. Moreover, cancer cells which are obtained by freezing or embedding the sample in, e.g., resin, slicing and attaching it to a base material such as a slide glass, are also included.

### (2-4) Specific Procedure for detecting cancer cells attached to base material

The cancer cell-specific probe of the present invention can be applied to the case of detecting cancer cells present in a group of cells, which are cultured and present on a base material such as beads, hollow filaments or a flat plate; or attached onto a base material.

In the case, a cancer cell-specific labeled-probe is added in a solution in which the base material is present. The "solution" herein may be a culture solution, a buffer solution from which medium components are removed, physiological saline or the like. Cancer cells is generally detected by analyzing the responsiveness of the probe to Fucα1-2Galβ1-3GlcNAc/GalNAc specifically expressed on the surface of cancer cells, by use of e.g. a fluorescence microscope or ELISA.

According to such an analysis method, at the time of biopsy, a sample from which a label such as fluorescence is not detected (the same level (value) as in background is obtained) can be evaluated as a sample in which cancer cells are not present. In order to control quality (maintenance) of cancer cells for e.g., research use, an aliquot of the cancer cells can be sampled periodically or at the time of need and subjected to measurement of intensity of label such as intensity of fluorescence by the cancer cell-specific labeled-probe of the present invention.

### (2-5) Specific Procedure for detecting cancer cells suspended in solution

The cancer cell-specific probe of the present invention can be applied to the case of detecting cancer cells in a solution.

In the case, a cancer cell-specific labeled-probe is added in the solution. The "solution" herein may be, e.g., a culture solution or a buffer solution from which medium components are removed and physiological saline.

If the cancer cell-specific labeled-probe of the present invention is used, cancer cells alone can be directly tagged with a fluorescent label. Thus, flow cytometry can be applied.

More specifically, a tissue taken by, e.g., biopsy is enzymatically treated to dissociate cells, which are reacted with a cancer cell-specific labeled-probe and subjected to flow cytometry analysis using a FACS instrument. In this way, even if the amount of sample is small, a diagnosis (cell detection) system of determining the presence or absence of cancer cells without fail can be provided.

Alternatively, BC2LCN lectin is immobilized onto a transparent base material such as a slide glass. A cancer cell-containing test sample in which cancer cells are suspended in a solution is directly used or diluted or previously concentrated into a protein fraction alone, and then, labeled with, e.g., "Cy3-NHS ester" and reacted with the immobilized BC2LCN lectin. The binding may be determined by, e.g., a plate reader, a fluorescent scanner and/or an evanescent wave excitation fluorescence detection system.

### (2-6) Specific Procedure for separating cancer cells

Cancer cells alone can be isolated by combination use of flow cytometry and a cell sorter, more specifically, by a flow cytometer provided with a cell sorter.

More specifically, a tissue taken by, e.g., biopsy is enzymatically treated to dissociate cancer cells, which are reacted with a cancer cell-specific fluorescent-labeled-probe and subjected to flow cytometry. In this manner, cancer cells can be separated while keeping alive.

Alternatively, if a magnetic bead labelling method is employed, a tissue taken by, e.g., biopsy is enzymatically treated, to dissociate cancer cells, which are reacted with a cancer cell specific magnetic bead-labeled-probe and then subjected to a magnetic cell separator. In this manner, cancer cells alone can be separated.

### 3. Kit or apparatus for detecting the presence or absence of cancer cells

If a kit or apparatus is constituted of a cancer cell-specific probe (1) of the present invention together with the following means (2) and (3), a kit or apparatus for detecting the presence or absence of cancer cells can be obtained.
(1) BC2LCN lectin,
(2) a labelling agent,
(3) a means or device for detecting the label.

According to the above items (1) and (2), high-grade cancer cell-specific labeled-probe consisting of BC2LCN lectin labeled with, e.g., a fluorescent dye, an enzyme or biotin, is provided. A kit or apparatus can be prepared by using lectin previously bound to a labelling agent in place of the items (1) and (2), in combination with the item (3).

As the item (3), for example, a fluorescence microscope or a plate reader is used if a fluorescent label is used; whereas, e.g., an image analyzer is used if an enzyme label or a biotin label is used.

The kits or apparatuses mentioned above may each contain a labelled antibody binding to BC2LCN-positive sugar chain complex (complex of BC2LCN lectin-Fucα1-2Galβ1-3GlcNAc/GalNAc) and/or an antibody binding to the BC2LCN-positive sugar chain complex.

The apparatus may have a means (for example, automatic dispenser) for bringing a cancer cell-specific labeled-probe into contact with a cell surface or a tissue surface, or for adding the probe in a body fluid sample. Owing to the means, analysis for cancer cells can be automatically carried out. However, since the operation performed by the means can be manually performed, the means is not essentially provided.

### 4. Kit or apparatus for separating cancer cells

If a kit or apparatus is constituted of the following means (1) and (2), only high-grade cancer cells expressing Fucα1-2Galβ1-3GlcNAc/GalNAc on the surface can be isolated.
(1) labeled BC2LCN lectin,
(2) means or device for detecting a label and separating the labeled cells.

The above item (1) is a cancer cell-specific labeled-probe (BC2LCN lectin labeled with, e.g., fluorescent dye, an enzyme, biotin or magnetic beads).

The above item (2) is a means or device for detecting and separating a label such as a fluorescent label and magnetic bead label and separating the labeled cells. The item (2) is, for example, a flow cytometer provided with a cell sorter or a magnetic cell separator.

The apparatus may have a means (for example, automatic dispenser) for bringing a cancer cell-specific labeled-probe into contact with a cell surface or tissue surface, or for adding the probe in a body fluid sample. However, since the operation performed by the means can be manually performed, the means is not essentially provided.

According to the cancer cell-specific labeled-probe of the present invention, since cancer cells can be separated from normal cells, the cancer cells can be isolated. Further, BC2LCN lectin has high responsiveness particularly to high-grade cancer cells, and thus, high-grade cancer cells can be separated from low-grade cancer cells or normal cells.

### 5. Methods for diagnosing cancer and determining therapeutic effect and method for collecting data for these methods

In the present invention, the presence or absence of expression of a sugar chain having a BC2LCN lectin binding activity in a test sample taken from a test individual or the expression level of the sugar chain are measured to determine the presence or absence of cancer cells. Based on the results, whether the test individual is affected with a cancer or not or the prognosis and therapeutic effect of the cancer are determined. The present invention includes a method for detecting a cancer, determining a degree of malignancy of cancer or a degree of drug resistance acquisition, determining prognosis and therapeutic effect, an examination reagent and an examination kit

The test sample to be subjected to the methods for diagnosing a cancer and determining a therapeutic effect, according to the present invention is the same as those used in the cancer cell detection method and cancer cell separation method mentioned above. The test individual refers to an individual who has been determined or not determined to have a cancer; and an individual who already underwent a therapy such as a surgery or administration of an anti-cancer agent is also included. In the former case, it is possible to determine whether or not an individual is affected with a cancer; at the same time, whether or not the cancer is malignant or whether or not the cancer is drug resistant. In the latter case, it is possible to determine prognosis or therapeutic effect.

The method for diagnosing a cancer according to the present invention includes a step of measuring the amount of a sugar chain having a BC2LCN lectin binding activity in a test sample of a test individual suspected to be affected with a cancer. In this method, if the amount is significantly high compared to the amount of a healthy person, it is determined that the test individual is affected with a cancer or the possibility that the test individual is affected with a cancer is high.

The method for diagnosing a cancer according to the present invention includes a step of measuring the amount of a sugar chain having a BC2LCN lectin binding activity in a test sample of a test individual affected with a cancer. In this method, if the amount is significantly high compared to the amount of a healthy person or a patient with a low-grade cancer, it can also be determined that the prognosis of the test individual is poor.

The method for determining the therapeutic effect on cancer according to the present invention includes the steps of: measuring the amount of a sugar chain having a BC2LCN lectin binding activity in a test sample of a test individual to which a cancer therapy is applied; and comparing the amount obtained above to the amount of a sugar chain having a BC2LCN lectin binding activity of the test sample of the test individual previously determined before the therapy. In this method, if the amount after the therapy is significantly low compared to the amount before the therapy, it is determined that the therapy is effective.

### <Method for diagnosing cancer using tissue section>

A tumor tissue taken from an organ or tissue of a test individual affected with a cancer is fixed with formalin, embedded in paraffin and sectioned to obtain tissue sections. The obtained tissue sections were stained with BC2LCN lectin labelled with, e.g., an enzyme or a fluorescence dye. Tissue images of the tissue sections are observed by a microscopy. The presence of a cancer can be detected by checking staining, and quantitatively determined by use of lectin array, flow cytometry or ELISA, and, in addition, a degree of malignancy of the cancer can be also determined.

### <Method for diagnosing cancer using biopsy material>

Whether or not cancer cells are present in a biopsy material can be checked by reacting a fluorescent labeled BC2LCN lectin-containing solution with a tissue sample or a cell sample. The content (ratio) of cancer cells in the biopsy material can be determined. Based on the fluorescence intensity measured, the degree of malignancy of the cancer cells in the biopsy material can be evaluated. At this time, a tissue sample or a cell sample may be fixed; however, if a cell sample not fixed is fluorescently labeled and flow cytometry is applied, the degree of malignancy of cancer cells can be more quantitatively evaluated. If a cell sorter is used in combination, the proportion of cancer cells fluorescently labeled in the biopsy sample can be accurately determined. After a membrane protein fraction is separated from a tissue sample or a cell sample by a known method, the protein fraction can be suspended in a buffer solution or physiological saline and then subjected to a measuring step. In this case, the measuring method is carried out in accordance with the measuring method using a body fluid sample as descried next.

### <Measuring method using body fluid sample>

When a body fluid sample such as blood is used as a test sample, the body fluid sample can be directly used without passing through a purification step, or diluted or concentrated into a protein fraction alone in advance and then subjected to a measuring step.

BC2LCN lectin is immobilized onto a transparent base material such as a slide glass. A solution of a test sample labeled with, e.g., "Cy3-NHS ester" is allowed to directly react with BC2LCN lectin. Binding between them is determined by an evanescent wave excitation fluorescence detection system.

Alternatively, BC2LCN lectin is immobilized to a support such as an ELISA plate, magnetic beads or a filter. To the support, a test sample labeled with e.g. an enzyme, a fluorescent dye or biotin is allowed to react. The binding strength between them can be determined by measuring, e.g., color development, emission of light, or fluorescence. At this time, sandwich assay can be employed. In this assay, a labelled antibody or labelled lectin binding to a BC2LCN-positive sugar chain complex is added in the solution obtained after the reaction with a test sample, and then allowed to react. In particular, if "lectin-lectin sandwich method" or "lectin-antibody sandwich method" is used, measurement can be more sensitively performed. Of them, a method using lectin and an antibody is preferable. Since BC2LCN lectin is extremely sensitive, even if the amount of BC2LCN-positive sugar chain in a test sample is as low as the order of picomole (pM) or nanomolar (nM) level in reacting the test sample solution to a BC2LCN lectin-immobilized support, the presence or absence of the sugar chain can be determined. Because of this, if the serum is used as a test sample, measurement is successfully made even if about 0.1 to 10 µl of the serum is taken.

A test sample is reacted with a support, onto which an antibody against a known cancer marker or lectin is immobilized, and then, BC2LCN lectin labelled with, e.g., an enzyme, biotin or a fluorescent dye, is allowed to react. If so, detection can be made by a known method such as fluorescent staining, flow cytometry, ELISA and lectin blotting.

In the present invention, as the antibody to be used in the aforementioned method, an antibody against BC2LCN lectin or a labeled antibody capable of binding to a complex of BC2LCN lectin-Fucα1-2Galβ1-3GlcNAc/GalNAc (BC2LCN-positive sugar chain complex) can be mentioned. As such an antibody, an IgG antibody produced by hybridoma R-10G (accession number: FERM BP-11301) is preferably used.

In the present invention, the term "antibody" includes a "functional fragment of an antibody". The "functional fragment of an antibody" refers to a partial fragment of an antibody having a binding activity to an antigen and includes, e.g., Fab, F(ab')2 and scFv. Furthermore, Fab', which is a monovalent fragment of a variable region of an antibody obtained by treating F(ab')2 under reducing conditions, is included as the functional fragment of an antibody. The functional fragment of an antibody is not limited to these molecules as long as it has a binding ability to an antigen. Not only a full-length antibody protein molecule treated with an appropriate enzyme but also a protein produced in an appropriate host cell by using an antibody gene modified by genetic engineering is included as the functional fragment.

The procedure of the method for detecting the presence or absence of cancer cells in the present invention includes a complex-formation step of forming a complex constituted of lectin, Fucα1-2Galβ1-3GlcNAc/GalNAc and an antibody, by bringing a test sample, lectin and the antibody into contact with each other and a step of detecting the complex. Now, the case where BC2LCN lectin and R-10G antibody are used will be more specifically described.

In the complex-formation step, BC2LCN lectin and R-10G antibody are simultaneously brought into contact with a test sample; however, it is more preferable that a test sample is brought into contact with BC2LCN lectin, and thereafter, R-10G antibody is allowed to react. More specifically, the complex-formation step preferably consists of a first step of bringing a test sample into contact with the lectin to form a first complex (complex of BC2LCN lectin-Fucα1-2Galβ1-3GlcNAc/GalNAc) consisting of BC2LCN lectin and Fucα1-2Galβ1-3GlcNAc/GalNAc contained in the test sample; and a second step of bringing the first complex into contact with R-10G antibody to form a second complex (BC2LCN lectin-Fucα1-2Galβ1-3GlcNAc/GalNAc-R-10G antibody) which is constituted of BC2LCN lectin, Fucα1-2Galβ1-3GlcNAc/GalNAc and R-10G antibody.

The complex-formation step may be carried out in a homogeneous method without separating B/F or in a heterogenous method by separating B/F by using an insoluble carrier.

The homogeneous method is carried out, for example, in accordance with the following procedure.

### <Method 1>

(i) A test sample, free BC2LCN lectin (not immobilized to an insoluble carrier) and free R-10G antibody (not immobilized to an insoluble carrier) are brought into contact with each other to form a complex of BC2LCN lectin and Fucα1-2Galβ1-3GlcNAc/GalNAc in the sample and R-10G antibody.
(ii) The mount of the complex is measured.
(iii) The amount of Fucα1-2Galβ1-3GlcNAc/GalNAc in the sample is determined based on the resultant amount of the complex.

### <Method 2>

(i) A test sample and free BC2LCN lectin (not immobilized to an insoluble carrier) are brought into contact with each other to form complex-1 of Fucα1-2Galβ1-3GlcNAc/GalNAc in the sample and BC2LCN lectin.
(ii) Complex-1 (not immobilized to an insoluble carrier) and free R-10G antibody are into contact with each other to form complex-2 of BC2LCN lectin, Fucα1-2Galβ1-3GlcNAc/GalNAc in the sample and R-10G antibody.
(iii) The amount of complex-2 is measured.
(iv) The amount of Fucα1-2Galβ1-3GlcNAc/GalNAc in the sample is determined based on the resultant amount of complex-2.

### <Method 3>

(i) A sample, free BC2LCN lectin and free R-10G antibody tagged with a label substance are brought into contact with each other to form a complex of BC2LCN lectin, Fucα1-2Galβ1-3GlcNAc/GalNAc in the sample and the labelled R-10G antibody.
(ii) The amount of the label substance in the complex is measured.
(iii) The amount of Fucα1-2Galβ1-3GlcNAc/GalNAc in the sample is determined based on the resultant amount of the label substance.

### <Method 4>

(i) A sample and free BC2LCN lectin are brought into contact with each other to form complex-1 of Fucα1-2Galβ1-3GlcNAc/GalNAc in the sample and BC2LCN lectin.
(ii) Complex-1 and free R-10G antibody tagged with a label substance are brought into contact with each other to form complex-2 of complex-1 and labelled R-10G antibody.
(iii) The amount of the label substance in complex-2 is measured.
(iv) The amount of Fucα1-2Galβ1-3GlcNAc/GalNAc in the sample is determined based on the resultant amount of the label substance.

The amounts of test samples, and the amounts (concentrations) of lectin and antibody to be reacted with the test samples are appropriately set depending on the type of cells, the requisite measurement sensitivity, the measuring method and the apparatus to be used.

B/F separation using an insoluble carrier is carried out by bringing, for example, BC2LCN lectin bound to an insoluble carrier and an R-10G antibody not bound to the insoluble carrier are brought into contact with a test sample to form a complex.

More specifically, B/F separation is carried out by a method including a first step of bringing a test sample and BC2LCN lectin bound to an insoluble carrier into contact with each other to form a first complex constituted of BC2LCN lectin and Fucα1-2Galβ1-3GlcNAc/GalNAc; and a second step of bringing the first complex and free R-10G antibody into contact with each other to form a second complex constituted of BC2LCN lectin, Fucα1-2Galβ1-3GlcNAc/GalNAc and R-10G antibody.

As the insoluble carrier for use in B/F separation, a base material usually used in a protein immobilization method, such as a slide glass, an ELISA plate, magnetic beads, a filter, a film and a membrane, can be used. As the material for a base material, e.g., glass, silicon, polycarbonate, polystyrene or polyurethane is usually used.

A method for immobilizing lectin to an insoluble carrier is not particularly limited, a known method such as a chemical binding method (a binding method via a covalent bond) and a physical adsorption method can be applied. Lectin can be immobilized to an insoluble carrier by using an extremely strong binging reaction such as an avidin-biotin reaction. In this case, biotinylated lectin obtained by binding biotin to lectin may be immobilized to a streptavidin plate coated with streptavidin. Alternatively, lectin may be immobilized to an insoluble carrier via a linker (various linkers are known) usually used in the art.

The B/F separation method using an insoluble carrier may have a washing step for removing unnecessary substances from a solid-phase surface, after the first step of reacting a test sample and BC2LCN lectin immobilized to an insoluble carrier, and before the second step of reacting the first complex and free R-10G antibody; and may have a washing step after the second step and before the detection step. Contaminants in a sample and unreacted R-10G antibody are removed from the solid-phase surface by the washing step. In this manner, the second complex alone can be isolated on the solid-phase surface.

In the method including no B/F separation step, a complex of BC2LCN lectin, Fucα1-2Galβ1-3GlcNAc/GalNAc and R-10G antibody can be separated by applying, for example, a chromatography, high performance liquid chromatography, electrophoresis, capillary electrophoresis, capillary chip electrophoresis and/or a method using an automatic immunoassay system, for example, LiBASys (manufactured by Shimadzu Corporation).

The conditions employed in the method may be set in accordance with a known method. For example, if HPLC is used, separation may be made in accordance with the method described in Anal. Chem. 65,5,613-616 (1993) or Japanese Patent Laid-Open No. 9-301995. If capillary electrophoresis is used, separation may be made in accordance with the method described in J. Chromatogr. 593 253-258 (1992), Anal. Chem. 64 1926-1932 (1992) or WO2007/027495. If, e.g., LiBASys is used as the automatic immunoassay system, separation may be made in accordance with the method described in Biological Sample Analysis Vol. 22, No. 4, 303-308 (1999).

In the detection step, a second complex constituted of BC2LCN lectin, Fucα1-2Galβ1-3GlcNAc/GalNAc and R-10G antibody is detected by use of a label substance. Examples of the label substance include label substances usually used in the art, such as an enzyme usually used in, e.g., immunoassay, a radioactive isotope, a fluorescent substance, a luminescent substance, DNA, RNA, a coenzyme or a substance specifically binding to a coenzyme (biotin, avidin), a tag, a substance having absorption within an ultraviolet to infrared region, a chromogenic fine particle, a fluorescent fine particle, a metallic fine particle, a magnetic substance and a substance having a property as a spin labelling agent.

A label substance is bound to BC2LCN lectin and/or R-10G antibody, preferably to R-10G antibody, by appropriately using, for example, a labelling method usually employed in, e.g., immunoassay. Also, a method of binding a label substance to an antibody via a single or several amino acids, or a single or several amino acids and a linker may be employed. Since various kits for binding a label substance to a protein are commercially available, labelling can be made in accordance with the instruction manual attached to a kit.

A method of separating B/F by using, for example, BC2LCN lectin immobilized to an insoluble carrier and a free R-10G antibody tagged with horseradish peroxidase (HRP) as a label substance, is outlined as follows.

A test sample is brought into contact with an insoluble carrier to which BC2LCN lectin is immobilized, allowed to react at 4 to 40°C for 3 minutes to 20 hours to form a first complex of BC2LCN lectin and Fucα1-2Galβ1-3GlcNAc/GalNAc on a solid-phase surface. Next, a solution containing R-10G antibody labeled with HRP is supplied onto the solid-phase surface and allowed to react at 4 to 40°C for 3 minutes to 16 hours to form a second complex of immobilized BC2LCN lectin-Fucα1-2Galβ1-3GlcNAc/GalNAc-labelled R-10G antibody. Subsequently, a solution containing TMB (3,3'5,5'-tetramethylbenzidine) in an appropriate concentration, is added and allowed to react for a predetermined time. Thereafter, a reaction-termination solution such as 1 M sulfuric acid is added to terminate the reaction and absorbance at 450 nm is measured. From the resultant measurement value and a calibration curve, which is obtained by subjecting a Fucα1-2Galβ1-3GlcNAc/GalNAc solution having a predetermined concentration to the same measurement, the amount of Fucα1-2Galβ1-3GlcNAc/GalNAc (sugar chain represented by (Formula 1) or (Formula 2)) in the test sample can be obtained.

Also, the sugar chain represented by (Formula 1) or (Formula 2) can be measured by using, for example, BC2LCN lectin labeled with, e.g., Alexa Fluor-488 tetrafluorophenyl ester and R-10G antibody labeled with, e.g., Alexa Fluor-647 succinimidyl ester in accordance with known Fluorescence Correlation Spectroscopy (FCCS).

A complex of "BC2LCN lectin-Fucα1-2Galβ1-3GlcNAc/GalNAc-R-10G antibody" can be detected without using a label substance but using, e.g., the property derived from a complex, more specifically, a measurement system such as homogeneous immunoassay (surface plasmon resonance).

Note that, the lectin-antibody sandwich method according to the present invention is not limited to a manual method. If the lectin-antibody sandwich method is applied to the measurement system using an automatic analyzer, measurement can be easily and quickly carried out. Combination of reagents in measurement in manual or measurement by an automatic analyzer is not particularly limited and the most appropriate combination of reagents in consideration of the environment and model of the automatic analyzer to be employed or other factors is selected and put in use. Furthermore, the lectin-antibody sandwich method according to the present invention can be applied to Micro-TAS (Micro-Total Analysis Systems: µ-TAS).

The aforementioned specific measuring method using a body fluid sample can be used for detecting cancer cells of the present invention.

### <Determination of degree of malignancy>

The degree of malignancy is determined as follows. When the expression level of a BC2LCN-positive sugar chain measured in a test sample of the test individual is high compared to the expression level of a healthy person or a low-grade cancer patient, it is determined that the degree of malignancy of a test individual is high. In measuring the expression level of a BC2LCN-positive sugar chain in a test individual, the expression level of a BC2LCN-positive sugar chain of a healthy person or a low-grade cancer patient is measured and used as a control and comparison can be made. Alternatively, the expression level of a BC2LCN-positive sugar chain in a test sample of a healthy person or a low-grade cancer patient is measured in advance and a cutoff value of expression level is previously determined. Then, if the expression level of a BC2LCN-positive sugar chain measured in a test sample of a test individual exceeds the cutoff value, it can be determined that the degree of malignancy of cancer of the test individual is high. In this case, if a cell sample is used as a test sample, the expression level of a BC2LCN-positive sugar chain can be expressed by the ratio of cells expressing the BC2LCN-positive sugar chain. Since BC2LCN can specifically detect cancer cells and has a high binding activity particularly to malignant cancer cells, early-stage cancer can be detected and the degree of malignancy can be evaluated. In this sense, the present invention deals with an examination method for determining whether a test individual is affected with a cancer, particularly a high-grade cancer, and also includes (deals with) a method for collecting data for determining whether or not a test individual is affected with a cancer, particularly a high-grade cancer.

### <Determination of therapeutic method>

Based on the expression level of a BC2LCN-positive sugar chain in a test individual affected with a cancer, the therapeutic effect of cancer can be determined. For example, based on the expression level of a BC2LCN-positive sugar chain measured in a test individual, a therapeutic method such as a chemotherapy, a combination therapy of the present invention (described later) using BC2LCN and a toxin preparation in combination and a surgical therapy, can be determined.

For example, if the expression level of a BC2LCN-positive sugar chain is low, a chemotherapy can be selected in expectation of efficacy of an anti-cancer agent. In contrast, if the expression level of a BC2LCN-positive sugar chain is high, it can be determined that the degree of malignancy is high and life extension effect by a chemotherapy is low. In this case, a combination therapy using a BC2LCN-toxin preparation in combination, or a pain relief care can be selected. If the expression level of a BC2LCN-positive sugar chain in a test sample of a cancer patient is periodically measured, a suitable therapeutic method can be determined in each period. In this sense, the present invention deals with an examination method for selecting a therapeutic method for a test individual affected with a cancer and also includes (deals with) a method for collecting data for selecting a therapeutic method for a test individual affected with a cancer.

### <Determination of prognosis/therapeutic effect>

Based on the expression level of a BC2LCN-positive sugar chain measured in a test sample of a test individual affected with a cancer such as pancreatic cancer, the prognosis of the patient can be determined. For example, if the expression level of a BC2LCN-positive sugar chain is high, it can be evaluated that the prognosis is poor. In this sense, the present invention deals with an examination method for predicting prognosis of a test individual and includes (deals with) a method for collecting data for predicting prognosis of a test individual.

If a surgical treatment such as a surgery, a chemotherapy such as a treatment with an anti-cancer agent, an immunotherapy or a radiotherapy is applied to a test individual affected with a cancer, the efficacy of the therapy applied can be determined by checking the difference in expression level of a BC2LCN-positive sugar chain in a test sample between before and after treatment.

### 6. Reagent for introducing substance into cancer cells

### (6-1) Migration ability of BC2LCN lectin into cells

The present inventors previously found that BC2LCN lectin migrates into undifferentiated cells through binding to a sugar chain, Fucα1-2Galβ1-3GlcNAc/GalNAc on the surface of the undifferentiated cell surface, or a membrane protein or lipid containing the sugar chain; and further that a compound fused to BC2LCN is introduced into undifferentiated cells. In addition, they found that a "BC2LCN-toxin" agent, which is prepared by fusing a toxin exhibiting toxicity within a cell to BC2LCN lectin, can specifically and efficiently remove only human iPS/ES cells remaining in human iPS/ES cell derived cells for transplantation use (Patent Literature 19).

In the present invention, it was newly found that a fusion protein of BC2LCN lectin and cell killing toxin extremely efficiently kills, in vitro and in vivo, BC2LCN-positive cancer cells and exhibits a remarkable antitumor effect (see Examples).

Exhibiting not only cytotoxicity to cancer cells in vitro but also an antitumor effect in vivo is an advantageous effect in view of clinical application. This is because a side effect on a normal tissue and rapid decomposition and removal by, e.g., protease are problems in vivo.

Cancer cells can be specifically and efficiently killed by treating the cancer cells by a fusion protein, which is prepared by fusing a toxin or a domain thereof having an ability to kill cells to BC2LCN lectin excellent in specificity and affinity to the cancer cells.

### (6-2) Re: Toxin that can be fused

As the agent for killing cancer cells of the present invention, a "BC2LCN-toxin" fusion described in Patent Literature 19 can be used.

The "toxin" of the "BC2LCN-toxin" fusion of the present invention is used as a general term representing a substance exhibiting toxicity within cancer cells. Examples of the toxin include a toxic protein, a toxic low molecular-weight compound; a cytotoxic nucleic acid such as an RNAi substance (RNA interference molecule), an antisense nucleic acid and ribozyme; and a known anti-cancer agent such as cyclophosmid, docetaxel and GEM. The toxic protein refers to, e.g., a protein, glycoprotein and peptide having cytotoxicity. The "toxic low molecular-weight compound" includes all toxic compounds except antibiotic substances, dyes, toxic proteins and nucleic acids.

Of these toxins, a toxin capable of exhibiting cytotoxic or cell killing function within cancer cells when it is fused to "BC2LCN" to form a "BC2LCN-toxin fusion", which is applied to cancer cells; a protein toxin exerting a protein synthesis inhibitory action particularly within cells; a nucleic acid such as an RNAi substance; or a toxic low molecular-weight compound is preferable. Generally, a cell has a receptor specifically binding to the toxin having a protein synthesis inhibitory action, on the surface thereof. Thus, if a wild type full-length toxic protein is used, even if it is used in the form of fusion with BC2LCN, it can exhibit cytotoxicity indiscriminately to normal cells and presumably produces a significant side effect. Accordingly, it is preferable that binding ability of a toxic protein to a specific receptor present on the surface of cells is inhibited by modification, for example, deleting a receptor binding domain from the toxic protein in advance or introducing a mutation to the protein. These modification methods are well known to those skilled in the art. For example, in the case of p. aeruginosa toxin (PE), which is an exotoxin produced by Pseudomonas aeruginosa, a PE variant is known, which is produced by removing a domain I region having a binding site to a PE receptor and a binding ability to normal cells, at the genetic level (Non Patent Literature 8).

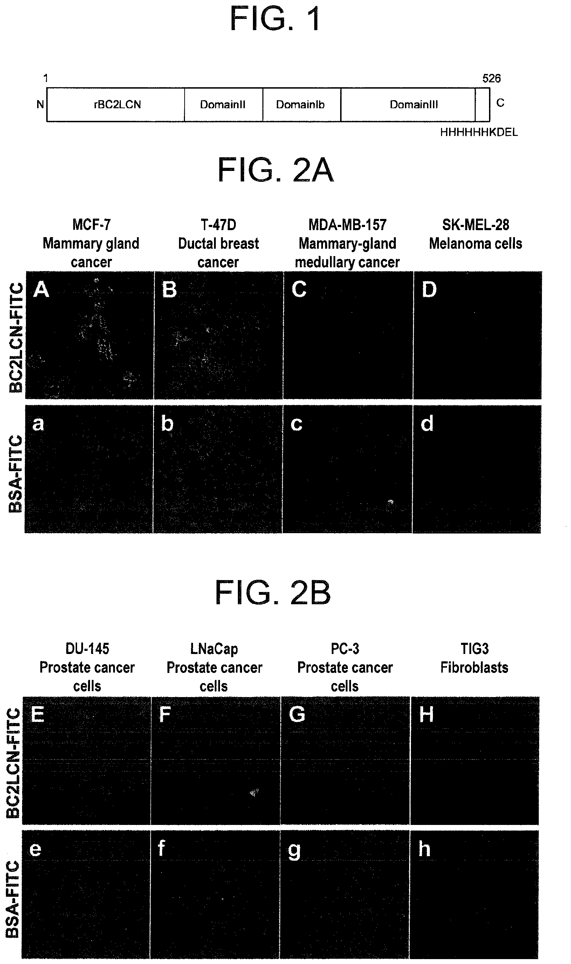

It is preferable to use a domain having ability to kill cells (expected to have a significant cytotoxic effect) and exerting an effect on a higher order structure that BC2LCN lectin may have, as little as possible. A "BC2LCN-ETA" fusion, in which a cell killing domain (ETA) region derived from pseudomonas exotoxin A is used as the domain, is preferably used. More specifically, e.g., a "BC2LCN-ETA (SEQ ID No: 2)" can be designed by optimizing a gene encoding an amino acid sequence corresponding to a domain region ("ETA") having ability to kill cells of pseudomonas exotoxin A (PDB Registration No: 1XK9) to a host such as E. coli, and ligating, e.g., a spacer sequence thereto, in accordance with the description in Patent Literature 19. The "BC2LCN-ETA" can be produced in a large amount from the host transformed with BC2LCN-ETA (SEQ ID No: 2). At this time, the length of the cell killing domain region is appropriately controlled and a sequence having a high cell killing activity can be selected. For example, the "BC2LCN-ETA (PE23)" fusion used in Examples of the present invention is designed and constructed by binding a partial region 23 kDa ("ETA (PE23)") of the cell killing domain (ETA) region to BC2LCN with two repeats of "GSGGG" sequence as a linker (SEQ ID No: 2) and binding ER retention signal (SEQ ID No: 6) at the C terminal and was previously used as a remover for undifferentiated cells by the present inventors (Patent Literature 19, Non Patent Literature 1). Further, in Examples of the present invention, a 38 kDa portion (PE38) (SEQ ID No: 3) of the cell killing domain region is directly bound to BC2LCN via a peptide bond as shown in Figure 1 to prepare a "BC2LCN-ETA (PE38)" fusion, which is used as an agent for killing pancreatic cancer cells.

BC2LCN-PE38 exerts a marvelous cytotoxic effect, which is 100 times or more as strong as conventional BC2LCN-ETA (PE23). "BC2LCN-ETA (BC2LCN-PE23)" is a fusion protein prepared by binding a cell killing domain, i.e., 23 kDa domain (PE23), which consists only of ETA catalytic domain (domain III), to BC2LCN via a peptide linker. "BC2LCN-PE38" is a fusion protein prepared by binding 38 kDa domain region (PE38) containing domain II and domain Ib, to BC2LCN via a peptide bond. The linker sequence consists of two repeats of Gly-Ser-Gly-Gly-Gly sequence (SEQ ID No: 2).

It has been confirmed that the fusion protein, "BC2LCN-ETA", has the same sugar-chain binding ability as BC2LCN lectin; and that "BC2LCN-ETA" has no effect on normal human differentiating cells, similarly to BC2LCN lectin (Patent Literature 19, Non Patent Literature 9).

In the present invention, the nucleotide sequences and amino acid sequences of other toxic proteins, which can be bound to BC2LCN lectin, can also be obtained from commercial database. Examples of the toxic proteins include diphtheria toxin (PDB Registration No: 1MDT), ricin (PDB Registration No: 2AAI), saporin (PDB Registration No: 3HIS), cholera toxin (PDB Registration No: 1XTC), enterotoxin (PDB Registration No: 1LTH) and pertussis toxin (PDB Registration No: 1PRT). These toxic proteins may not have a whole length and may be sufficient as long as they contain a domain region having an ability to kill cells. Note that, even if a toxin is not a protein, the toxin can be used as long as it is a toxic compound capable of binding to a linker or a spacer.

### (6-3) Fusion method

As a method for fusing BC2LCN lectin and a target substance to be transported into cancer cells, a chemical method and a gene-level linking method are known. In the case of the chemical method, e.g., a biotin-streptavidin bond is used other than a covalent bond. In the case of using a low-molecular compound such as FITC, a BC2LCN fusion can be formed by binding FITC randomly to a functional group (e.g., a hydroxyl group, an amino group) present on the surface of BC2LCN through a general chemical reaction (a covalent bond and a hydrogen bond used as a binding mode).

For example, as a fusion method, BC2LCN lectin and a toxin, preferably fused with a covalent bond. As a general method for use in fusing toxin including a low-molecular compound, a chemical binding method using a bivalent crosslinking agent can be used. In the case where an RNAi substance such as siRNA is bound, a biotin-streptavidin bond or e.g., a fusion protein of BC2LCN lectin and a positively charged DNA binding peptide (for example, cluster sequence rich in Arg derived from protamine; Winkler J, et al., Mol Cancer Ther. 2009 Sep; 8 (9): 2674-83) is used.

In contrast, since a nucleic acid such as RNAi is usually negatively charged, it is preferable to modify it in order to avoid direct contact with a cancer-cell surface also negatively charged, for example, forming a complex between a fusion protein consisting of BC2LCN lectin and a positively charged DNA binding peptide, and a nucleic acid, in advance.

If the toxin is a protein (toxin), binding at the genetic level is preferable. At that time, both genes thereof can be directly bound or bound via a general DNA linker in accordance with a well-known method.

It was previously confirmed that if BC2LCN lectin is bound to a toxin via a spacer sequence to form a fusion protein, the ability of BC2LCN lectin to form a multimer and binding property thereof do not change (Patent Literature 19). A toxin-fused protein obtained by binding them at the genetic level can be prepared as a protein having lot-to-lot uniformity (small between-lot variation) and thus particularly expected as a cancer cell remover that can be stably supplied. As a conjugate method, e.g., a biotin-streptavidin system is used, and then, a toxin can be bound to BC2LCN lectin; however, this method requires time and labor. In addition, since a toxin is introduced randomly to BC2LCN lectin, lot-to-lot variation is produced. This method has a problem: it is difficult to prepare a protein having lot-to-lot uniformity. Accordingly, in the case of chemical binding, as a toxin fused protein prepared through binding at the genetic level, a toxin-fused BC2LCN lectin is desirably formed via a covalent bond.

### (6-4) Linker or spacer

In the present invention, in fusing a compound, which is desired to be transported into cancer cells and work there, to BC2LCN lectin, a linker (cross linker) or a spacer (spacer sequence) can be used. In order not only for BC2LCN lectin to exert its intrinsic functions: cancer-cell specific binding function, invasion function, and but also for the compound to be transported to exert its intrinsic function as much as possible, both BC2LCN lection and the compound preferably keep a certain distance between them. Because of this, both are preferably bound via a linker/spacer having an appropriate length. The linkers/spacers having an appropriate length are well known to those skilled in the art and can be appropriately synthesized and are commercially available.

As the spacer sequence for binding to a peptide and used in the present invention, a well-known spacer sequence is used, which consists of an amino acid sequence having 4 to 10 amino acid residues capable of binding via a peptide bond and is used as a 1 to 3 time-repetitive sequence. Typically, an amino acid sequence constituted of glycine and/or serine such as "GSGGG (SEQ ID No: 4)", "GGGS (SEQ ID No: 5)" which will not form a higher order structure, is preferably used. For example, in transporting a toxic protein into cancer cells, if BC2LCN lectin and a toxin to be fused to BC2LCN lectin or its domain having an ability to kill cells are bound through a peptide bond with a spacer sequence interposed between them, a sufficient distance between them can be maintained between them, and respective abilities: sugar chain binding ability and an ability to kill cells, can be produced to a maximum extent.

In the present invention, the above peptide linker may be used in conjugation via a chemical bond. As a preferable linker, polyethylene glycol, and particularly preferably, e.g., a thiol linker prepared by introducing a thiol group cleavable by a reducing agent, is mentioned.

A low molecular-weight toxic compound is bound frequently by using a chemical binding agent such as a bivalent crosslinking agent. Accordingly, BC2LCN lectin binds to a low molecular-weight toxic compound via a linker derived from the binding agent.

When BC2LCN lectin of the present invention is used in order to introduce an RNAi substance such as siRNA and miRNA into a nucleic acid, generally BC2LCN is directly bound not to a nucleic acid but to a nucleic acid carrier such as a positively charged DNA binding peptide. Because of this, to keep a proper distance between BC2LCN lectin and the nucleic acid carrier, an appropriate linker/spacer is sometimes used.

In forming a fusion, if a transport signal targeting to a desired cytoplasmic organella within cancer cells to which a toxin is to be transported is bound to a toxin, the fusion can be further efficiently led to the desired cytoplasmic organella (for example, Figure 1, e.g., "KDEL (SEQ ID No: 6) sequence at the C terminal, corresponding to endoplasmic reticulum retention signal"). If a desired substance such as an RNAi substance needs to be transported within a nucleus, it is effective to use a nuclear transport signal.

In the case where a toxic protein is desirably cut off in a target site (although it is not required for the case where a toxic protein sufficiently produces toxicity in the state of a fusion with BC2LCN lectin), if an intracellular protease cleavage site is previously inserted into a fusion, a compound transported in the form of a fusion can be appropriately cut off within the cell. An introduction method of a cleavage site is well known to those skilled in the art. For example, if a sequence consisting of basic amino acids (typically, Arg-X-(Arg/Lys)-Arg) is added as a target sequence, the sequence is cut by Ca2+-dependent transmembrane serine endoprotease called furin (Weldon JE, et al., FEBS J. 2011 Dec; 278 (23): 4683-700.). In the case where a nucleic acid such as DNA or an RNAi substance is introduced into undifferentiated cells, if the nucleic acid is introduced in the state of a complex with a positively charged substance (such as an Arg cluster) bound to BC2LCN lectin and formed via charge-charge interaction, the nucleic acid dissociates immediately upon reaching the interior of cancer cells.

### (6-5) Method for producing BC2LCN-toxic (fusion) protein

To the 5' or 3' terminal side of BC2LCN gene of a BC2LCN-containing expression vector, a toxin gene such as an ETA-derived Domain I-III (PE38) gene is introduced, if necessary, via a spacer. In this manner, a toxin-fused BC2LCN protein expression vector is constructed. Next, a competent cell is transformed with the expression vector. Then, the transformed host such as E. coli is cultured in a liquid in accordance with a routine method, to induce expression of a toxin-fused BC2LCN protein.

### (6-6) Method for purifying toxin-fused BC2LCN protein and method for confirming the purity

The toxin-fused BC2LCN protein induced in expression in E. coli can be purified by applying a protein purification method usually used; however, it is preferable that the protein is subjected to a fucose-immobilized column and purified by affinity chromatography. The degree of purification of the obtained toxin-fused BC2LCN protein can be confirmed by, e.g., electrophoresis and gel filtration.

### (6-7) Re: BC2LCN-PE38 and method for preparing BC2LCN-PE38

BC2LCN-PE38 used in Examples of the present invention is a fusion protein prepared by binding a domain (SEQ ID No: 3) of 38 kDa constituted of Domain I to Domain III of ETA and having an ability to kill cells to BC2LCN lectin (SEQ ID No: 1) via a peptide bond, as shown in Figure 1, and adding a HIS tag and an endoplasmic reticulum transfer signal (KDEL) to the C terminal.

A specific preparation method is the same as the method described in Patent Literature 19.

### (6-8) Agent for killing cancer cells (used in in vitro system)

In the present invention, the "BC2LCN-toxin" fusion prepared as mentioned above is applied to cultured cancer cells or cancer cells or a tissue taken out from a living body to specifically kill cancer cells.

When an agent for killing cancer cells of the present invention is applied to target cancer cells in order to kill them in an in-vitro system, the cytotoxic agent is added in a medium so as to obtain a final concentration of 10 to 100 µg/ml and culture is carried out for 24 to 48 hours. In this manner, cancer cells alone can be killed.

### 7. Anti-cancer agent (cancer therapy and therapeutic composition)

The "BC2LCN-toxin" fusion of the present invention can be used alone, together or in combination with a known anti-cancer agent as a treatment or therapeutic agent for gastrointestinal cancers such as pancreatic cancer, large intestine cancer and stomach cancer; epithelial cancers such as lung cancer, breast cancer, uterine cancer, ovarian cancer and prostate cancer; malignant tumor such as a brain tumor; and, malignant sarcoma, and in particular, a high-grade cancer.

The treatment and therapeutic agent of the present invention can kill or decrease cancer cells, particularly high-grade cancer cells including cancer stem cells such as drug resistant cancer cells. In addition, since the toxicity of the treatment and therapeutic agent of the present invention to normal organ cells is extremely low, the treatment and therapeutic agent of the invention can be used as a fundamental therapeutic agent for tumors.

More specifically, when the "BC2LCN-toxin" fusion of the present invention is used as an anti-cancer agent, since it is expected to have an ability to kill cancer stem cells, the fusion has a high recurrence prevention effect and improves prognosis of the treatment.

For the reason that the cytotoxicity particularly to high-grade cancer cells including cancer stem cells such as drug resistant cancer cells is high, it is effective that the "BC2LCN-toxin" fusion of the present invention is used in combination with a known anti-cancer agent. There is a high possibility that the fusion is applied to a patient treated with a known anti-cancer agent and gave up the treatment because of drug resistance. As the toxin to be used in combination with BC2LCN lectin to form a "BC2LCN-toxin" fusion, a known anti-cancer agent is effectively used.

The treatment and therapeutic agent for a high-grade cancer according to the present invention is preferably administered as a pharmaceutical composition further containing a pharmacologically acceptable carrier, an excipient or an auxiliary agent. The pharmacologically acceptable carrier and the like used in the composition are known to those skilled in the art. The timing and times of administration and dosage are appropriately determined depending on, e.g., the state of a cancer tumor, severe or mild symptom and the state of the patient.

Now, a typical high-grade cancer, i.e., pancreatic cancer, will be described below; however, the same applies to other gastrointestinal cancers such as large intestine cancer and stomach cancer, general epithelial cancers such as lung cancer, breast cancer, uterine cancer, ovarian cancer and prostate cancer, other malignant tumors such as a brain tumor and malignant sarcoma.

The BC2LCN-toxic protein of the present invention can be used alone or in combination with a known anti-cancer agent as a treatment and therapeutic agent for pancreatic cancer.

The treatment and therapeutic agent for pancreatic cancer of the present invention can kill or decrease pancreatic cancer cells, particularly drug resistant cancer cells and in addition are virtually nontoxic to normal pancreas cells. Because of this, the treatment and therapeutic agent for pancreatic cancer can be used as a fundamental therapeutic agent for pancreatic cancer.

For the reason that, of the pancreatic cancer cells, cytotoxicity to drug resistant cancer cells is high, it is effective to use the treatment and therapeutic agent for pancreatic cancer in combination with a known anti-cancer agent for pancreatic cancer. It is particularly effective to apply the treatment and therapeutic agent for pancreatic cancer to a patient treated with a known anti-cancer agent and gave up the treatment because of drug resistance. If the treatment and therapeutic agent is directly administered to the abdominal cavity after a surgical operation, pancreatic cancer cells which cannot be visually checked can be killed without fail and thus a recurrence prevention effect in the prognosis can be enhanced.

The treatment and therapeutic agent for pancreatic cancer of the present invention is preferably administered as a pharmaceutical composition further containing a pharmacologically acceptable carrier, an excipient or an auxiliary agent.

As a method for administering the treatment and therapeutic agent for pancreatic cancer of the present invention, parenteral administration such as intravenous administration, intradermal administration and subcutaneous administration are applied. It is most effective to directly administer the treatment and therapeutic agent for pancreatic cancer of the present invention to the abdominal cavity.

The timing and times of administration are appropriately determined depending on, e.g., the state of a tumor of pancreatic cancer, severe or mild symptom and the state of the patient. The dosage may be administered once or divided into portions and administered several times within the range of one hour to 10 weeks; however, the administration method is not limited to this.

The dosage is appropriately determined in view of safety and effectiveness depending on, e.g., the state of a tumor, severe or mild symptom and the state of the patient. The dosage that can be administered falls, for example, in the range of 10 to 250 µg/body weight (kg); however, the dosage is not limited to the rage.

The present invention is mainly directed to a human; however, the invention is not limited to a human and directed widely to pancreatic cancers of mammals including a primate such as a monkey, a dog, a cat, a cow, a horse and a rodent such as a mouse.

A pharmacologically acceptable carrier and the like for use in parenteral administration such as intravenous administration of the pharmaceutical composition for treating pancreatic cancer are known to those skilled in the art. Examples thereof that can be appropriately used include a sterile diluent such as distilled water for injection, saline solution, glycerin and propylene glycol; an antimicrobial agent such as benzyl alcohol; an antioxidant such as ascorbic acid; a chelating agent such as ethylenediaminetetra acetic acid; a buffer such as phosphoric acid; a tonicity agent such as sodium chloride or D glucose; and a pH adjustment liquid.

The treatment and therapeutic agent for pancreatic cancer of the present invention can be used in combination with a known anti-cancer agent approved as for a pancreatic cancer. At this time, the treatment and therapeutic agent can be used as a pharmaceutical composition for cancer therapy in combination with another anti-cancer agent; however, the timing and/or dosage form of the treatment and therapeutic agent may not be the same as those of the other anti-cancer agent. As an administration method, different timing and different dosage form can be selected.

In particular, an anti-cancer agent, which is expected to produce a combination effect when it is used in combination with the anti-cancer agent of the present invention, is a GEM preparation used in Examples of the present invention, but not limited to this. Many anti-cancer agents and immunological preparations (including a molecular target drug and an antibody drug) such as TS-1 and erlotinib and drugs for mitigating symptoms, can be used. The anti-cancer agent of the present invention is preferably used in combination with an anti-cancer agent, a molecular target drug, and an antibody drug such as an antimetabolite (5-FU, methotrexate), an alkylating agent (cyclophosmid) and taxane series (paclitaxel, docetaxel) in the case of, e.g., a gastrointestinal cancer except pancreatic cancer or an epithelium cancer such as breast cancer, prostate cancer and gynecologic cancer.

### 8. Other use of the cancer cell-specific intracellular transport system

If a compound such as a nucleic acid, a physiologically active protein, a lipid and a low-molecular compound except a toxin and a dye compound for a label (compounds except a toxin described in Patent Literature 19) is fused with BC2LCN and applied to cancer cells, the compound can be efficiently transported within cells and exert its intrinsic function. As a method for fusing them, the method described in, e.g., the above sections (6-3) (6-4) can be used.

More specifically, in the present invention, BC2LCN lectin is used as a carrier (intracellular introduction agent) for transporting compounds specifically to cancer cell within cells; in other words, the present invention is used as an intracellular transport system specific to cancer cells by using BC2LCN lectin as a carrier. Use as the agent for killing cancer cells and an anti-cancer agent can be positioned as one of utilization forms of the mechanism of the invention.

### Examples

The present invention will be more specifically described by way of Examples; however, the present invention is not limited to these.

Other terms and concepts of the present invention are used based on the meanings of those conventionally used in the art and various techniques for carrying out the present invention can be easily carried out by those skilled in the art without fail based on known documents except the techniques referring to specific documents. Various analyses were carried out in accordance with the methods described in, e.g., user's manuals and catalogs of analyzers, reagents or kits.

The clinical pancreatic cancer cells used in the present invention were derived from patients who gave informed consent at the clinical institution that the inventors belong to, i.e., University of Tsukuba, checked by the ethical review committee and received ethical approval.

Note that, the descriptions of the technical documents, the description contents of patent publications and patent applications cited in the specification are incorporated by reference in this specification.

### [Example 1: Evaluation of binding activity of BC2LCN lectin to cancer cells and cancer tissue]

### (Example 1-1) Cell staining of various cancer cell strains

The binding activity of BC2LCN lectin to various cancer cell strains was immunohistochemically evaluated.

Mammary gland cancer cells (MCF7 strain), ductal breast cancer cells (T-47D strain), mammary-gland medullary cancer (MDA-MB-157 strain), melanoma cell (SK-MEL-28 strain) and prostate cancer cell strain (DU-145 strain, LNaCap strain, PC-3 strain) used in this experiment were purchased from ATCC and cultured in accordance with the culture method specified by ATCC. Fetal lung fibroblasts (TIG3 strain) were cultured in accordance with the culture method of Nishimura et al. (Nishimura K, et al. J Biol Chem. 2011 Feb 11; 286 (6): 4760-71).

The cells were fixed with 4 % paraformaldehyde and washed with PBS. Fluorescently labeled (FITC-bound) BC2LCN was added to the fixed cells and a reaction was carried out at room temperature for one hour.

Two types of breast cancer cells (MCF7 strain, T-47D strain) (Figure 2A a, b) were strongly stained with fluorescently labeled BC2LCN; however, strong staining was not observed in other breast cancer cells (MDA-MB-157 strain), prostate cell strain (DU-145 strain, LNaCap strain, PC-3 strain), melanoma cells (SK-MEL-28 strain) and fetal lung fibroblasts (TIG3 strain) (Figure 2A c, d, Figure 2B e-h). T47-D strain was sparsely stained (Figure 2A b).

Since the cells were not crushed and directly subjected to the above experiment, the sugar chain structure: "Fucα1-2Galβ1-3GlcNAc/GalNAc" recognized by BC2LCN lectin seems to be present as a constituent sugar of a glycoprotein and a glycolipid largely expressed in breast cancer cells so as to cover the cell surface. Since the sugar chain structure recognized by BC2LCN lectin is present on the cell surface, high usefulness as a kit for determining the presence or absence of cancer cells is demonstrated.

### (Example 1-2) Flow cytometry analysis of various cancer cell strains by Hilyte Fluor™ 647-labelled BC2LCN lectin

The binding activity of BC2LCN lectin to various cancer cell strains was evaluated by flow cytometry.

In addition to the cell strains used in Example 1, skin fibroblasts (HDF strain) purchased from ATCC, were cultured in accordance with the culture method specified by ATCC, and used in this experiment. Adipose-derived mesenchymal stem cell strain (ADSC strain) was purchased from Life Technologies and cultured in accordance with the attached manual. Pluripotent stem cell strain (201B7 strain) was purchased from the Riken Bioresource Center and cultured in accordance with the mTeSR1 culture method of Stemcell Technologies. The aforementioned cells were dissociated by an enzymatic treatment, fluorescently labelled (HiLyte Fluor 647-bonded) BC2LCN lectin was allowed to react in a concentration of 1 µg/ml and then subjected to flow cytometry analysis using FACS instrument.

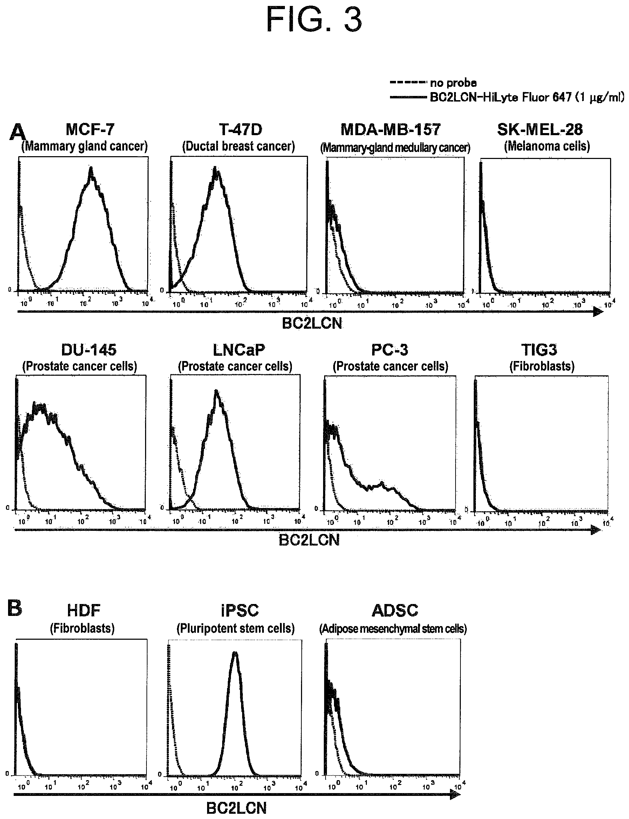

As a result, it was found that MCF7 strain and T-47D strain are cell populations strongly stained in the same manner as in human iPS cells (Figure 3). It was also found that MDA-MB-157 strain, DU-145 strain, LNaCap strain and PC-3 strain, from which no signals were detected in the cell staining of Figure 2, partly contain cells stained with BC2LCN. SK-MEL-28 strain (melanoma cell) were seldom detected similarly to HDF strain (skin fibroblast strain) and ADSC strain (adipose-derived mesenchymal stem cell strain).

### (Example 1-3) Lectin array analysis using cell membrane protein fractions of various cancer cells

Cell membrane protein fractions were extracted, from MCF7 strain, DU-145 strain, LNaCap strain, PC-3 strain, and SK-MEL-28 strain by using CelLytic™ MEM Protein Extraction Kit (Sigma) in accordance with the manual and BC2LCN responsiveness of them were analyzed by using a lectin array (Non Patent Literature 2).

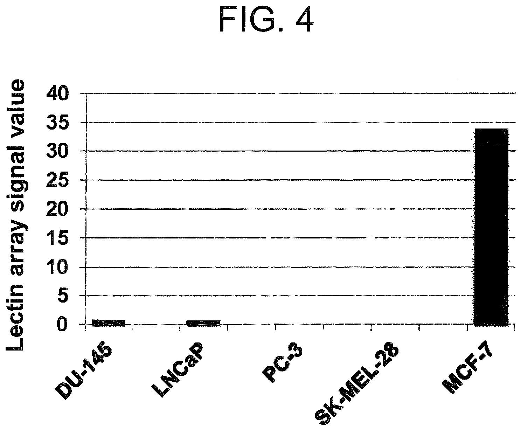

As a result, in MCF7 strain, from which strong signals were detected in cell staining and flow cytometric analysis, strong binding of BC2LCN lectin was detected (Figure 4). From this result, it was demonstrated that the surface antigen of cancer cells serving as a binding target of BC2LCN lectin is a glycoprotein.

### (Example 1-4) Binding of BC2LCN lectin to human cancer tissue

The binding activity of BC2LCN lectin to various cancer tissues was immunohistochemically evaluated.

A human cancer tissue array (CC08-10-001U, CC17-00-001) purchased from Cybdri and a human cancer tissue array (401 2204) purchased from Provitro were treated with HRP-labeled BC2LCN. In this manner, BC2LCN lectin recognition sites in human cancer tissues were analyzed.

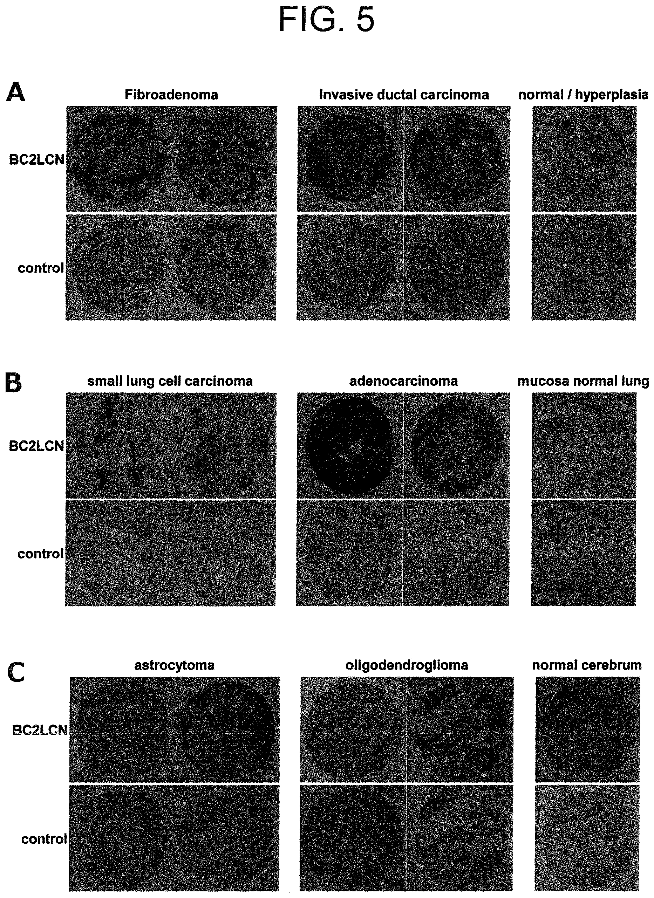

As a result, it was found that breast cancer (Figure 5A) and lung cancer (Figure 5B) have cell groups stained with BC2LCN lectin. BC2LCN-positive cells were partly present in a tumor (Figure 5C).

### (Example 1-5) Human clinical pancreatic cancer (specimen number 1) tumor site stained with BC2LCN lectin

Using clinical samples, the binding activity of BC2LCN lectin to a cancer tissue was immunohistochemically evaluated.

Tumors were removed from pancreatic cancer patients (specimen number 1) to (specimen number 3), individually fixed with formalin and embedded in paraffin, and then tissue sections were prepared. The tissue sections obtained were stained with horseradish peroxidase (HRP)-labelled BC2LCN lectin and then stained with hematoxylin/eosin. Thereafter, the tissue images were observed by a microscope.

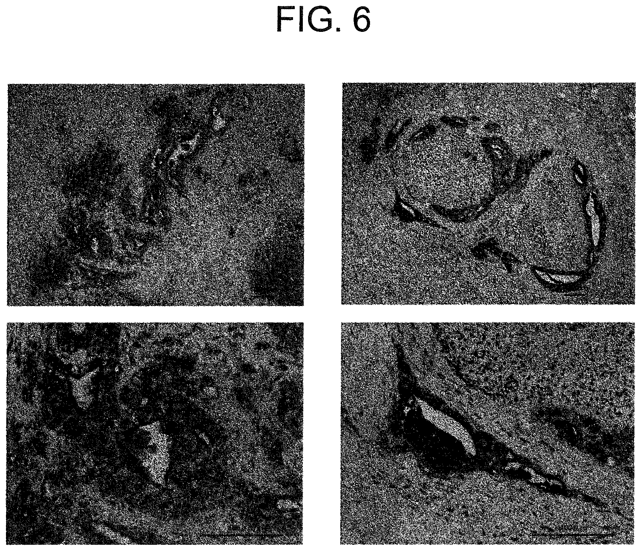

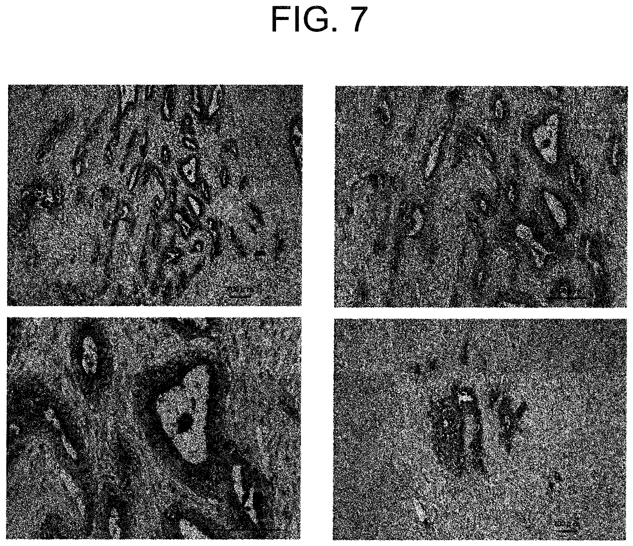

As a result, it was found that cells of human clinical pancreatic cancer (specimen number 1) to (specimen number 3) are all strongly stained with HRP-labelled BC2LCN lectin; and that, in particular, a duct-like configuration site is strongly stained and the peripheral normal pancreas cell site is not stained at all (Figures 6 to 8).

### (Example 1-6) Staining of tumor site of human clinical large intestine cancer with BC2LCN lectin

Using clinical samples, the binding activity of BC2LCN lectin to a cancer tissue was immunohistochemically evaluated.

Tumors were removed from large intestine cancer patients, individually fixed with formalin and embedded in paraffin, and then tissue sections were prepared. The tissue sections obtained were stained with horseradish peroxidase (HRP)-labelled BC2LCN lectin and then stained with hematoxylin/eosin. Thereafter, tissue images were observed by a microscope.

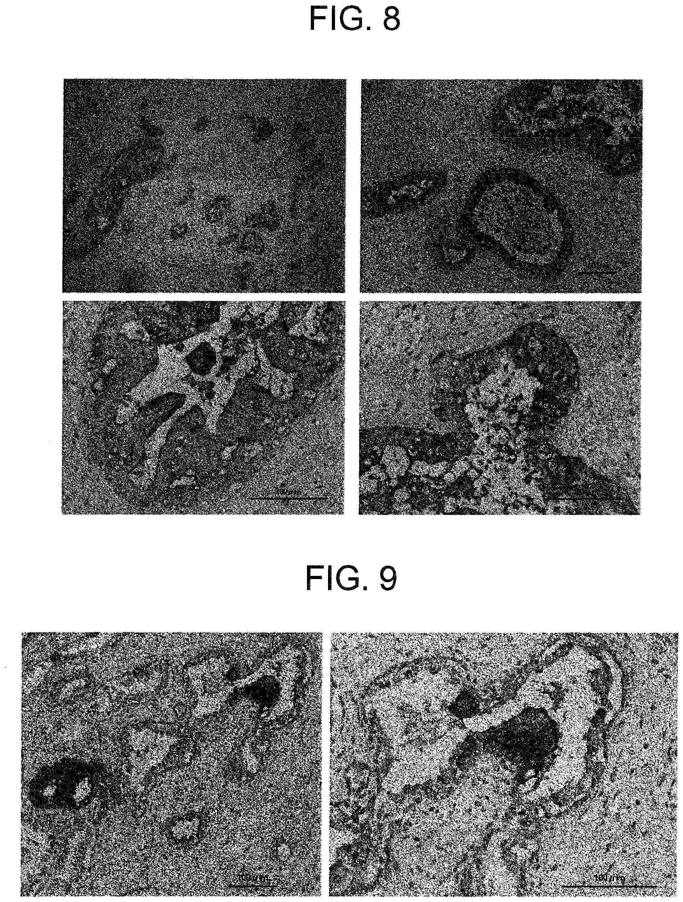

As a result, it was found that the peripheral normal cell site of cancer cells is not stained at all; however, the whole human clinical large intestine cancer cell site is more or less stained; more precisely, a poorly differentiated cancer cell site in which large intestine cancer nuclei are concentrated, was strongly stained; however, a highly differentiated site in which the polarity of nuclei is maintained was relatively weakly stained (Figure 9). The poorly differentiated site is considered as the site corresponding to cancer stem cells.

### (Example 1-7) Staining of various clinical cancer tissue arrays with labelled BC2LCN lectin

Using clinical samples, the binding activity of BC2LCN lectin to cancer tissue cells was immunohistochemically evaluated.

### (1-7-1) Staining of tissue sections derived from stomach cancer, large intestine cancer, mammary gland cancer, liver cancer, pancreatic cancer, bile duct cancer and lung cancer

FFPE tissue specimens (approved by the ethics committee) taken in University of Tsukuba hospital were used. Cancer sites and noncancerous sites of tissue sections of stomach cancer, large intestine cancer, mammary gland cancer, liver cancer, pancreatic cancer, bile duct cancer and lung cancer were punched out, and tissue arrays were prepared and stained with HRP-labelled BC2LCN lectin. As a control, the normal tissue sections of the corresponding organs were stained in the same manner.

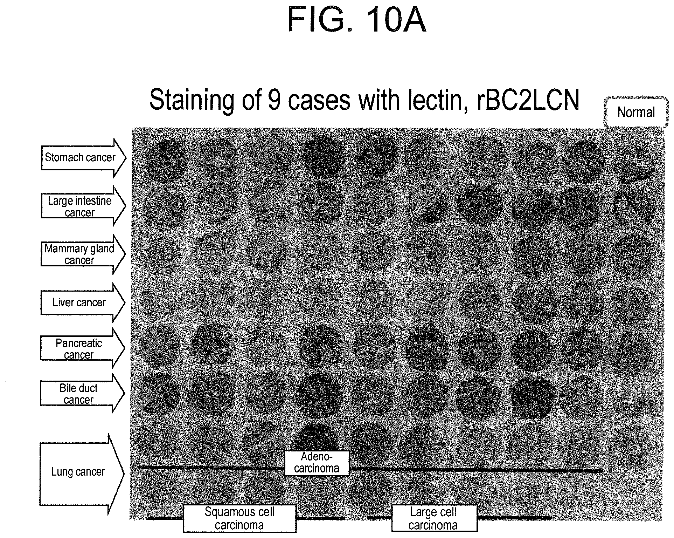

As a result, what are clearly stained compared to the normal tissues were tissues derived from stomach cancer, large intestine cancer, pancreatic cancer and biliary tract cancer; and tissues derived from adenocarcinoma of lung cancers; whereas, tissues derived from mammary gland cancer and liver cancer; and tissues derived from squamous cell carcinoma and large cell carcinoma of lung cancers were rarely stained (Figure 10A) .

### (1-7-2) Staining of uterine body cancer, cervical cancer, prostate cancer, renal cancer, bladder cancer, testicular cancer, ovarian cancer, endocrine system cancer, other organ cancers

FFPE tissue specimens (approved by the ethics committee) taken in University of Tsukuba hospital were used. Tissue sections derived from uterine body cancer, cervical cancer, prostate cancer, renal cancer, bladder cancer, testicular cancer, ovarian cancer, endocrine system cancer, other organ cancer (hepatoblastoma, malignant mesothelioma, osteosarcoma, glioblastoma, pancreatic endocrine tumor, metastatic breast cancer, metastatic uterine cancer) were punched out; and tissue arrays were prepared and stained with HRP-labelled BC2LCN lectin. As a control, the normal tissue sections of the corresponding organs were stained in the same manner.

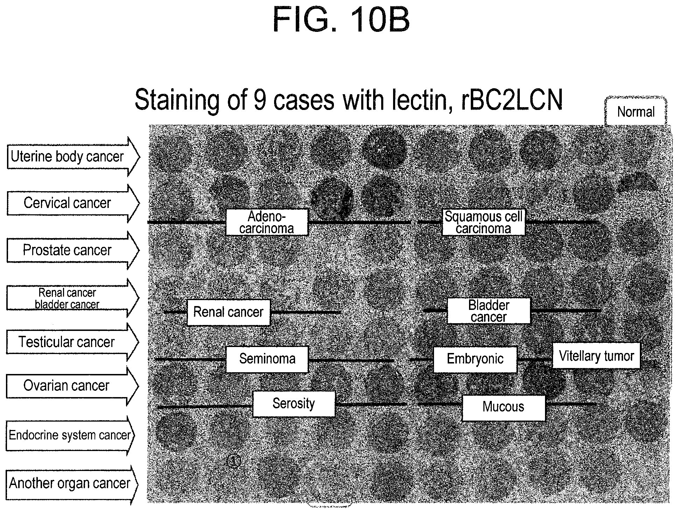

As a result, what are clearly stained compared to the normal tissues were tissues derived from adenocarcinoma of uterine body cancer and cervical cancer, and tissues derived from fetal cancer and egg yolk tumor of ovarian cancer; whereas a tissue derived from squamous cell carcinoma of cervical cancer, a tissue derived from seminoma of ovarian cancer, and tissues (metastatic sarcoma) derived from prostate cancer, renal cancer, bladder cancer, testicular cancer, endocrine cancer, and other organ cancer were rarely stained (Figure 10B).

### (Example 1-8) Histopathological examination of BC2LCN lectin binding site in human cancer tissue array

The binding activity of BC2LCN lectin to various cancer tissues cells was immunohistochemically evaluated.

Human cancer tissue arrays (CC08-10-001U: breast cancer, CC17-00-001: brain tumor) purchased from Cybdri and human cancer tissue array (401 2204: lung cancer) purchased from Provitro were treated with HRP-labelled BC2LCN lectin to stain BC2LCN recognition sites of human cancer tissues. Further, histopathological examination for confirming whether cancer cells were stained or not in the sections of the cancer tissue arrays was requested to New Histo. Science Laboratory Co., Ltd.

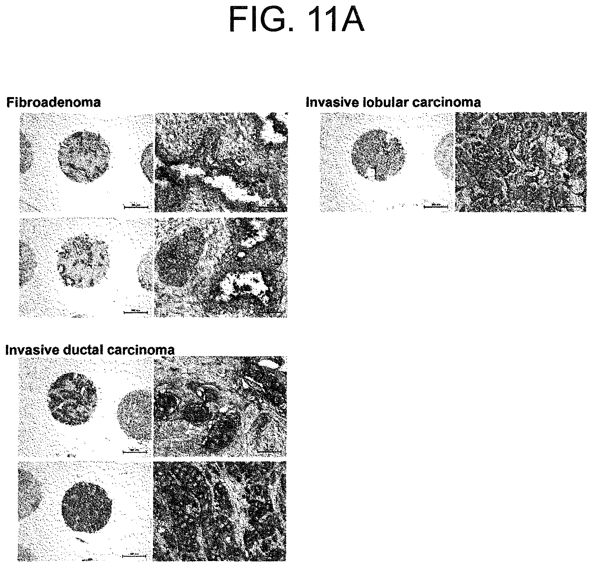

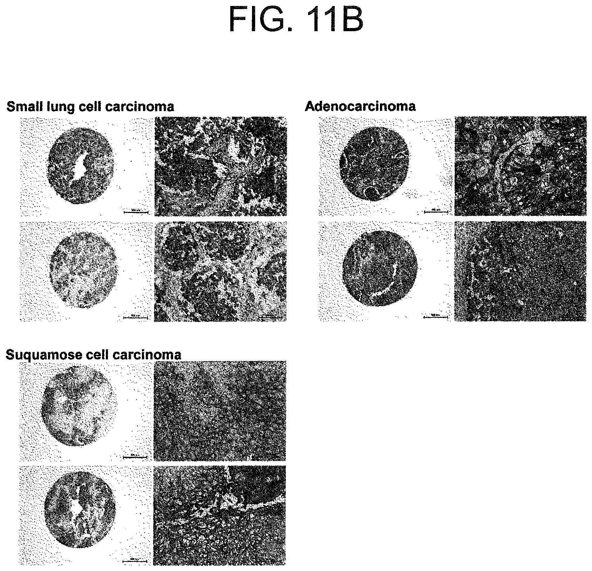

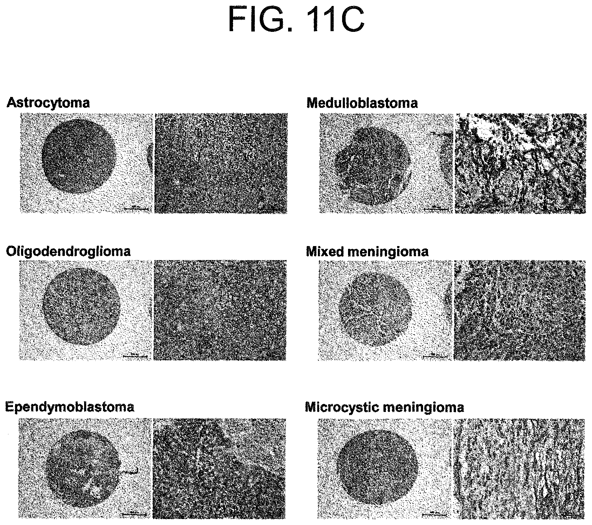

As a result, it was confirmed that, in a breast cancer tissue array, cell membrane and cytoplasm of each of fibroadenoma, invasive ductal breast cancer and invasive lobule cancer are stained. In the section of fibroadenoma, strong staining was detected on a lumen side (Figure 11A). It was also confirmed that, in a lung cancer tissue array, cell membrane and cytoplasm of each of small lung cell cancer, part of lung squamous cell carcinoma and lung adenocarcinoma are stained (Figure 11B). It was further confirmed that, in a brain tumor tissue array, cell membrane and cytoplasm of each of astrocytoma, oligodendroglioma, ependymoblastoma and medulloblastoma; and cytoplasm of each of mixed meningioma and microcystic meningioma is stained (Figure 11C). In each of the pairs of photographs in the figures, the left one is a photograph at a low magnification; whereas the right one is partly magnified view of the left photograph.

### (Example 1-9) Binding of BC2LCN lectin to human normal tissue

For comparison, the binding activity of BC2LCN lectin to normal tissues was evaluated.

Human normal tissue array (401 1110) purchased from Provitro was treated with HRP-labelled BC2LCN lectin. In this manner, the BC2LCN lectin recognition site in the human normal tissue was analyzed.

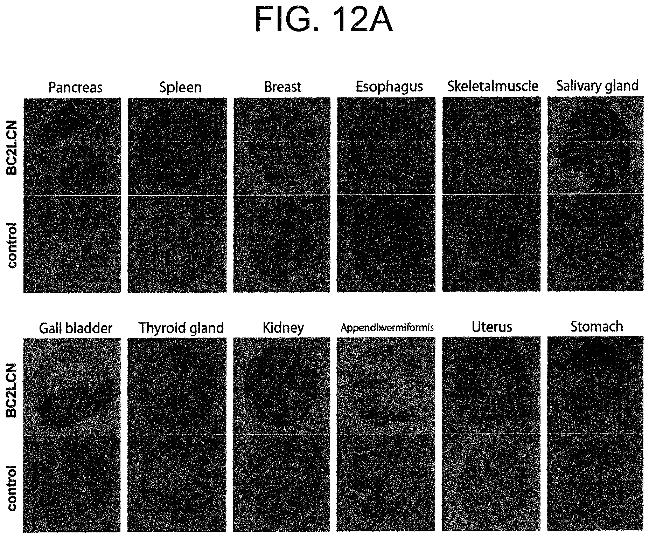

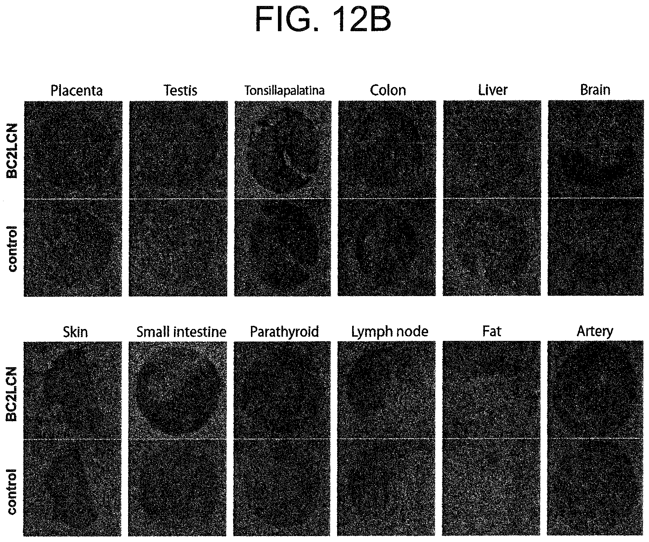

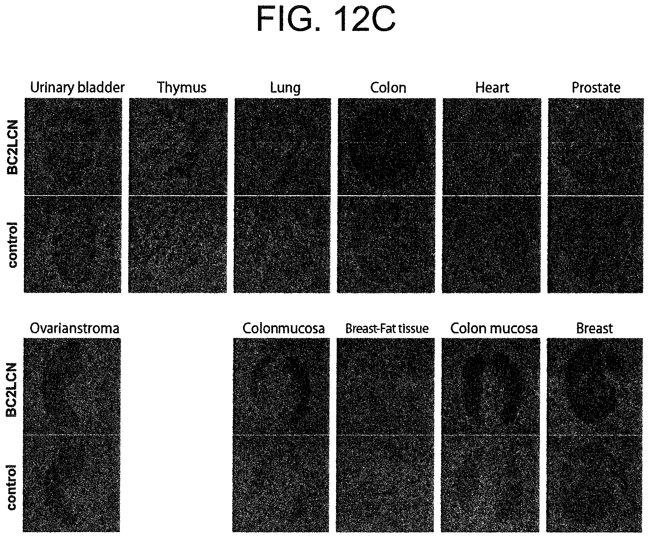

As a result, it was confirmed that tissues of, e.g., normal breast, lung, brain tissue, prostate, uterus, thyroid gland, parathyroid gland, liver, ovary and lymph nodes were not stained with BC2LCN lectin (Figure 12). From this, it was found that BC2LCN lectin can be used to distinguish a cancer cell-containing tissue from some types of normal tissues in a biopsy sample taken from a test individual suspected to have a cancer.

### [Example 2: Evaluation of degree of malignancy of BC2LCN-positive cancer cells]

### (Example 2-1) Sort of prostate cancer cell strain (PC-3) by BC2LCN lectin and adherent culture

As described above, some types of cancer tissues were stained with BC2LCN lectin; however, staining degree of cell strain/tissue varied. The cells of a prostate cancer cell strain (PC-3) (Non Patent Literature 7) were separated by a cell sorter into a cell group highly stained with BC2LCN (hereinafter referred to as BC2LCN+ group) and a cell group poorly stained with BC2LCN (hereinafter referred to as BC2LCN- group) and then proliferation rate, morphology and gene expression were analyzed. First, proliferative abilities of BC2LCN+ group and BC2LCN- group in adherent culture were evaluated.

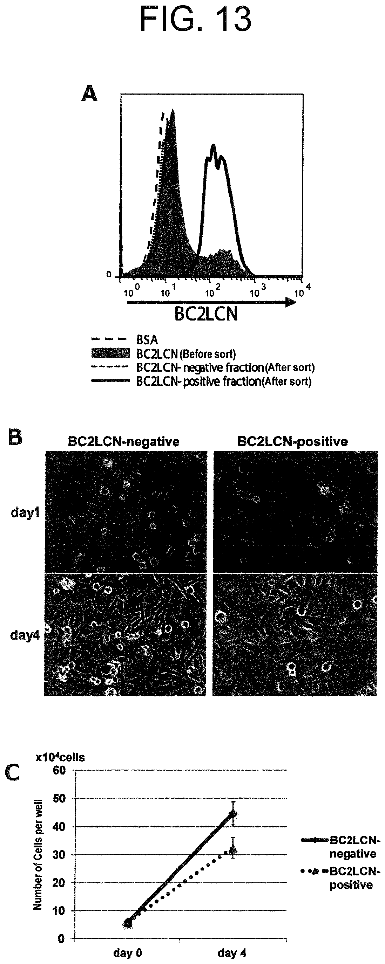

A prostate cancer cell strain (PC-3 strain) was cultured and fluorescently labelled (HiLyte Fluor 647-bound) BC2LCN lectin was reacted in a concentration of 1 µg/ml and then, the resultant cells were sorted into a BC2LCN+ group and a BC2LCN- group by FACS instrument. (Figure 13A). After sorting, the cells (6 × 10⁴ cells) of each group were seeded and cultured.

As a result, it was observed that the morphology of the cells differs between the BC2LCN+ group and the BC2LCN- group (Figure 13B). After 4-day culture, the number of cells were counted. As a result, proliferative ability of the BC2LCN- group was high as long as the culture is anchorage dependence (Figure 13C). In this experiment, cells of the BC2LCN+ group and BC2LCN- group are separately cultured in 4 wells. A confirmation test is independently repeated three times and average values and standard deviations are computationally obtained.

### (Example 2-2) Sorting of prostate cancer cell strain (PC-3) by BC2LCN lectin and anchorage-independent culture

The anchorage independent proliferative ability of BC2LCN-positive cancer cells and BC2LCN-negative cancer cells were evaluated.

The cells of a prostate cancer cell strain (PC-3 strain) were cultured and sorted in the same manner as in Example 2-1. BC2LCN+ group and BC2LCN- group were separately subjected to a soft agar colony formation test and the presence or absence of "anchorage independent proliferative ability", which is a major factor indicating the degree of malignancy of cancer cells, was checked.

Cell culture for soft agar colony formation and count of the number of cells were performed by using "CytoSelect™ 96-well malignant transformation assay - soft agar colony formation test kit - " (http://www.cosmobio.co.jp/product/detail/products_cbl_20 060613.asp?entry_id=7330).

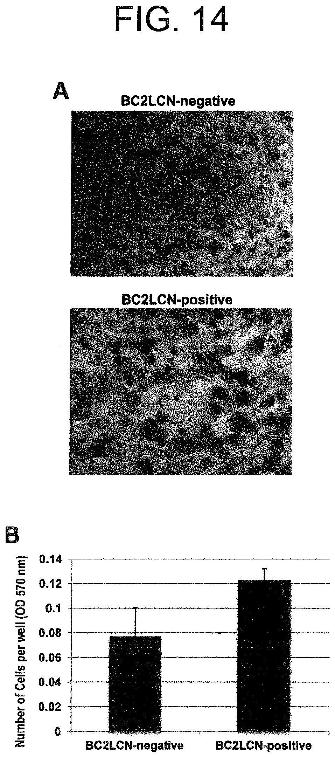

Seven days after initiation of the culture, images of cells proliferated were compared. As a result, it was found that the cells of BC2LCN+ group can proliferate in an anchorage-independent manner (Figure 14A). More specifically, in the BC2LCN- group, the same state when seeded is maintained; cell division does not occur and single cells float without change. In contrast, in the BC2LCN+ group, cell division occurs and clusters consisting of more than dozen cells are formed. Culture was continued and at Day 14 after initiation of culture, the number of cells was determined by using the above kit based on colorimetry. As a result, the cells of the BC2LCN+ group advantageously proliferated in an anchorage-independent manner (Figure 14B). In this experiment, cells of the BC2LCN+ group and BC2LCN- group are separately cultured in 3 wells. Confirmation test was independently repeated three times and average values and standard deviations are computationally obtained. It was demonstrated that high-grade cancer cells having high anchorage independent proliferative ability can be detected and concentrated by using BC2LCN.

### (Example 2-3) Sort of prostate cancer cell strain (PC-3) by BC2LCN lectin and expression analysis of cancer stem cell marker

Expression of a cancer stem cell marker in BC2LCN-positive cancer cells was analyzed.

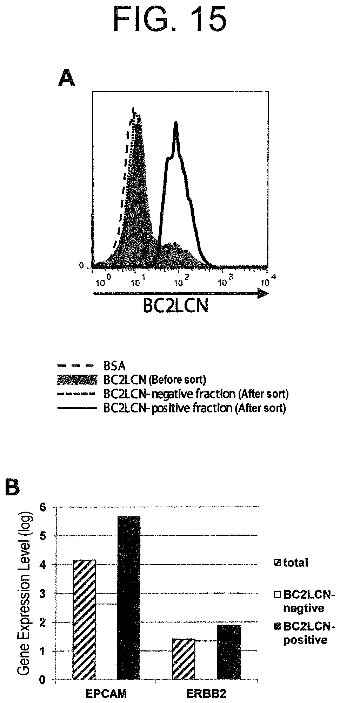

The cells of prostate cancer cell strain (PC-3 strain) were cultured in the same manner as in Example 2-1 and sorted. RNA was extracted separately from a BC2LCN+ group and a BC2LCN- group. Using a cDNA microarray (SurePrint G3 Human GE microarray kit 8x60K (Agilent)), global gene expression was analyzed (Figure 15A).

As a result, in the BC2LCN+ group, unlike the BC2LCN- group, known cancer stem cell markers such as EPCAM (Non Patent Literature 4) and ERBB2 (Non Patent Literature 7) were highly expressed (Figure 15B). BC2LCN-positive high-grade cancer cells had characteristics of cancer stem cells. More specifically, it was found that BC2LCN lectin is only one progressive marker capable of selectively detecting and separating cancer stem cells serving as the most valuable target for drug discovery/treatment.

### [Example 3: Evaluation of cytotoxicity of BC2LCN-ETA to cancer cells]

(Example 3-1) Cytotoxicity of BC2LCN-ETA to various cancer cell strains and fibroblast strain

Mammary gland cancer cells (MCF7 strain), ductal breast cancer cells (T-47D strain), mammary-gland medullary cancer (MDA-MB-157 strain), melanoma cell (SK-MEL-28 strain) and prostate cancer cell strain (DU-145 strain, LNaCap strain, PC-3 strain) were cultured. Fetal lung fibroblasts (TIG3 strain) were cultured in accordance with the culture method of Nishimura et al. (Nishimura K, et al. J Biol Chem. 2011 Feb 11; 286 (6): 4760-71). BC2LCN-ETA was added in each of the culture solutions so as to obtain a final concentration of 0.1 mg/ml to react with each of the cancer cell strains and the fibroblast strain during culturing. Twenty four, forty eight and seventy two hours after addition of BC2LCN-ETA, whether the cells are dead or alive was determined by using LIVE/DEAD Cell Imaging Kit (488/570) (Life Technologies).

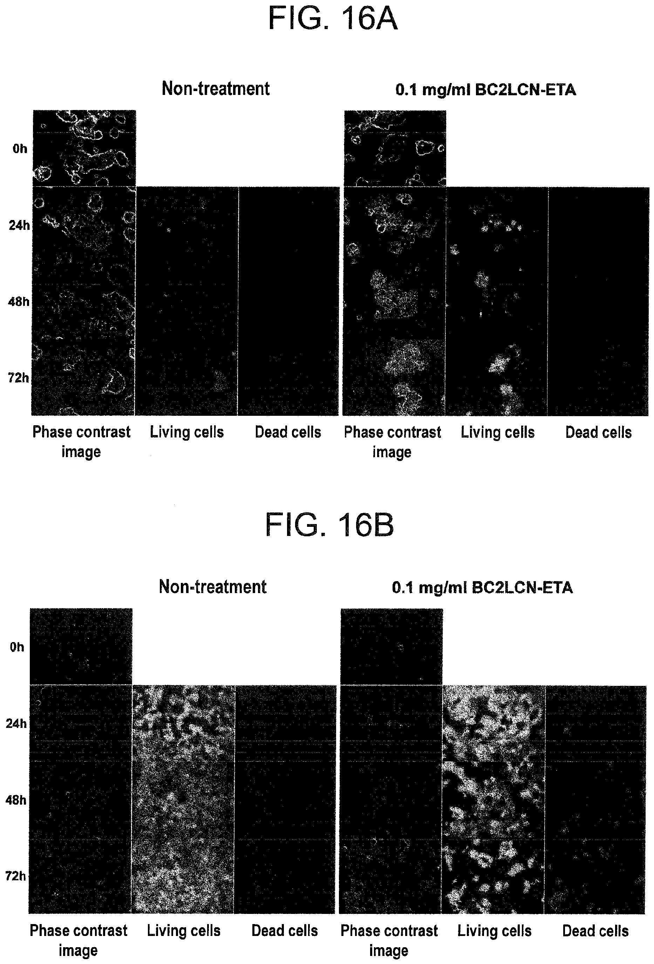

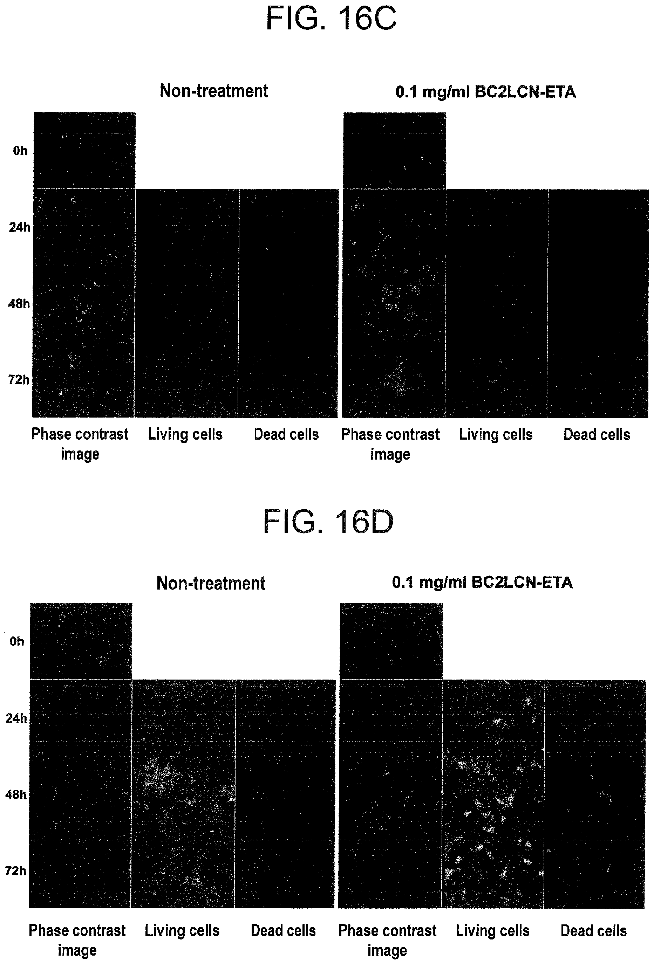

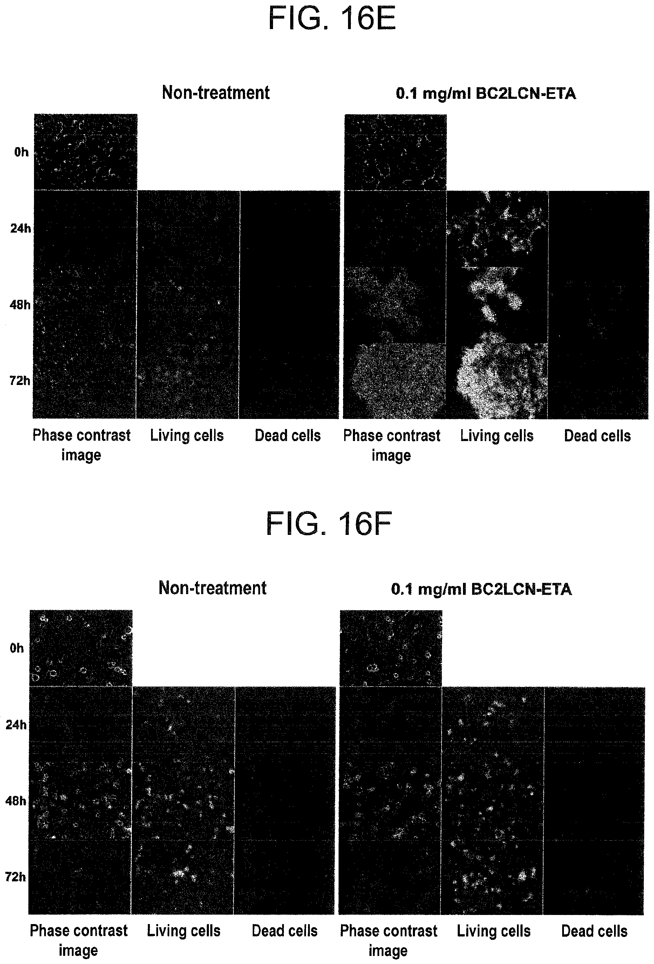

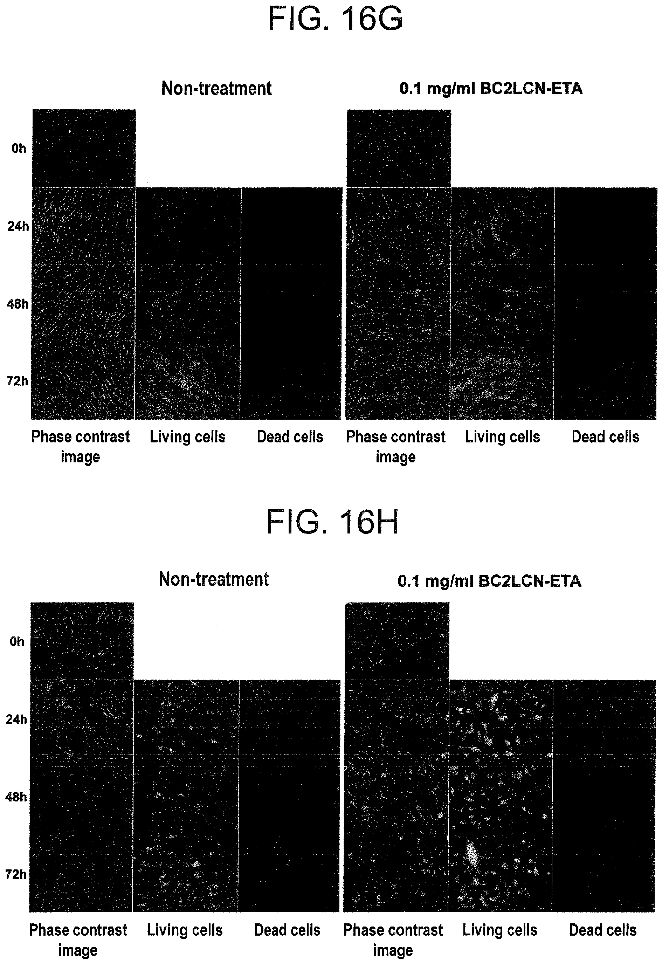

As a result, in MCF7 strain (Figure 16A), T-47D strain (Figure 16B), MDA-MB-157 strain (Figure 16C), DU-145 strain (Figure 16D), LNaCap strain (Figure 16E) and PC-3 strain (Figure 16F) containing BC2LCN-positive cells, dead cells were observed; however, TIG3 strain (Figure 16G) and SK-MEL-28 strain (Figure 16H) containing no BC2LCN-positive cells, dead cells were not observed.

Since BC2LCN-ETA does not affect normal cells at all, the cytotoxicity of BC2LCN-ETA is considered as being specific to cancer cells.

### (Example 3-2) Internalization of FITC-labelled BC2LCN lectin into breast cancer cell MCF-7 strain

Mammary gland cancer cells (MCF7 strain) were cultured. To the culture solution of MCF7 strain, FITC-labelled BC2LCN lectin was added in a concentration of 1 µg/mL and reacted at 37°C for 2 hours. Immediately after the reaction (2 hours) and 48 hours later, phase difference was observed by a microscope while applying excitation light.

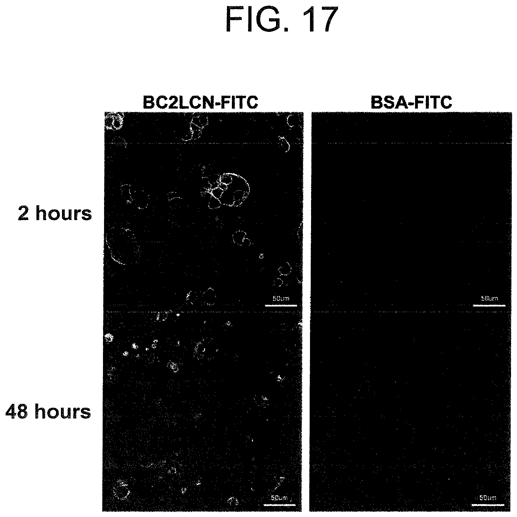

Immediately after the culture medium was exchanged with a fresh culture medium containing no FITC-labelled BC2LCN lectin, the cell surface is clearly stained (Figure 17, upper left). Forty eight hours later, dot-like stains are clearly observed in a cell (lower left). From this, it was found that FITC-labelled BC2LCN lectin is integrated within the cell. In the case where FITC-labelled BSA was added in a concentration of 1 µg/mL, the same view is not obtained. From this, it was demonstrated that BC2LCN lectin bound to a cancer cell surface is specifically integrated into the cell.

When a lectin recognizes a predetermined sugar chain on a cell surface, the lectin specifically bound to the sugar chain on the cell surface is generally observed; however, a phenomenon where a lectin enters the interior of the cell through the sugar chain recognized is rarely known. A phenomenon where "BC2LCN lectin" fused to ETA enters the interior of a cancer cell is beyond expectation. It was considered that "BC2LCN lectin" has not only a binding property to a sugar chain: "Fucα1-2Galβ1-3GlcNAc/GalNAc" on a cancer cell surface but also an invasive property into the cancer cell through the sugar chain after binding.

It was suggested that, if a nucleic acid, a physiologically active protein and a low-molecular compound except a toxin and a labelling dye compound, that is, various compounds except a toxin described in Patent Literature 19, each are fused with BC2LCN lectin and allowed to act on cancer cells, such a substance or a compound can be efficiently transported into the cancer cells and perform its inherent function.

### [Example 4: Detailed evaluation of binding activity of BC2LCN lectin to pancreatic cancer cells]

### (Example 4-1) Morphology of cancer cells formed in mice by xenografts of six types of pancreatic cancer cell strains

In order to develop effective diagnosis and therapeutic method for a high-grade cancer, a pancreatic cancer was selected as a typical high-grade cancer and an experiment was carried out in accordance with the following procedure.

Six types of typical pancreatic cancer cell strains (AsPC-1, BxPC-3, Capan-1, MIApaca-2, PANC-1, SUIT-2) were subcutaneously transplanted to mice in a rate of 3.0 × 10⁶ cells/mouse. Fourteen days after the transplantation, a tumor part was excised out, fixed with formalin, embedded in paraffin, sectioned by a microtome (Retoratome, REM-700 manufactured by YAMATO KOHKI) to produce tissue sections having a thickness of 5 µm, and stored at room temperature until use. After deparaffinization treatment, the tissue sections were stained with hematoxylin/eosin (Cat#032-14635, WAKO) and the tissue (image) was observed by an optical microscope (manufactured by KEYENCE: BZ9000).

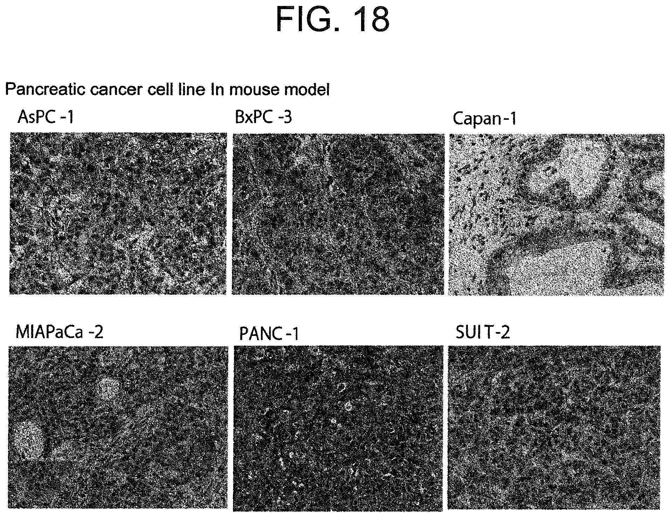

As a result, it was found that Capan-1 alone forms a lumen structure (duct), which is typical adenocarcinoma-like morphology (Figure 18). In contrast, formation of a lumen structure was not observed in the cases of other five types of cell strains; in other words, morphology analogous to clinical pancreatic cancer cells was not observed. From the above, it was found that Capan-1 of the six types of pancreatic cancer cell strains has an ability to form the characteristic morphology closest to clinical pancreatic cancer.

### (Example 4-2) Reaction strength of BC2LCN lectin to six types of pancreatic cancer cells obtained by high-density lectin microarray

Lectin which specifically reacts to Capan-1 was searched by a high density lectin array. From six types of pancreatic cancer cell strains, hydrophobic fractions were prepared by CelLytic™ MEM Protein Extraction Kit (Sigma, CE0050), labeled with Cy3-NHS (GE HEALTHCARE JAPAN, PA13104) and subjected to a high-density lectin microarray. Detection was made by an evanescent wave fluorescent scanner (GlycoStation™ Reader 1200, manufactured by Glyco Technica). The results obtained were converted into numerical values by Array-Pro Analyzer (manufactured by Media Cybernetics). Based on the numerical values obtained, the six types of pancreatic cancer cell strains were divided into two groups: one is Capan-1, which can generate duct of the gland and the other group consisting of other 5 types of cell strains. Lectin, which was remarkably differ between the two groups, was statistically extracted by Student's t-test.

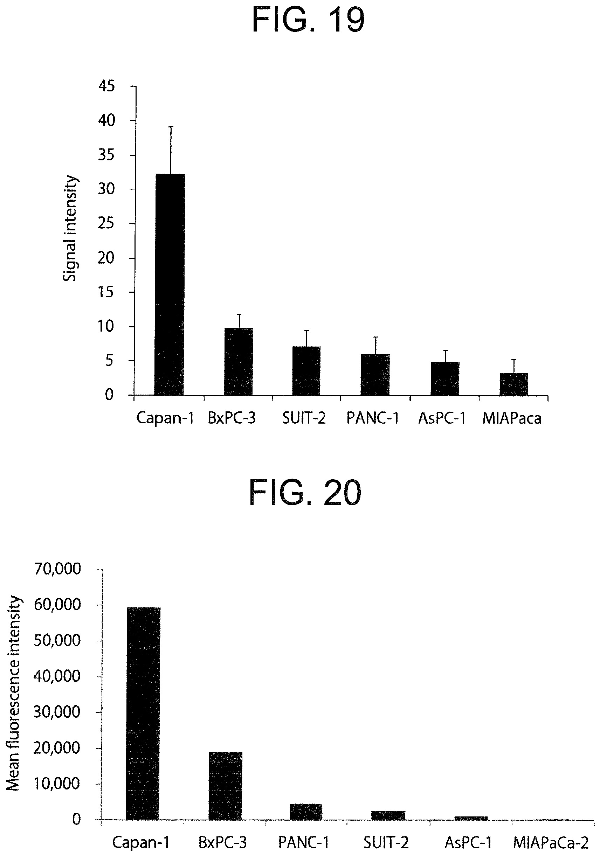

As a result, BC2LCN lectin represents p = 9.44E-17 and extracted as a most remarkably different lectin between the two groups. The binding strength values of BC2LCN lectin to pancreatic cancer cell strains are shown in Figure 19. BC2LCN lectin showed the strongest responsiveness to Capan-1.

### (Example 4-3) Analysis on binding of BC2LCN lectin to six types of pancreatic cancer cell strains by flow cytometry

Cells (1 × 10⁵ cells) of pancreatic cancer cell strains were reacted with 1 µg/mL BC2LCN labeled with R-Phycoerythrin Labelling Kit-NH2 (PE, manufactured by Dojindo, LK23) on ice for one hour and then analyzed by BD FACSCantoII flow cytometer (manufactured by BD Biosciences).

The resultant mean fluorescence intensity (MFI) is shown in the graph of Figure 20. It was found that BC2LCN lectin shows the strongest responsiveness to Capan-1, as the same as in the analysis by the high-density lectin microarray.

### (Example 4-4) Staining of tumor site in Capan-1 transplanted mouse model with BC2LCN lectin

Capan-1 (3.0 × 10⁶ cells) was subcutaneously transplanted to nude mice (BALB/c nunu female, 6 weeks old). Fourteen days later, a tumor was excised out, fixed with formalin and embedded in paraffin, and then, tissue sections were prepared. The tissue sections obtained was subjected to a deparaffinization treatment, antigen activation, peroxidase inactivation and blocking, and then, reacted with BC2LCN lectin labelled with horseradish peroxidase (HRP, Cat#: LK11, Dojindo) at room temperature for one hour. Thereafter, color was developed by a histofine DAB base material kit (Nichirei, 425011) and staining with hematoxylin/eosin (manufactured by WAKO) was performed. The tissue image was observed by a microscope (company: KEYENCE; BZ9000).

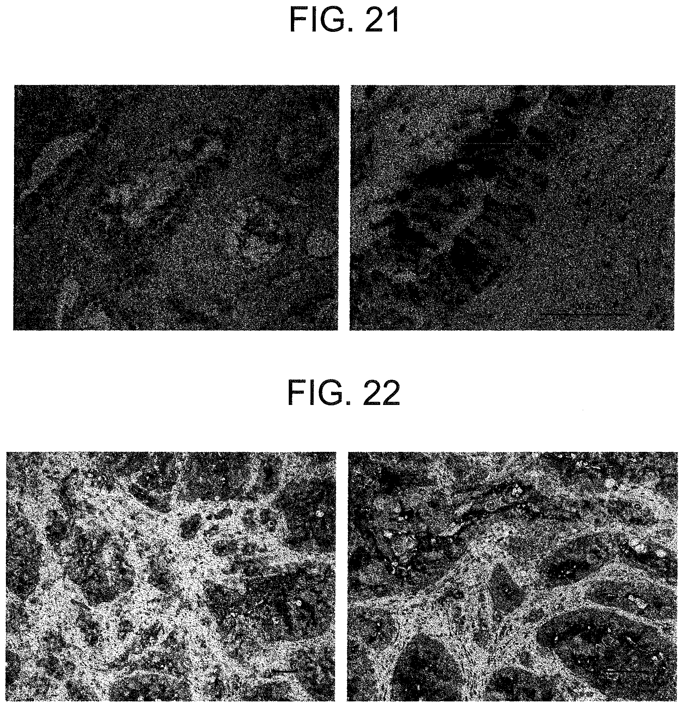

As a result, it was found that a lumen site showing adenocarcinoma-like morphology of the tumors formed is strongly stained and that peripheral normal cells are not stained at all (Figure 21).

### (Example 4-5) Staining of tumor site of human pancreatic cancer transplanted mouse model (PC-3 line) with BC2LCN lectin

A human clinical pancreatic cancer tumor was cut into pieces of 2-mm squares and subcutaneously transplanted in a SCID mouse (C.B-17/Icr-scid/scid female, 6 weeks old, CREA FARM) to obtain a human pancreatic cancer transplanted mouse model (PC-3 line, passage number: 7 to 10). Twenty eight days after the transplant, a tumor was excised out from the model, fixed with formalin and embedded in paraffin, and then, tissue sections were prepared. The tissue sections obtained were stained with horseradish peroxidase (HRP)-labeled BC2LCN and stained with hematoxylin/eosin. Then, a tissue image was observed by a microscope.

As a result, it was found that a lumen (formation) site showing adenocarcinoma-like morphology of the tumors formed is strongly stained, and that peripheral normal cells are not stained at all (Figure 22), similarly to the case of Example 4-4.

### (Example 4-6) BC2LCN lectin staining of a tumor site of human pancreatic cancer transplanted mouse model (PC-3 line) treated with gemcitabine hydrochloride (GEM)

Pancreatic cancer has a high recurrence rate and has a problem in that even if it is treated with a standard therapeutic agent, i.e., gemcitabine hydrochloride (GEM), growth and metastasis of pancreatic cancer cannot be completely suppressed. Because of this, development of an anti-cancer agent against GEM resistant cancer cells has been strongly desired. Then, a human pancreatic cancer transplanted mouse model (PC-3 line) was treated by different doses of GEM. After that, responsiveness of the remaining drug resistant pancreatic cancer cells to BC2LCN lectin was checked.

Human clinical pancreatic cancer cells were subcutaneously transplanted to prepare human pancreatic cancer transplanted mouse models (PC-3 line, passage number: 7 to 10), in the same manner as in Example 4-5. Day 10 after the transplantation, 50 mg and 100 mg of gemcitabine hydrochloride (for Gemzar injection (Eli Lilly Japan K.K. Lot.C177339CA)) were injected to the mouse models four times in total at intervals of 3 days through the tail vein. Day 14 after completion of the administration, a tumor was excised out from each of the models, fixed with formalin and embedded in paraffin, and then, tissue sections were prepared. The tissue sections obtained were stained with horseradish peroxidase (HRP)-labelled BC2LCN lectin and stained with hematoxylin/eosin. Then, tissue images were observed by a microscope.

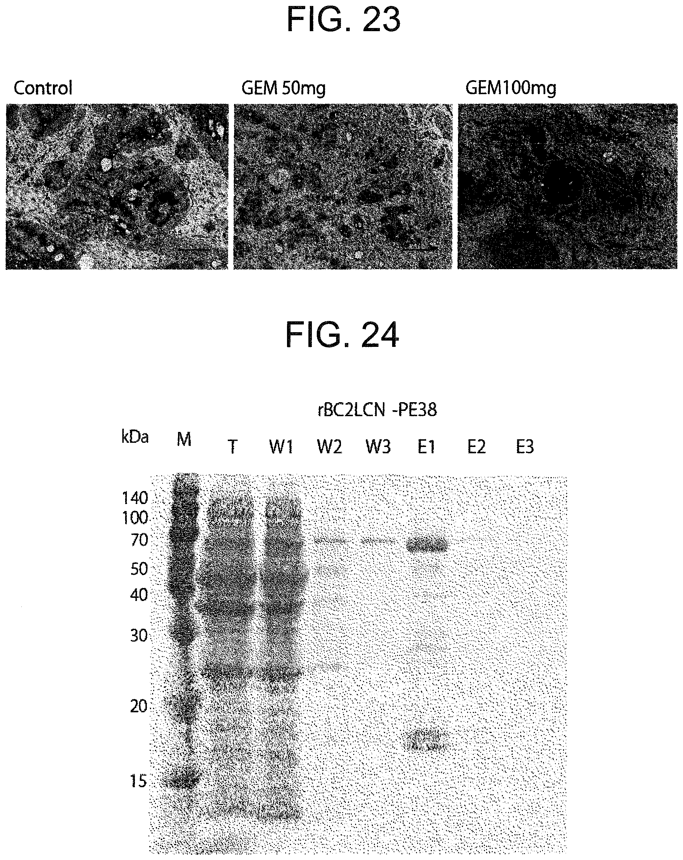

As a result, it was found that a tumor remaining after treatment with GEM is also strongly stained with BC2LCN lectin, and that intensity of staining of anticancer agent-resistant cells remaining after treatment with GEM is stronger (Figure 23). From the results, it was found that BC2LCN lectin is used as a satisfactory probe for targeting GEM resistant pancreatic cancer cells.

### [Example 5: Evaluation on cytotoxicity of BC2LCN-PE38 to cancer cells]

### (Example 5-1) Preparation of BC2LCN-PE38

BC2LCN-PE38 (protein described in Figure 1) was designed, constructed and integrated in pET27b (company: Stratagene), and then, introduced into Escherichia coli BL21 CodonPlus (DE3)-RIL strain (Company: Stratagene, #230245). Transformants were suspended in 5-mL-LB culture medium containing 10 µg/mL kanamycin and cultured overnight. Five mL of the culture solution (preculture) was added to 1L of LB culture medium and culture was performed. Two or three hours later when absorbance (OD₆₀₀) reached about 0.4, 1 mL of 1 M IPTG (Company: Fermentus #R-0392) was added so as to obtain a final concentration of 1 mM. After culture was performed while shaking at 20°C for 24 hours, the cells were centrifugally collected and suspended in a buffer and ultrasonically treated, and then, a soluble-protein fraction was extracted. The E. coli soluble-protein fraction was purified by affinity chromatography using a column of fucose sepharose, which was prepared by covalently binding fucose to commercially available sepharose (manufactured by GE Healthcare), in accordance with the Matsumoto et al. method (Matsumoto I, Mizuno Y, Seno N. (1979) J Biochem. Apr; 85 (4): 1091-8) and eluted with 0.2 M fucose.

The degree of purity was checked by subjecting fractions collected at the time a sample was just passed once (T), washed once (W1), washed twice (W2), washed three times (W3), eluted once with fucose (E1), twice with fucose (E2) and three times with fucose (E3) to SDS-PAGE electrophoresis (Figure 24). It was confirmed that the fraction of a sample once eluted provides a single band of about 70 kDa. The molecular weight of the band corresponds to a BC2LCN-PE38 monomer purified. BC2LCN-PE38 purified was treated or not treated in the presence or absence of 2-mercaptoethanol (2-ME) at 95°C for 5 minutes to prepare samples. The samples were subjected to SDS-PAGE and stained with coomassie brilliant blue (Figure 24). As a result, it was confirmed that purified BC2LCN-PE38 was obtained as a single band of about 56 kDa in any conditions.

### (Example 5-2) Cytotoxicity of BC2LCN-PE38 to Capan-1

To a culture solution of Capan-1, BC2LCN-PE38 was added in different concentrations. After culture for 48 hours, living cells of Capan-1 were counted by Cell Counting Kit-8 (Cat#: CK-04, Dojindo) and absorbance of OD₄₅₀ was measured.

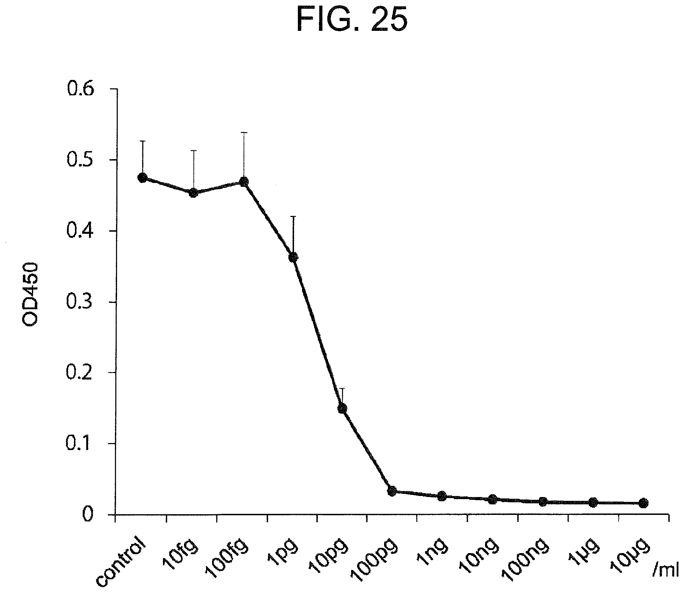

As a result, it was confirmed that the number of Capan-1 dead cells increases in a BC2LCN-PE38 concentration-dependent manner, and that Capan-1 cells are almost completely killed at a concentration of 100 pg/mL (Figure 25).

### (Example 5-3) Cytotoxicity of BC2LCN-PE38 to cancer cells in Capan-1 transplanted mouse model

### (5-3-1) Tumor volume change

Capan-1 (3.0 × 10⁶ cells) was subcutaneously transplanted to nude mice (BALB/c nunu female, 6 weeks old). Fourteen days later, formation of a tumor was confirmed. The minor axis and major axis of the tumor were measured and the volume of the tumor was obtained in accordance with the expression: (minor axis)²× (major axis)/2. The mice were divided into three groups (n = 6 per group) such that the tumor sizes of individual groups became equal (Control group, 1 µg/body, 10 µg/body). BC2LCN-PE38 was diluted with PBS to obtain solutions of 1 µg/100 µl and 10 µg/100 µl. The solutions of 1 µg/body and 10 µg/body were separately and subcutaneously injected to local sites near the tumor of mice four times in total from Day 1 at intervals of 3 days.

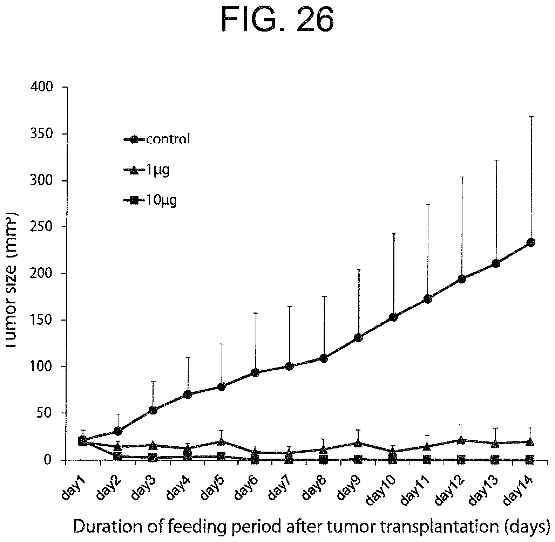

In the administration (10 µg/body) group, a tumor was significantly reduced by two-time administration. Since the tumor was not distinguishably observed, administration was terminated after the administration twice. Tumor volume was determined every day in the same manner as above and a change of tumor volume with time was observed. As a result, a tumor shrinkage effect was observed both in the administration (1 µg/body) group and the administration (10 µg/body) group compared to the control group, and significant effect was observed in the administration (10 µg/body) group (Figure 26).

### (5-3-2) Mouse weight change and tumor weight change

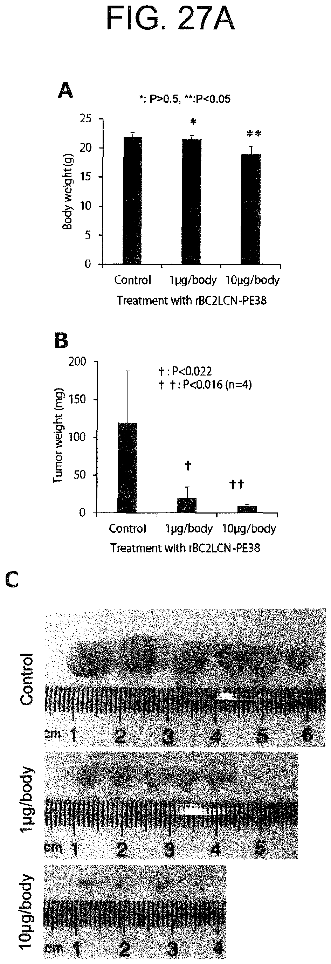

After tumor volume observation was carried out for 14 days, the body weight of mice was measured. In the BC2LCN-PE38 administration (1 µg/body, 4 times) group, a significant difference was not observed in mouse body weight; activities of individual mice were satisfactory; and whole body conditions were maintained, compared to Control group. In contrast, the BC2LCN-PE38 administration (10 µg/body, twice) group, 2 out of 6 mice died at Day 14; body weights of the remaining 4 mice were low compared to those of Control group (Figure 27A). A tumor was excised out from the mice and the weight of individual tumors was measured. As a result, it was found that both in the 1 µg-administration group and 10 µg-administration group, the tumor weight significantly decreases and a significant shrinkage of the tumor is visually observed (Figures 27B, C).

### (5-3-3) Pathological observation of excised tumor

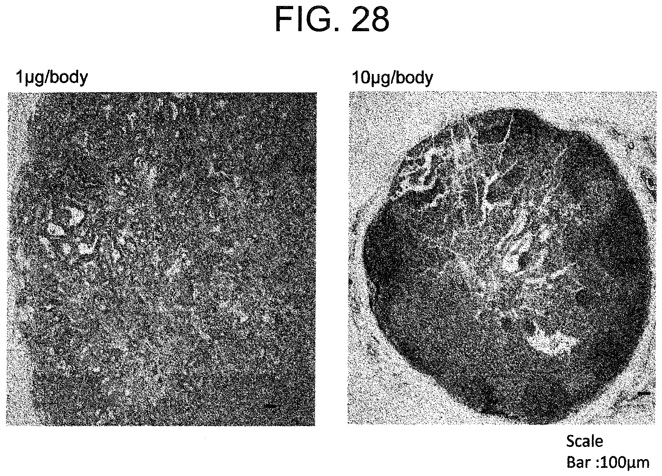

The tumor excised out, fixed with formalin and embedded in paraffin, and then tissue sections were prepared. The tissue sections obtained were stained with hematoxylin/eosin and observed the tissue images by a microscope. As a result, it was found that, in the 1 µg-administration group, the size of a tumor reduces; however, cancer cells having a duct-like configuration characteristic in Capan-1 still remain; and that in the 10 µg-administration group, cancer cells having a duct-like configuration disappear and infiltration with inflammation cells such as lymphocytes is only observed. From this, it was found that cancer cells completely disappeared (Figure 28).

### (Example 5-4) Cytotoxicity to BC2LCN-PE38 to cancer cells in PDX mouse model

### (5-4-1) Tumor volume change

A cancer tissue piece excised out from a patient with pancreatic cancer was subcutaneously transplanted in immunodeficient mice (NOD/SCID). Twenty one days later, formation of a tumor was confirmed and the minor axis and major axis were measured and the volume of the tumor was obtained in accordance with the expression: (minor axis)²× (major axis)/2. The mice were divided into three groups (n = 5 per group) such that the tumor sizes of individual groups became equal (Control group, 40 ng/mouse, 1 µg/mouse, 1 µg/mouse (intraperitoneal injection: i.p.)). BC2LCN-PE38 was diluted with PBS to obtain solutions of 1 µg/100 µl and 5 µg/100 µl. The solutions (1 µg/body and 5 µg/body) were separately and subcutaneously injected to local sites near the tumor of each mouse, five times in total from Day 1 at intervals of 2 days.

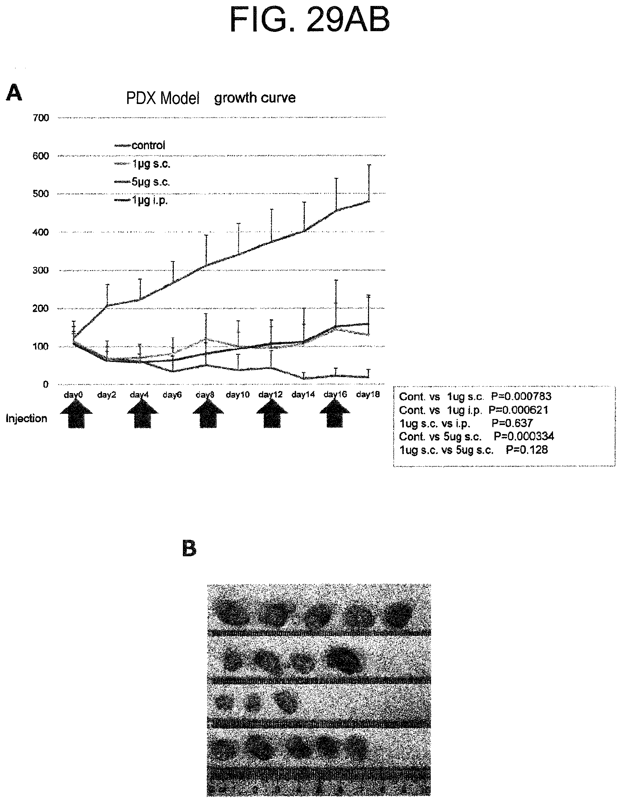

Since the number of tumor cells to be transplanted was not determined, variation was not small; however, a significant tumor shrinkage was observed in each of the 1 µg/body group and 5 µg/body group (1 µg; P = 0.011, 5 µg = P < 0.001). The volume of a tumor was measured every day in the same method as above, a change of tumor volume with time was observed. As a result, a tumor shrinkage effect was clearly observed both in the 1 µg/body group and the 5 µg/body group in a dose-dependent manner, compared to Control group. In the 5 µg/body administration group, a particularly significant effect was obtained (Figure 29A). BC2LCN-PE38 (1 µg) was dissolved in 300 µl of PBS and intraperitoneally administered, and then, tumor shrinkage effect was checked in the same manner. As a result, also in the case of the intraperitoneal administration, the same effect as in the local administration case (1 µg) was obtained (Figure 29A).

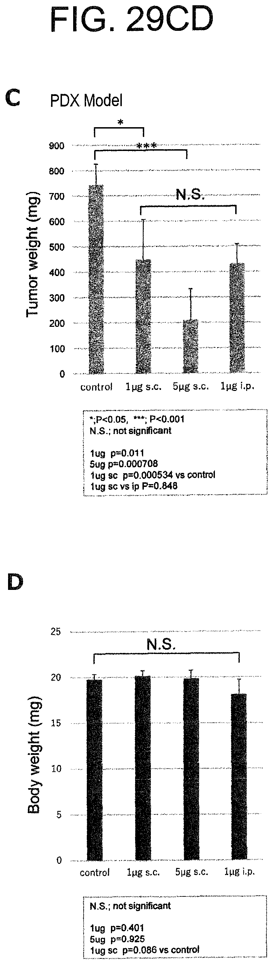

BC2LCN-PE38 was administered five times in total. Thirty days later, tumors were excised out and the weight of each of the tumors was measured. As a result, it was found that the weight significantly decreases both in the 1 µg-group and 5 µg-group and a significant shrinkage is visually observed (Figures 29A, C). In the study of intraperitoneal administration of BC2LCN-PE38 (1 µg), it was also confirmed that the same antitumor effect as in the study of local administration is obtained (Figure 29C) .

### (5-4-2) Body weight change of mouse (effect on whole body)

After tumor volume observation for 21 days, the body weight of mice was measured. In BC2LCN-PE38 (1 µg/body) and (5 µg/body) administration groups (five times administration in total), no significant difference was observed in the body weight of mice, compared to Control group (Figure 29D); activities of individual mice were satisfactory; and whole body conditions were maintained.

### (Example 5-5) Antitumor effect of BC2LCN-PE38 on Capan-1 transplanted mouse or SUIT-2 transplanted mouse

As described in Example 4-1 to Example 4-3, the responsiveness of BC2LCN is significantly high to Capan-1 strain of the pancreatic cancer cell strains, compared to, e.g., SUIT-2 strain. In this Example, these two types of pancreatic cancer cell strains were intraperitoneally transplanted to nude mice and the antitumor effect of BC2LCN-PE38 against proliferating cancer cells was compared.

### (5-5-1) Antitumor effect of BC2LCN-PE38 intraperitoneally administered

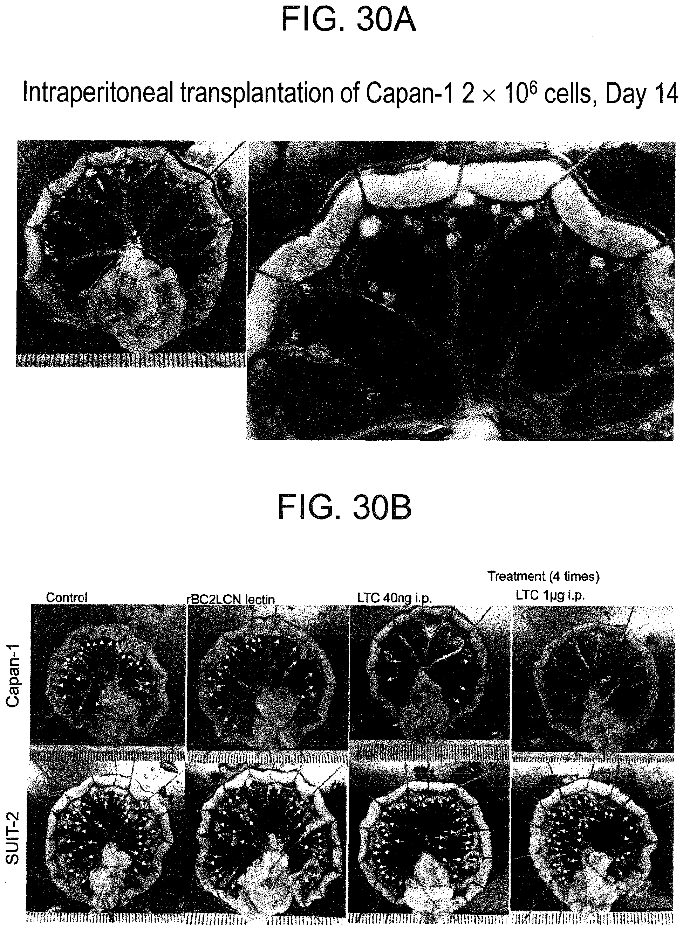

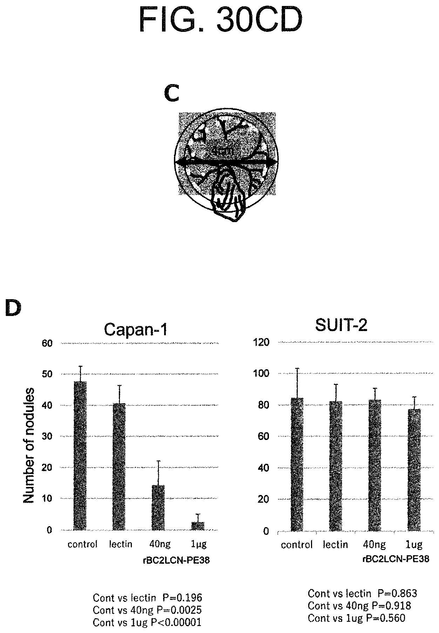

A pancreatic cancer cell strain (Capan-1 or SUIT-2) was intraperitoneally transplanted to nude mice in a ratio of 2 × 10⁶ cells. Day 14, the nude mice were each sacrificed; the digestive tract was excised out; and dissemination was observed. As a result, formation of milky white disseminated node was observed in the periphery of the mesentery (Figure 30A). Similarly, peritoneal dissemination was formed in 20 nude mice. Day 14, the mice was randomly divided into 4 groups: (Control: 0 g, BC2LCN alone 1 µg, BC2LCN-PE38 40 ng, BC2LCN-PE38 1 µg). BC2LCN-PE38 diluted with 300 µl of PBS was administered in an amount of 40 ng, 1 µg, and 2 µg/mouse, four times in total (Day 14, 18, 22, 26). Day 30 after intraperitoneal transplantation of pancreatic cancer cell strains, the mice were each sacrificed and the abdomen was opened, and the digestive tract was removed. At a position at a distance of 2 cm from the ileocecal region, the small intestine was opened like a fan (4 cm) and disseminated cells in the range of the opening (270°) were counted (Figure 30C) (N = 4 per group).

As a result, in Capan-1, which has high responsiveness to BC2LCN, the number of disseminated cells decreased in a content-dependent manner of BC2LCN-PE38. In a 1 µg-administration case, it was successful to almost completely kill disseminated cells. In contrast, in the SUIT-2, no antitumor effect was confirmed (Figures 30B, D). In the administration of BC2LCN lectin alone, no antitumor effect was confirmed in the cell strains.

### (5-5-2) Antitumor effect of BC2LCN-PE38: Staining of tissue piece taken from disseminated metastasis model

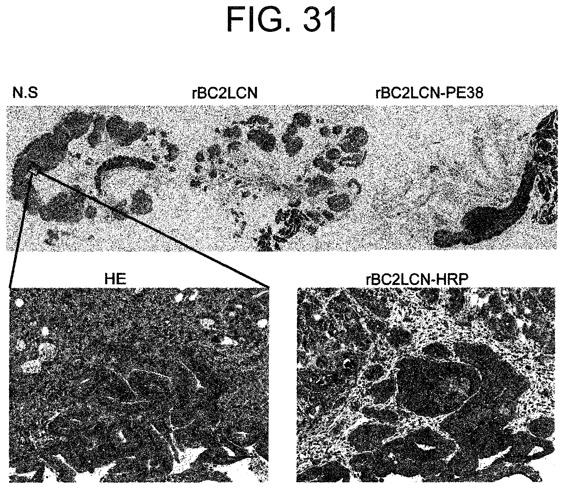

To check micro dissemination remaining in the mesentery after a treatment, the mesentery throughout the whole intestinal tract was excised out, stained with HE and then a tumor was checked. In a BC2LCN-PE38 (1 µg) administration group, a remaining tumor was not observed. In Control group (peritoneally disseminated tumor was stained with HRP-labelled BC2LCN lectin), it was confirmed that a cancer exposed from the surface of the abdominal cavity was specifically stained, specifically to cancer cell (Figure 31).

### (Example 5-6) Antitumor effect of BC2LCN-PE38 by administration in blood (through tail)

In Example 5-5-1, in the observation results on the digestive tract on Day 14 after pancreatic cancer cell strain (Capan-1) (2 × 10⁶ cells) was intraperitoneally transplanted in nude mice, formation of milky white disseminated node was observed in the periphery of the mesentery (Figure 30A). Peritoneal dissemination was formed in 20 nude mice in the same manner as in Example 5-5-1 and the nude mice were randomly divided into 5 groups on Day 14 as follows: intraperitoneal administration groups (Control: 0 g, BC2LCN alone 1 µg, BC2LCN-PE38 40 ng, BC2LCN-PE38 1 µg) and the blood administration group (BC2LCN-PE38 1 µg). Note that each group consisted of n = 10. A treatment was applied four times in total in the same manner as in Example 5-5-1 and then, dissemination of the mesentery was observed on Day 30 after Capan-1 transplantation.

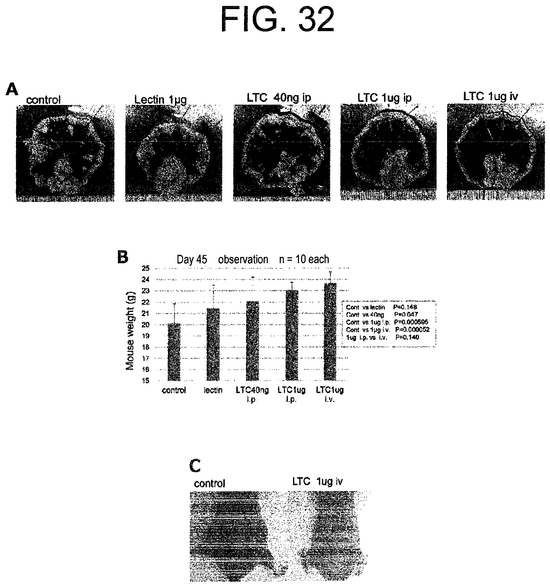

In Control group, a BC2LCN single administration group and a BC2LCN-PE38 (40 ng) group, a tumor was observed; however, in both of BC2LCN-PE38 (1 µg) administration groups (administration in the blood and abdominal cavity), a tumor disappeared (Figure 32A). In addition, whole body condition and ascites retention in the treatment groups were significantly improved. The body weight at Day 45 after the dissemination in the treatment groups significantly increased (intraperitoneal administration group; P = 0.00059, in the blood administration group; P = 0.00052, vs Control group) (Figures 32B, C).

The total survival period (median) of mice was 62 days in Control group; 65 days in the 40 ng-intraperitoneal administration group. Although it was not statistically significant (Log-Rank verification), the survival period tended to extend. In the 1 µg-blood administration group and the 1 µg-intraperitoneal administration group, the survival period were 90 days and 105 days, respectively. In both cases, extension of the survival period was confirmed with significance (P < 0.0001, Log-Rank test).

### (Example 5-7) Toxicity experiment

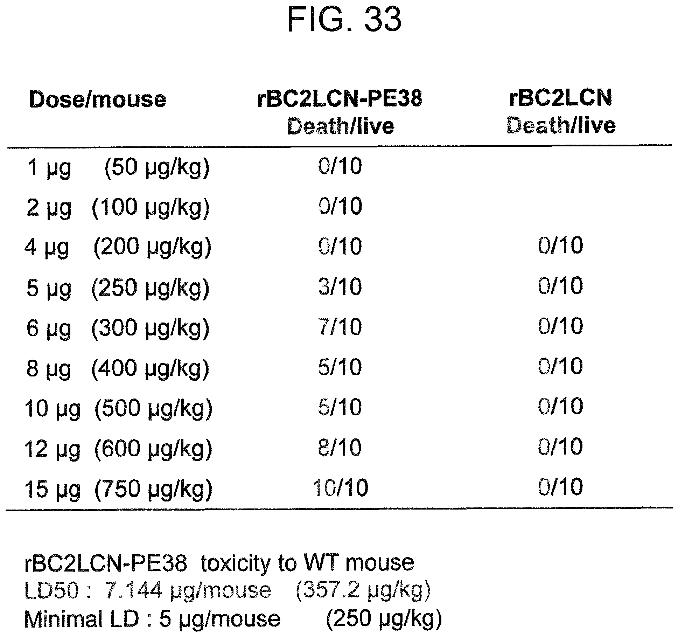

To wild type mice (C57BJ/6J, 6 week female), BC2LCN-PE38 diluted with PBS (1 µg/mouse to 15 µg/mouse) was intraperitoneally administered once. The mice were observed for 14 days to obtain mortality (N = 10 per group). To Control group, the same amount of BC2LCN was administered, administration of rBC2LC was not toxic and mouse death was not confirmed. As a result, the median lethal dose (LD50) was 7.144 µg/mouse (357.2 µg/kg) and minimum lethal dose was 5 µg/mouse (250 µg/kg) (Figure 33) .

Since it was confirmed that BC2LCN-PE38 has an ability to kill and remove pancreatic cancer cells alone without affecting the survival rate even in an in vivo system, BC2LCN-PE38 is effective as an anti-cancer agent. In addition, based on the finding that BC2LCN lectin has high binding activity to drug resistant cancer cells, the cytotoxicity of BC2LCN-PE38 is expected to be more effective to drug resistant cancer cells or cancer stem cells. Because of this, it is expected to use BC2LCN-PE38 as a composition for treating cancer in combination with a known anti-cancer agent for treating, particularly, patients affected with a drug-resistant cancer.

### [Example 6: Detection of cancer cells using the culture supernatant or body fluid sample of test individual]

### (Example 6-1) Detection of cancer cells using the culture supernatant

Biotinylated rBC2LCN (0.3 µg/mL in concentration) was immobilized to an avidin plate (manufactured by Sumitomo Bakelite Co., Ltd.). A culture supernatant of Capan-1 was reacted to the plate and then, a 1 µg/mL peroxidase labeled R-10G antibody (manufactured by Wako Pure Chemical Industries Ltd.) was reacted and absorbance at 450 nm was measured. As a control, a culture medium not subjected to cell culture was used.

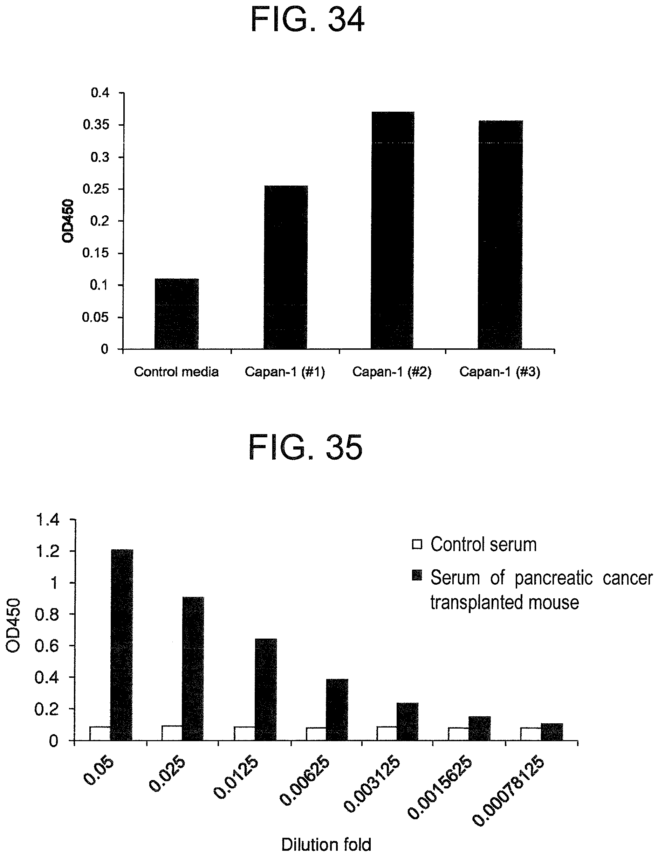

As a result, in the culture supernatant of Capan-1, a preferentially strong signal compared to the control was detected. It was found that in culture supernatant of Capan-1, Fucα1-2Galβ1-3GlcNAc/GalNAc is detected (see, Figure 34). From this, it was found that cancer cells contained in cells can be detected by using the culture supernatant of the cells.

### (Example 6-2) Detection of cancer using the serum of cancer transplanted mouse

Biotinylated rBC2LCN (0.3 µg/mL in concentration) was immobilized to avidin plates (manufactured by Sumitomo Bakelite Co., Ltd.). The serum of a control mouse and the serum of a Capan-1 transplanted mouse model were serially diluted and reacted with the plates, and then a 1 µg/mL peroxidase labeled R-10G antibody (manufactured by Wako Pure Chemical Industries Ltd.) was reacted, and absorbance at 450 nm was measured. As the control, the serum of a normal mouse was used.

As a result, no responsiveness was confirmed in the control mouse serum; however, a high signal was detected in the serum of a Capan-1 transplanted mouse model. It was found that Fucα1-2Galβ1-3GlcNAc/GalNAc is detected in the serum of a cancer transplanted mouse (see, Figure 35). From this, it was found that a cancer in a living body can be detected by using the serum of an individual affected with a cancer.

### (Example 6-3) Detection of cancer using the serum of cancer patient

Biotinylated rBC2LCN (0.3 µg/mL in concentration) was immobilized to avidin plates (manufactured by Sumitomo Bakelite Co., Ltd.). The serum was taken from two patients with extrahepatic bile duct cancer before and after surgery to remove the cancer. The sera were each diluted 10 folds with PBS and reacted with the plate, and then, a 1 µg/mL peroxidase labeled R-10G antibody (manufactured by Wako Pure Chemical Industries Ltd) was reacted and absorbance at 450 nm was measured.

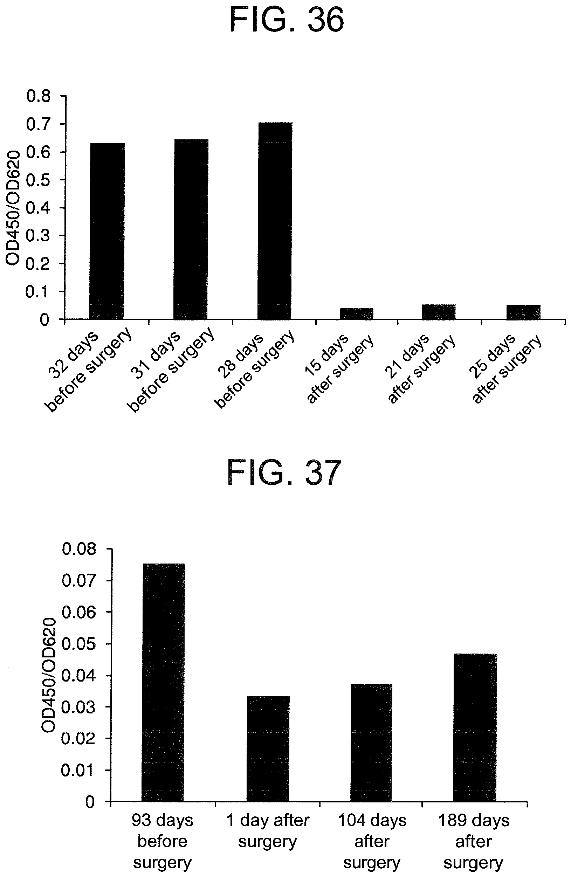

The results of the two patients are shown in Figure 36 and Figure 37, respectively. In either one of the patients, in the serum taken before surgery, a high signal was detected; however, in the serum taken after removal of the cancer, signal intensity significantly decreased. It is considered that the significant decrease of signal intensity after surgery is due to removal of the cancer. The signal intensity tends to gradually increase after surgery. This is considered that the cancer was not completely removed and the remaining cancer may grow again. These results support that a cancer in the living body can be detected by using the serum of an individual affected with a cancer, and demonstrate that effectiveness of a treatment can be determined by comparing signal intensity of the serum before and after the treatment.

Further, detection of Fucα1-2Galβ1-3GlcNAc/GalNAc in the sera of patients with various types of cancers after surgery to remove the cancer. (preoperative in patient ID: 150424006881 alone) was performed in the same manner. As a control, a buffer solution (PBS) was used.

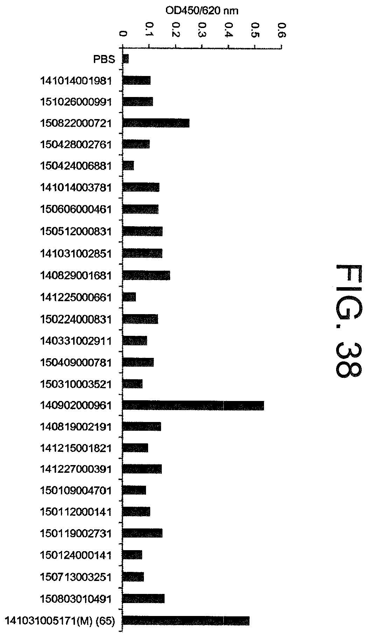

As a result, in the sera of the patients with extrahepatic bile duct cancer, stomach cancer, esophagus cancer and large intestine cancer (colon cancer, rectal cancer), high signals were detected. The results of a large intestine cancer patient are shown in Figure 38. In the figure, "PBS" on the horizontal axis indicates absorbance of a control, numbers indicate patient ID Nos. Although clinical findings of patients with large intestine cancer are not identical, significant signals were still detected in many of the postoperative patients.

### Sequence free text

SEQ ID No: 1: Amino acid sequence of BC2LCN.
SEQ ID No: 2: Amino acid sequence of BC2LCN-ETA (PE23).
SEQ ID No: 3: Amino acid sequence of cell killing domain (Domain I to III: PE38) derived from pseudomonas exotoxin A.
SEQ ID No: 4: Spacer sequence.
SEQ ID No: 5: Spacer sequence.
SEQ ID No: 6: ER retention signal sequence.

## Claims

1. A method for detecting cancer cells, comprising the steps of:
bringing BC2LCN lectin into contact with a test sample; and
determining the presence or absence or an amount of a sugar chain having a BC2LCN lectin binding activity in the test sample.

2. The detection method according to Claim 1, wherein the test sample is a body fluid sample of a test individual.

3. The detection method according to Claim 2, wherein the body fluid sample is derived from blood.

4. The detection method according to any one of Claims 1 to 3, wherein the cancer cells are large intestine cancer cells or bile duct cancer cells.

5. The detection method according to any one of Claims 1 to 3, wherein the cancer cells are high-grade cancer cells.

6. The detection method according to Claim 5, wherein the high-grade cancer cells are drug resistant cancer cells or cancer stem cells.

7. The detection method according to Claim 1, wherein the test sample is a tumor tissue excised out from an organ or a tissue of the test individual or a peripheral tissue thereof, or a tissue sample or a cell sample derived from a biopsy material thereof.

8. A kit or apparatus for detecting the presence or absence of cancer cells in a test sample, containing at least the following (1) to (3);
(1) BC2LCN lectin,
(2) a labelling agent, and
(3) a means or device for detecting a label.

9. A method for collecting data for determining that a test individual is affected with a cancer, comprising a step of measuring an amount of a sugar chain having a BC2LCN lectin binding activity in a test sample of a test individual suspected to be affected with a cancer, wherein if the amount is significantly high compared to an amount of a healthy person, it is determined that the test individual is affected with a cancer.

10. A method for collecting data for determining that a therapy is effective, comprising the steps of:
measuring an amount of a sugar chain having a BC2LCN lectin binding activity in a test sample of a test individual to which a cancer therapy is applied, and
comparing the above amount to an amount of a sugar chain having a BC2LCN lectin binding activity previously measured before the therapy in a test sample of the test individual,
wherein if the amount after the therapy is significantly low compared to the amount previously measured before the therapy, it is determined that the therapy is effective.

11. The collection method according to Claim 9 or 10, wherein the test sample is a body fluid sample of the test individual.

12. A reagent containing BC2LCN lectin and a substance to be fused to the BC2LCN lectin, for introducing the substance into cancer cells.

13. The reagent according to Claim 12, wherein the substance is a substance which can exhibit cytotoxicity in cancer cells.

14. The reagent according to Claim 13, wherein the substance which can exhibit cytotoxicity in cancer cells is a toxic protein or a domain thereof having an ability to kill cells.

15. The reagent according to Claim 14, wherein the substance which can exhibit cytotoxicity in cancer cells is a cell killing domain derived from a pseudomonas exotoxin A.

16. The reagent according to Claim 15, wherein the cell killing domain derived from a pseudomonas exotoxin A is Domain I-III (PE38) represented by SEQ ID No: 3

17. The reagent according to any one of Claims 12 to 16, wherein the cancer cells are high-grade cancer cells.

18. The reagent according to Claim 17, wherein the high-grade cancer cells are drug-resistant cancer cells or cancer stem cells.

19. The reagent according to Claim 17, wherein the high-grade cancer cells are pancreatic cancer cells.

20. A composition for treating a cancer, comprising a BC2LCN-toxin fusion prepared by fusing BC2LCN lectin and a substance which can exhibit cytotoxicity in cancer cells, as an active ingredient and a pharmacologically acceptable carrier.

21. The composition according to Claim 20, wherein the substance which can exhibit cytotoxicity in cancer cells is a cell killing domain derived from pseudomonas exotoxin A.

22. The composition according to Claim 21, wherein the cell killing domain is Domain I-III (PE38) derived from pseudomonas exotoxin A represented by SEQ ID No: 3.

23. The composition according to any one of Claims 20 to 22, for use together or in combination with a known therapeutic composition applicable to a cancer.
